(19) Europäisches Patentamt
European Patent Office
Office européen des brevets

(11) EP 3 137 458 B1

(12) **EUROPÄISCHE PATENTSCHRIFT**

(45) Veröffentlichungstag und Bekanntmachung des Hinweises auf die Patenterteilung:
**24.04.2019 Patentblatt 2019/17**

(21) Anmeldenummer: **15722050.0**

(22) Anmeldetag: **30.03.2015**

(51) Int Cl.:
***C07D 405/14*** (2006.01) ***C07D 409/14*** (2006.01)
***C07D 471/04*** (2006.01) ***C07D 487/04*** (2006.01)
***C09B 57/00*** (2006.01) ***H01L 51/00*** (2006.01)

(86) Internationale Anmeldenummer:
**PCT/EP2015/000680**

(87) Internationale Veröffentlichungsnummer:
**WO 2015/165563 (05.11.2015 Gazette 2015/44)**

# (54) MATERIALIEN FÜR ELEKTRONISCHE VORRICHTUNGEN

MATERIALS FOR ELECTRONIC DEVICES

MATÉRIAUX POUR DISPOSITIFS ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.04.2014 EP 14001525**

(43) Veröffentlichungstag der Anmeldung:
**08.03.2017 Patentblatt 2017/10**

(73) Patentinhaber: **Merck Patent GmbH**
**64293 Darmstadt (DE)**

(72) Erfinder:
- **PARHAM, Amir, Hossain**
  **60486 Frankfurt am Main (DE)**
- **GROSSMANN, Tobias**
  **64297 Darmstadt (DE)**
- **JATSCH, Anja**
  **60489 Frankfurt am Main (DE)**
- **EBERLE, Thomas**
  **76829 Landau (DE)**
- **KROEBER, Jonas, Valentin**
  **60311 Frankfurt (DE)**
- **PFLUMM, Christof**
  **64291 Darmstadt (DE)**
- **DOBELMANN, Lars**
  **64295 Darmstadt (DE)**

(56) Entgegenhaltungen:
**WO-A1-2012/130709** **WO-A2-2011/057706**
**JP-A- 2013 131 518** **US-A1- 2011 006 670**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Vorliegende Erfindung betrifft cyclische Verbindungen mit einer spezifischen Anordung von elektronenleitenden und lochleitenden Gruppen, deren Verwendung in elektronischen Vorrichtungen, deren Herstellung sowie elektronische Vorrichtungen.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es sowohl bei OLEDs, die Singulettemission zeigen, wie auch bei OLEDs, die Triplettemission zeigen, immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und insbesondere blau, emittieren.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden unter anderem Ketone (z. B. gemäß WO 2004/093207 oder WO 2010/006680) oder Phosphinoxide (z. B. gemäß WO 2005/003253) als Matrixmaterialien für phosphoreszierende Emitter verwendet. Weitere Matrixmaterialien gemäß dem Stand der Technik repräsentieren Triazine (bspw. WO 2008/056746, EP 0906947, EP 0908787, EP 0906948).

Für fluoreszierende OLEDs werden gemäß dem Stand der Technik vor allem kondensierte Aromaten, insbesondere Anthracenderivate, als Host-Materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis-(1-naphthyl)anthracen-Derivate für die Verwendung in OLEDs offenbart. Weitere Anthracenderivate sind in WO 01/076323, in WO 01/021729, in WO 2004/013073, in WO 2004/018588, in WO 2003/087023 oder in WO 2004/018587 offenbart. Host-Materialien, basierend auf Arylsubstituierten Pyrenen und Chrysenen, werden in WO 2004/016575 offenbart. Host-Materialien basierend auf Benzanthracenderivaten werden in WO 2008/145239 offenbart. Es ist für hochwertige Anwendungen wünschenswert, verbesserte Host-Materialien zur Verfügung zu haben.

Im Stand der Technik ist die Verwendung von Verbindungen enthaltend eine oder mehrere Carbazolgruppen in elektronischen Vorrichtungen, beispielsweise aus WO 2005/039246, US 2005/0069729, JP 2004/288381, EP 1205527 oder WO 2008/086851 bekannt

Weiterhin im Stand der Technik bekannt ist die Verwendung von Verbindungen enthaltend eine oder mehrere Indenocarbazolgruppen in elektronischen Vorrichtungen, beispielsweise aus WO 2010/136109 und WO 2011/000455.

Weiterhin im Stand der Technik bekannt ist die Verwendung von Verbindungen enthaltend eine oder mehrere elektronenarme heteroaromatische Sechsringe in elektronischen Vorrichtungen, beispielsweise aus WO 2010/015306, WO 2007/063754 und WO 2008/056746.

WO 2009/069442 offenbart Tricyclen, wie Carbazol, Dibenzofuran oder Dibenzothiophen, die hochgradig mit elektronenarmen Heteroaromaten (z.B. Pyridin, Pyrimidin oder Triazin) substituiert sind. Mit lochleitenden Gruppen, d.h. elektronenreichen Gruppen, sind die Tricyclen nicht substituiert.

JP 2009-21336 offenbart substituierte Dibenzofurane, die in 2 Position mit Carbazol und in 8 Position mit einem Triazin substituiert sind.

WO 2011/057706 offenbart vereinzelt substituierte Dibenzothiophene und Dibenzofurane als Matrixmaterialien, wobei die Verbindungen in spezifischer Weise mit einer elektronenleitenden und mit einer lochleitenden Gruppe substituiert sind.

WO 2011/057706 A2 offenbart Carbazole, dibenzofurane und dibenzothiophene, die mit einer lochleitenden und einer elektronenleitenden Gruppe substituiert sind, sowie deren Verwendung in elektrolumineszierenden Vorrichtungen.

WO 2012/130709 A1 offenbart Dibenzofurane, die mit Carbazolen und anderen stickstoffhaltigen Gruppen substituiert sind sowie deren Verwendung in elektrolumineszierenden Vorrichtungen.

JP 2013-131518 A offenbart Dibenzothiophene und Dibenzofurane, die in den Positionen 4 und 8 substituiert sind sowie deren Verwendung in elektrolumineszierenden Vorrichtungen.

Allerdings besteht bei Verwendung dieser Materialien ebenso wie bei anderen Materialien noch Verbesserungsbedarf, insbesondere in Bezug auf die Effizienz und die Lebensdauer der Vorrichtung.

Aufgabe der vorliegenden Erfindung ist daher die Bereitstellung von Verbindungen, welche sich für den Einsatz

in einer fluoreszierenden oder phosphoreszierenden OLED eignen, beispielsweise als Host- und/oder Matrixmaterial oder als Lochtransport-/Elektronenblockiermaterial bzw. Exzitonenblockiermaterial oder als Elektronentransport- bzw. Lochblockiermaterial, und welche bei Verwendung in einer OLED zu guten Device-Eigenschaften führen, sowie die Bereitstellung der entsprechenden elektronischen Vorrichtung.

Überraschend wurde gefunden, dass bestimmte, unten näher beschriebene Verbindungen diese Aufgaben lösen und zu guten Eigenschaften der organischen Elektrolumineszenzvorrichtung führen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Verbindungen sind daher der Gegenstand der vorliegenden Erfindung. Die überraschenden Effekte werden durch eine spezielle Anordnung elektronenleitender und lochleitender Gruppen in Verbindungen der unten aufgeführten Formeln erreicht.

Die erfindungsgemäßen Verbindungen zeichnen sich weiterhin durch eine hohe Temperaturstabilität aus, so dass sie im Hochvakuum unzersetzt verdampft werden können. Diese Eigenschaft ist eine Grundvoraussetzung für die reproduzierbare Darstellung von organischen elektronischen Vorrichtungen, wie den organischen Elektrolumineszenzvorrichtungen, und wirkt sich insbesondere positiv auf die operative Lebensdauer aus.

Die erfindungsgemäßen Verbindungen weisen auch eine hohe Glasübergangstempertur ($T_g$) auf, was vorteilhaft ist hinsichtlich der Prozessierung der Verbindungen bei der Herstellung elektronischer Vorrichtungen. Die hohe Glasübergangstemperatur der Verbindungen gestattet auch die Verwendung der Verbindungen in dünnen amorphen organischen Schichten.

Weiterhin erlauben die erfindungsgemäßen Verbindungen eine Stabilisierung der Ladungsträger im angeregten Zustand und weisen eine ausreichend hohe Triplett Energie auf, was für phosphoreszierende Vorrichtungen eine wichtige Vorraussetzung darstellt. Ferner zeigen die erfindungsgemäßen Verbindungen verbesserte Leistungsdaten in OLEDs gegenüber den Verbindungen aus dem Stand der Technik.

Die erfindungsgemäßen Verbindungen zeichnen sich auch durch eine verbesserte Redoxstabilität in Lösung gegenüber aus dem Stand der Technik bekannten Verbindungen aus. Das vereinfacht die Reinigung der Verbindungen, vereinfacht deren Handhabung und verbessert deren Lagerstabilität in Lösung, die zur Herstellung organischer elektronischer Vorrichtungen aus Lösung mit Hilfe von Druckverfahren hergestellt werden.

Schließlich zeichnen sich die erfindungsgemäßen Verbindungen durch eine sehr gute Löslichkeit aus, so dass die Verbindungen auch aus Lösung prozessiert werden können. Somit ist eine kostengünstige Herstellung organischer elektronischer Vorrichtung bewerkstelligen. Die erfindungsgemäßen Verbindungen eignen sich daher auch für die Massenproduktion organischer elektronischer Vorrichtungen.

Gegenstand der vorliegenden Erfindung ist eine Verbindung der allgemeinen Formel (1)

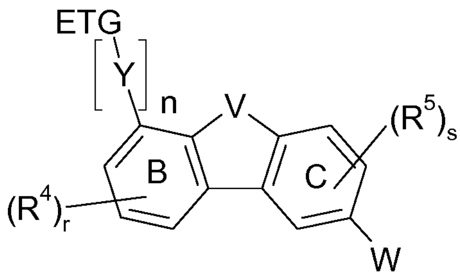

Formel (1)

wobei die verwendeten Symbole und Indizes die in Anspruch 1 angegebene Bedeutung haben.

Ferner kann V in der Verbindung der Formel (1) gleich S sein.

Elektronenarme heteroaromatische Gruppen als ETG können ausgewählt werden aus den folgenden Gruppen.

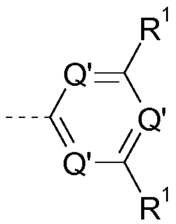

Formel (E-1)

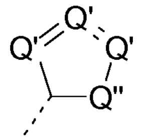

Formel (E-2)

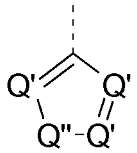

Formel (E-3)

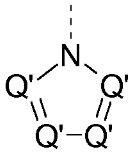

Formel (E-4)

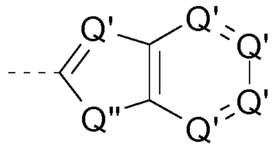

Formel (E-5)

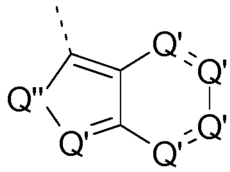

Formel (E-6)

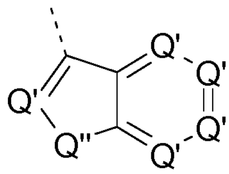

Formel (E-7)

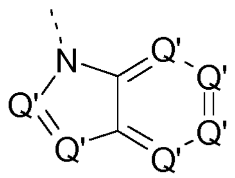

Formel (E-8)

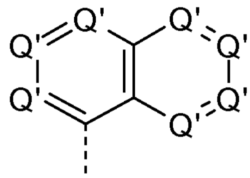

Formel (E-9)

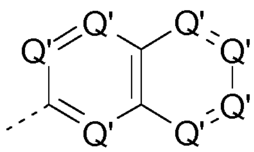

Formel (E-10),

wobei die gestrichelte Bindung die Anbindungsposition markiert, $R^1$ wie oben definiert ist und

Q' bei jedem Auftreten gleich oder verschieden $CR^1$ oder N darstellt, und

Q" $NR^1$, O oder S darstellt;

wobei wenigstens ein Q' gleich N und/oder wenigstens ein Q" gleich $NR^1$ ist.

Bevorzugte elektronenarme heteroaromatische Gruppen als ETG sind: Pyridine, Pyrazine, Pyrimidine, Pyridazine, 1,2,4-Triazine, 1,3,5-Triazine, Chinoline, Isochinoline, Chinoxaline, Pyrazole, Imidazole, Benzimidazole, Thiazole, Benzothiazole, Oxazole oder Benzooxazole, die jeweils mit $R^1$ substituiert sein können. Noch mehr bevorzugt ist die elektronentransportierende Gruppe ein mit einem oder mehreren Resten $R^1$ substituiertes Pyridin, Pyrazin, Pyrimidin, Pyridazin und 1,3,5-Triazin.

Die Verbindung der Formel (1) enthaltend die Elektronentransportgruppe weist bevorzugt eine LUMO (lowest unoccupied molecular orbital) Energie auf, die niedriger als -1.3 eV ist, ganz bevorzugt niedriger als -2.5 eV und ganz besonders bevorzugt niedriger als -2.7 eV.

HOMO- (highest occupied molecular orbital) und LUMO- (lowest unoccupied molecular orbital) Energieniveaus sowie die Energie des niedrigsten Triplettzustands $T_1$ bzw. die des niedrigsten angeregten Singulettzustands $S_1$ der Materialien werden über quantenchemische Rechnungen bestimmt. Hierzu wird vorliegend das Programmpaket "Gaussian09W" (Gaussian Inc.) verwendet. Zur Berechnung organischer Substanzen wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31 G(d)" verwendet (Charge 0, Spin Singlet). Aus der Energie--rechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplett-zustands mit der niedrigsten Energie, der sich aus der beschriebenen quantenchemischen Rechnung ergibt.

Der niedrigste angeregte Singulettzustand $S_1$ ist definiert als die Energie des angeregten Singulett-zustands mit der niedrigsten Energie, der sich aus der beschriebenen quantenchemischen Rechnung ergibt.

Weiter bevorzugt ist, wenn die Verbindung der Formel (1) eine Elektronenmobilität von $\mu$- $10^{-6}$ $cm^2/(Vs)$ oder mehr aufweist, ganz bevorzugt beträgt sie $10^{-5}$ $cm^2/(Vs)$ oder mehr und ganz besonders bevorzugt beträgt sie $10^{-4}$ $cm^2/(Vs)$ oder mehr.

In den Verbindungen nach Formel (1) ist das LUMO bevorzugt auf der Elektronentransportgruppe lokalisiert sein, wobei eine Aufenthaltswahrscheinlichkeit von 0.9 für die Orbitale angenommen wird. Ganz bevorzugt ist, wenn das LUMO mehr als 80 % auf elektrontransportierende Gruppe lokalisiert ist, noch bevorzugter, wenn das LUMO überhaupt nicht auf der Gruppe W (bspw. eine Carbazolgruppe) lokalisiert ist. Insbesondere bevorzugt ist, wenn das HOMO und das LUMO der erfindungsgemäßen Verbindung überhaupt nicht überlappen. Der Fachmann hat keinerlei Schwierigkeiten die Überlappung der Orbitale zu ermitteln.

Die Überlappung der Molekülorbitale, die bei bestimmten elektronischen Übergängen beteiligt sind (Charge-Transfer-Zustände) wird mit Hilfe des Parameters A beschrieben. Dabei ist dem Fachmann die Bedeutung des Parameters A gut bekannt. Die Bestimmung des Parameters mittels Verfahren, die im Stand der Technik beschrieben sind, bereitet dem Fachmann keinerlei Schwierigkeiten. Im Rahmen der vorliegenden Erfindung wird der Parameter A anhand der PBHT-Methode gemäß D. J. Tozer et al. (J. Chem. Phys. 128, 044118 (2008)) bestimmt, die beispielsweise in dem Programmpaket Q-Chem 4.1 von Q-Chem, Inc. Implementiert ist. Dabei werden die Molekülorbitale gemäß dem oben beschriebenen Verfahren berechnet. Anschließend werden die räumlichen Überlappungen für alle möglichen Paare von besetzten Molekülorbitalen, $\varphi_i$, und unbesetzten (virtuellen) Molekülorbitalen, $\varphi_a$, gemäß folgender Gleichung ermittelt

$$O_{ia} = \langle |\varphi_i| \, | \, |\varphi_a| \rangle$$

wobei für die Berechnung die Beträge der Orbitale verwendet werden.

Der Parameter $\Lambda$ ergibt sich dann aus der gewichteten Summe über alle Paare ia von besetzten und unbesetzten Molekülorbitalen gemäß

$$\Lambda = \frac{\sum_{ia} \kappa_{ia}^2 O_{ia}}{\sum_{ia} \kappa_{ia}^2}$$

wobei der Wert von $\kappa_{ia}$ gemäß Tozer et al. aus den Orbitalkoeffizienten in den Anregungsvektoren der gelösten TD-Eigenwertgleichung (Time-Dependent) ermittelt wird und wobei $0 \leq \Lambda \leq 1$ gilt.

In einer bevorzugten Ausführungsform betrifft die vorliegende Erfindung eine Verbindung der allgemeinen Formel (1) mit einer geringen räumlichen Überlappung der Molekülorbitale $\Lambda$, die bei bestimmten elektronischen Übergängen beteiligt sind (Charge-Transfer-Zustände).

Vorliegend bedeutet eine geringe Überlappung der Molekülorbitale, dass der Wert des Parameters $\Lambda$ 0.3 oder kleiner ist, bevorzugt ist er 0.2 oder kleiner, ganz bevorzugt ist er 0.15 oder kleiner, ganz besonders bevorzugt ist er 0.1 oder kleiner und insbesondere bevorzugt ist er 0.05 oder kleiner.

Die Verbindung der Formel (1) enthaltend die Lochtransportgruppe W hat vorzugsweise eine HOMO Energie ($HOMO_W$), die im Bereich der Elektronenaustrittsarbeit der verwendeten Anode ($\phi_{Anode}$) zuzüglich +1.5 eV oder niedriger entspricht, d.h. es gilt:

$$\text{HOMO}_W \leq (\phi_{\text{Anode}} + 1.5\ \text{eV})$$

Wenn die verwendete Anode eine Elektronenaustrittsarbeit von -5 eV hat, so ist die HOMO Energie der Verbindung der Formel (1) -3.5 eV oder niedriger (d.h. negativer als -3.5 eV). Ganz bevorzugt ist, wenn die Verbindung der Formel (1) eine HOMO Energie aufweist, die gleich der Elektronenaustrittsarbeit der Anode oder niedriger ist, ganz besonders bevorzugt ist sie niedriger.

Weiter bevorzugt ist die Verbindung der Formel (1) dadurch charakterisiert, dass die Lochmobilität $\mu_+$ $10^{-6}$ $cm^2/(Vs)$ oder mehr beträgt, ganz bevorzugt beträgt sie $10^{-5}$ $cm^2/(Vs)$ oder mehr und ganz besonders bevorzugt beträgt sie $10^{-4}$ $cm^2/(Vs)$ oder mehr.

Die Messung von Elektronen- und Lochmobilitäten werden routinemäßig vom Fachmann mittels Standardverfahren durchgeführt.

In den Verbindungen nach Formel (1) wird das HOMO maßgeblich auf der Lochtransportgruppe W lokalisiert sein. Maßgeblich bedeutet hierbei, dass das HOMO zu 80% oder mehr auf der lochleitenden Gruppe lokalisiert ist oder ist nicht auf elektronenarmen elektronentransportierenden Gruppe lokalisiert, wobei eine Aufenthaltswahrscheinlichkeit von 0.9 für die Orbitale angenommen wird.

Bevorzugt im Sinne der vorliegenden Erfindung ist eine Verbindung der allgemeinen Formel (2)

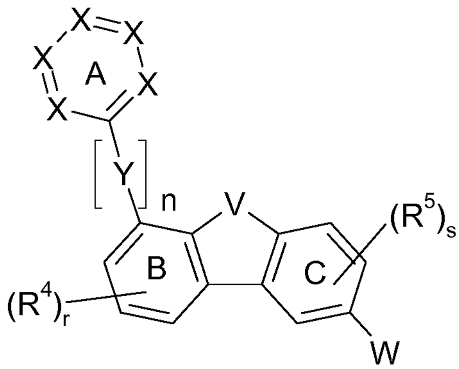

Formel (2)

wobei für die verwendeten Symbole und Indizes obige Definitionen gelten und weiterhin gilt:

X ist N oder $CR^1$, wobei X so ausgewählt ist, dass es sich bei dem Ring A um ein Triazin, Pyrimidin oder Pyrazin handelt,ganz besonders bevorzugt ist, wenn es sich bei dem Rng A um ein Triazin, insbesondere bevorzugt um ein 1,3,5-Triazin handelt;

W ist eine Gruppe der Formel (W-1)

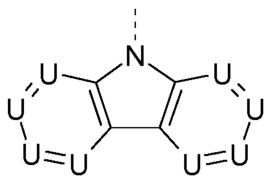

Formel (W-1)

U ist N oder $CR^1$, bevorzugt $CR^1$; wobei die gepunktete Linie die Bindung der Gruppe W and den Ring C in Formel (2) kennzeichnet;

In einer bevorzugten Ausführungsform können zwei oder mehr benachbarte Reste $R^1$ der Gruppen U = $CR^1$ miteinander ein mono- oder polycyclisches, aliphatisches oder aromatisches Ringsystem bilden.

In einer weiteren bevorzugten Ausführungsform können zwei oder mehr benachbarte Reste $R^1$ der Gruppen U = $CR^1$ miteinander kein mono- oder polycyclisches, aliphatisches oder aromatisches Ringsystem bilden.

Ferner ist bevorzugt, wenn wenigstens einer der Reste $R^1$ aus den Gruppen U = $CR^1$ ungleich H ist.

Ganz bevorzugt im Sinne der vorliegenden Erfindung ist, wenn $R^1$ als Gruppe U = $CR^1$ H oder ein aromatisches oder heteroaromatisches Ringsystem mit 5 bis 60 aromatischen Ringatomen, das jeweils durch einen oder mehrere Reste $R^2$ substituiert sein kann, wobei besonders bevorzugt ist, wenn wenigstens einer der Reste $R^1$ aus U = $CR^1$ ungleich H ist. Besonders bevorzugt ist, wenn $R^1$ in jedem Fall H oder ein aromatisches oder heteroaromatisches Ringsystem mit 5 bis 60 aromatischen Ringatomen, das jeweils durch einen oder mehrere Reste $R^2$ substituiert sein kann, wobei besonders bevorzugt ist, wenn wenigstens einer der Reste $R^1$ ungleich H ist.

Bevorzugt ist, wenn die Verbindung die allgemeine Formel (1) aufweist und wobei r entweder 0 oder 1 ist und wobei s entweder 0 oder 1 ist. Bevorzugt ist entweder nur r oder nur s gleich 1 und der jeweils andere Parameter gleich 0, ganz bevorzugt sind sowohl r als auch s gleich 0.

In einer weitere bevorzugten Ausführungsform der vorliegenden Erfindung ist n immer gleich 1, so dass zwischen Ring A und Ring B immer eine bivalente Brücke vorhanden ist.

Weiterhin bevorzugt ist, wenn die Verbindung die allgemeine Formel (4) aufweist, wobei für die verwendeten Indizes und Symbole obige Definitionen gelten und deren an anderer Stelle der vorliegenden Erfindung genannten bevorzugten Ausführungsformen auch bevorzugte Ausführungsformen für Verbindungen der Formel (4) darstellen.

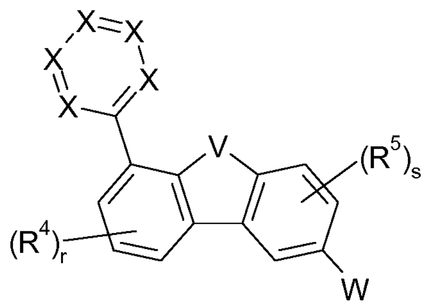

Formel (4)

wobei obige Definitionen für die verwendeten Symbole ind Indizes gelten und wobei r entweder 0 oder 1 ist und wobei s entweder 0 oder 1 ist.

Weiterhin bevorzugt ist, wenn r+s=1 gilt.

Auch bevorzugt ist, wenn die Verbindung die allgemeine Formel (6) aufweist, wobei für die verwendeten Indizes und Symbole obige Definitionen gelten und deren an anderer Stelle der vorliegenden Erfindung genannten bevorzugten Ausführungsformen auch bevorzugte Ausführungsformen für Verbindungen der Formel (6) darstellen.

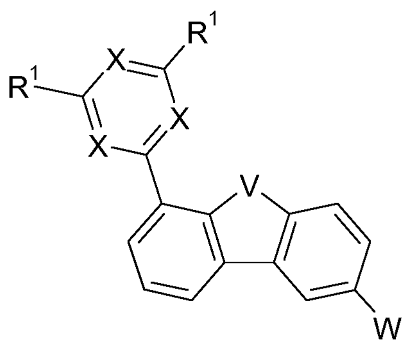

Formel (6)

Noch bevorzugter ist, wenn die Verbindung die allgemeine Formel (7) aufweist, wobei für die verwendeten Indizes und Symbole obige Definitionen gelten und deren an anderer Stelle der vorliegenden Erfindung genannten bevorzugten Ausführungsformen auch bevorzugte Ausführungsformen für Verbindungen der Formel (7) darstellen.

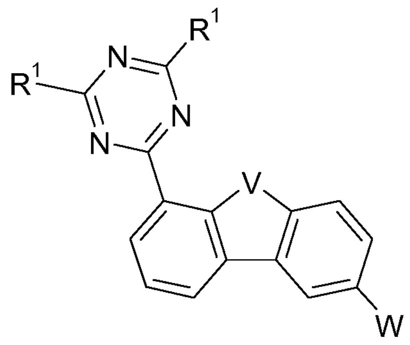

Formel (7)

Es ist bevorzugt wenn es sich bei der Gruppe W um ein Carbazol, ein Indenocarbazol oder um ein Indolocarbazol handelt, wobei die Gruppen wie hierin offenbart substituiert sein können.

In einer bevorzugten Ausführungsform der vorliegenden Erfindung handelt es sich bei der Gruppe W um ein Carbazol, das mit einem oder mehreren Resten $R^1$, die bei jedem Auftreten gleich oder verscheiden sein können, substituiert sein kann, wobei benachbarte Reste $R^1$ keinen Ringschluss miteinander bilden können.

Ganz bevorzugt ist, wenn es sich bei der Gruppe W um ein Indenocarbazol handelt, das mit einem oder mehreren Resten $R^1$ bzw. $R^2$, die bei jedem Auftreten gleich oder verscheiden sein können, substituiert ist.

Weiterhin ganz bevorzugt ist, wenn es sich bei der Gruppe W um ein Indolocarbazol handelt, das mit einem oder mehreren Resten $R^1$ bzw. $R^2$, die bei jedem Auftreten gleich oder verscheiden sein können, substituiert ist.

Eine Gruppe W der folgenden Formel (W-2) ist besonders bevorzugt

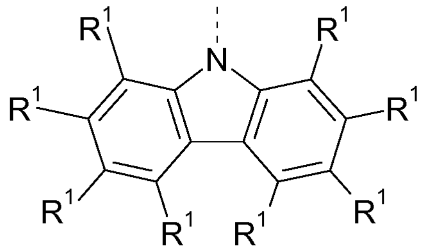

Formel (W-2)

Ganz besonders bevorzugt ist eine Guppe W der Formel (W-3)

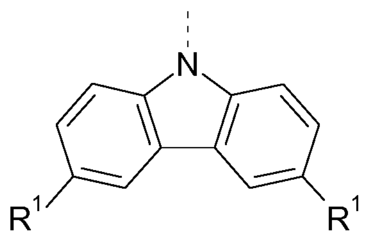

Formel (W-3)

Insbesondere bevorzugt ist eine Gruppe W der Formel (W-4)

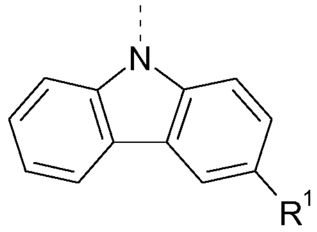

Formel (W-4)

Noch bevorzugter ist, wenn die Gruppe W die Formel (W-4) aufweist und der darin vorkommende Rest $R^1$ ungleich Wasserstoff ist, wobei noch bevorzugter ist, wenn $R^1$ in Formel (W-4) ein aromatisches oder heteroaromatisches Ringsystem mit 5 bis 60 aromatischen Ringatomen, das jeweils durch einen oder mehrere Reste $R^2$ substituiert sein kann, ist.

In einer anderen sehr bevorzugten Ausführungsform der vorliegenden Erfindung weist die Gruppe W die Formel (W-4) auf, wobei auch $R^1$ gleich H ist.

Besonders bevorzugt ist, wenn die Gruppe W eine Gruppe der Formel (W-5) ist

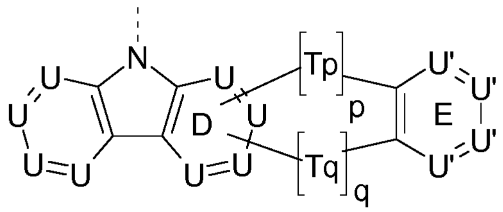

Formel (W-5)

wobei für die verwendeten Indizes und Symbole obige Definitionen gelten und wobei weiterhin gilt:

Tp, Tq sind gleich oder verschieden eine bivalente Brücke; bevorzugt sind Tp und Tq ausgewählt aus $N(R^2)$, $B(R^2)$, O, $C(R^2)_2$, $Si(R^2)_2$, C=O, $C=NR^2$, $C=C(R^2)_2$, S, S=O, $SO_2$, $P(R^2)$ und $P(=O)R^2$; ganz bevorzugt sind dabei $N(R^2)$, O, $C(R^2)_2$ und S und insbesondere bevorzugt sind $N(R^2)$ und $C(R^2)_2$;

U’ ist gleich oder verschieden bei jedem Auftreten $CR^2$ oder N, bevorzugt $CR^2$;

p ist 0 oder 1; wobei p gleich 0 bedeutet, dass der Ring E und der Ring D durch eine Einfachbindung verknüpft sind;

q ist 0 oder 1; wobei q gleich 0 bedeutet, dass der Ring E und der Ring D durch eine Einfachbindung verknüpft sind;

und wobei gilt, dass p + q = 1 oder 2 und bevorzugt gleich 1 ist;
und wobei Tp und Tq jeweils an benachbarte Gruppen U des Rings D in jeder möglichen Orientierung binden; und wobei weiterhin gilt, dass jede Gruppe U, die an Tp oder Tq bindet ein Kohlenstoffatom darstellt.

Ganz besonders bevorzugte Gruppen W sind ausgewählt aus den folgenden Gruppen der Formeln (W-6) bis (W-8), wobei die der Formel (W-7) insbesondere bevorzugt ist.

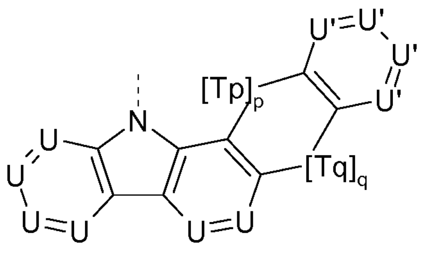

Formel (W-6)

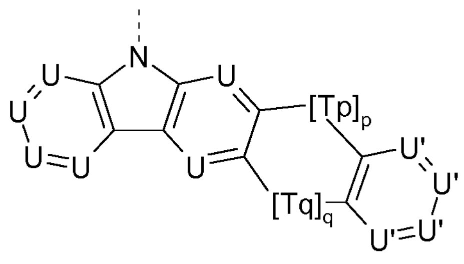

Formel (W-7)

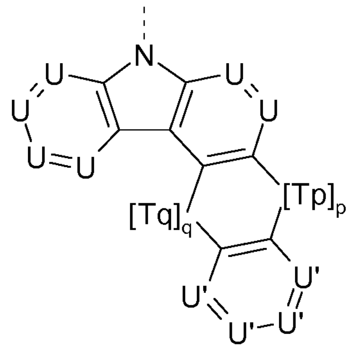

Formel (W-8)

Insbesondere bevorzugte Gruppen W stellen solche der Formeln (W-9) bis (W-14) dar.

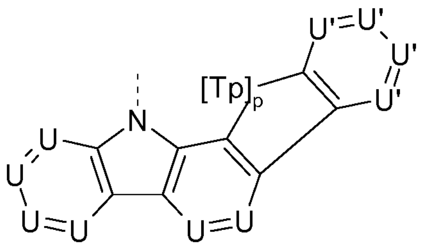

Formel (W-9)

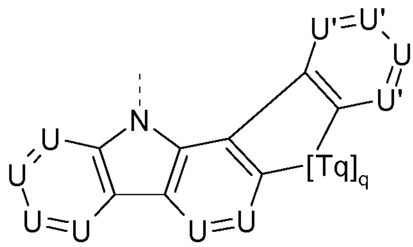

Formel (W-10)

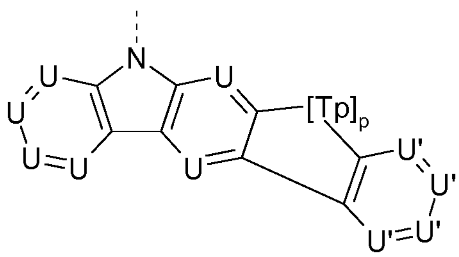

Formel (W-11)

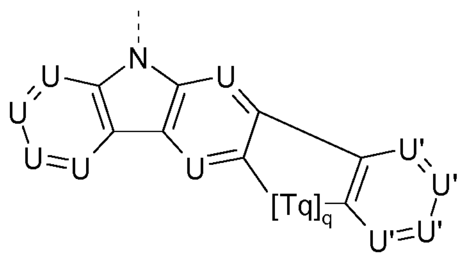

Formel (W-12)

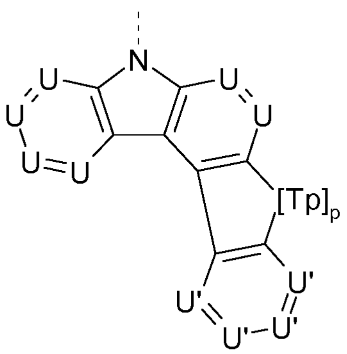

Formel (W-13)

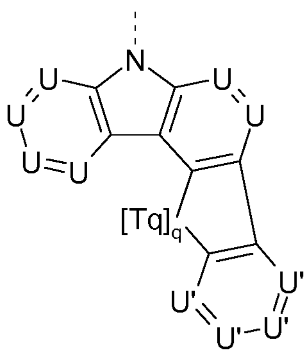

Formel (W-14)

In einer bevorzugten Ausführungsform der vorliegenden Erfindung ist die Gruppe W ein Indenocarbazol, das ganz bevorzugt eine der folgenden Formeln (W-15) bis (W-20) aufweist

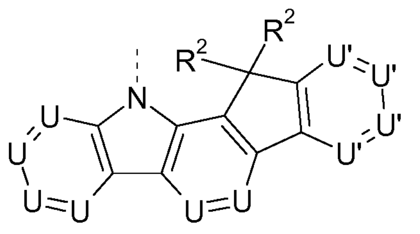

Formel (W-15)

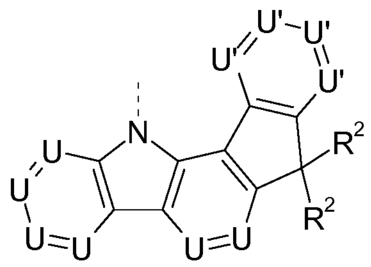

Formel (W-16)

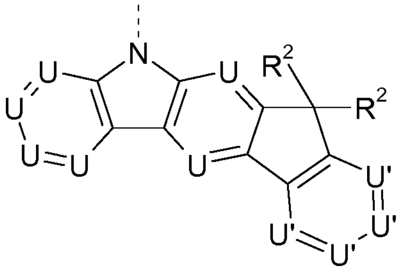

Formel (W-17)

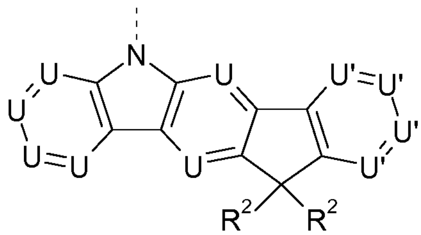

Formel (W-18)

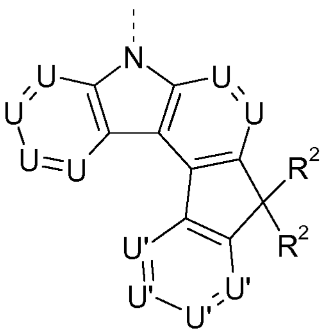

Formel (W-19)

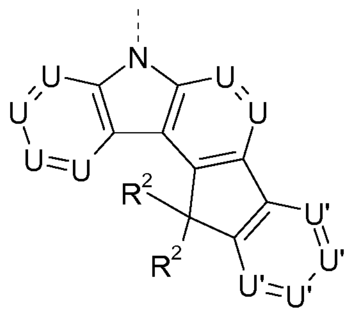

Formel (W-20)

wobei die Indenocarbazole der Formeln (W-17), (W-18), (W-19), (W-15) und (W-16) ganz besonders bevorzugt sind. Insbesondere bevorzugt sind die Indenocarbazole der Formeln (W-17), (W-18) und (W-19), noch bevorzugter die der Formeln (W-17) und (W-18) und am meisten bevorzugt sind die der Formel (W-17).

In einer weiteren bevorzugten Ausführungsform der vorliegenden Erfindung ist die Gruppe W ein Indolocarbazol, das ganz bevorzugt eine der folgenden Formeln (W-21) bis (W-25) aufweist.

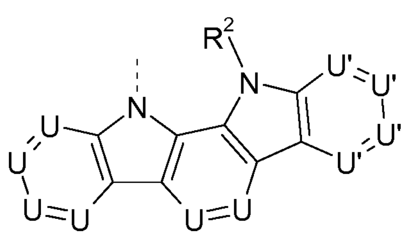

Formel (W-21)

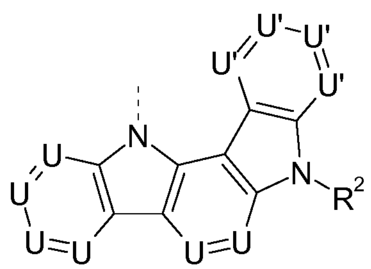

Formel (W-22)

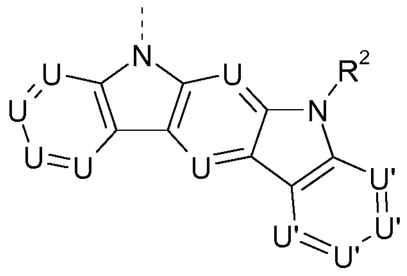

Formel (W-23)

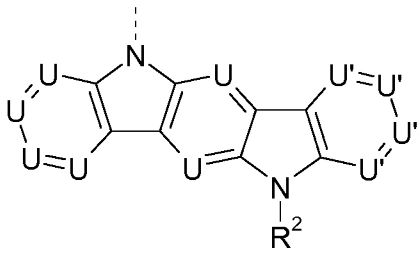

Formel (W-24)

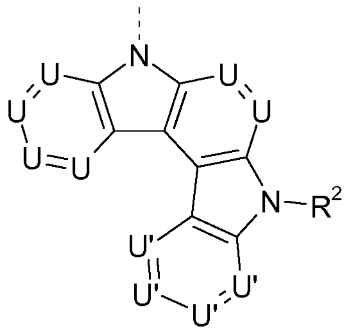

Formel (W-25)

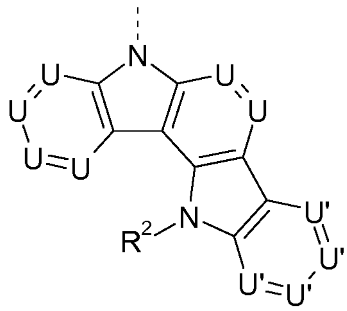

Formel (W-26)

wobei die Indolocarbazole der Formeln (W-21), (W-23) und (W-24) ganz besonders bevorzugt sind. Insbesonders bevorzugte Indolocarbazole sind die der Formeln (W-21) und (W-23), wobei die der Formel (W-21) am meisten bevorzugt sind.

Ganz bevorzugt im Sinne der vorliegenden Erfindung ist, wenn U in den Gruppen der Formeln (W-1) und (W-5) bis (W-26) immer gleich $CR^1$ ist, besonders bevorzugt ist, wenn die zu den Gruppen U = $CR^1$ gehörenden Reste $R^1$ dabei immer H sind.

Ganz bevorzugt im Sinne der vorliegenden Erfindung ist auch, wenn U' in den Gruppen der Formeln (W-5) bis (W-26) immer gleich $CR^2$ ist, besonders bevorzugt ist, wenn die zu den Gruppen U' = $CR^2$ gehörenden Reste $R^2$ dabei immer H sind.

Besonders bevorzugt ist, wenn in den Gruppen der Formeln (W-5) bis (W-26) U gleich $CR^1$ und U' gleich $CR^2$ ist und ganz besonders bevorzugt sind die zu der Gruppen U = $CR^1$ gehörenden Reste $R^1$ sowie die zu den Gruppen U' = $CR^2$ gehörenden Reste $R^2$ gleich H.

In einer weiteren bevorzugten Ausführungsform betrifft die vorliegende Erfindung eine Verbindung der Formel (1) wobei W wie in Formel (W-27) angegeben definiert ist.

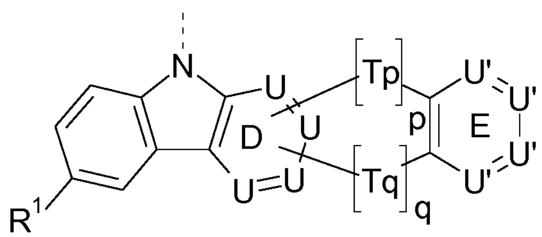

Formel (W-27)

wobei für die verwendeten Indizes und Symbole obige Definitionen gelten und deren an anderer Stelle der vorliegenden Erfindung genannten bevorzugten Ausführungsformen auch bevorzugte Ausführungsformen für die Gruppe der Formel (W-27) darstellen.

Wie bereits erläutert, können die Gruppen X des Rings A der Verbindung der Formel (2) N oder $CR^1$ sein, wobei wenigstens eine der fünf Gruppen X in Ring A ein N-Atom darstellt, bevorzugt sind zwei der fünf Gruppen X in Ring A gleich N und ganz bevorzugt sind drei der fünf Gruppen X in Ring A gleich N;

Bevorzugte Gruppen für den Ring A in Formel (2) der Formel (A-1)

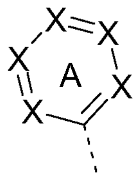

Formel (A-1)

sind solche der folgenden Formeln (A-2) bis (A-6), wobei die gepunktete Linie die Bindung zwischen dem Ring A und Y in Formel (2) bzw., wenn n gleich 0 ist, zwischen dem Ring A und dem Ring B in Formel (2) kennzeichnet.

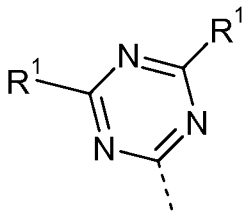

Formel (A-2)

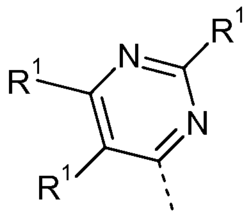

Formel (A-3)

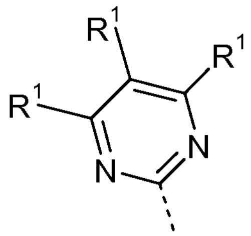

Formel (A-4)

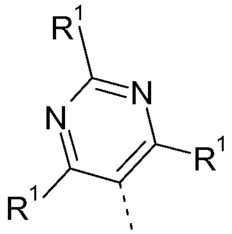

Formel (A-5)

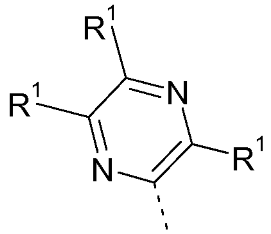

Formel (A-6)

wobei für die verwendeten Symbole und Indizes obige Definitionen und bevorzugte Ausführungsformen gelten.

Besonders bevorzugt ist dabei die Gruppe der Formel (A-2).

Eine weitere Gruppe für den Ring A in Formel (2) stellt die folgende Gruppe der Formel (A-14) dar.

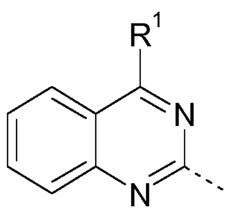

Formel (A-14)

Ganz bevorzugte Gruppen für den Ring A in Formel (2) der Formel (A-1) sind solche der Formeln (A-2) bis (A-6) mit $R^1$ gleich $Ar^1$, wobei $Ar^1$ bei jedem Auftreten gleich oder verschieden ein aromatisches oder heteroaromatisches Ringsystem mit 5 bis 60 aromatischen Ringatomen, das jeweils durch einen oder mehrere Reste $R^2$ substituiert sein kann, ist; bevorzugt ist $Ar^1$ bei jedem Auftreten gleich oder verschieden eine Phenyl-, Biphenyl-, Terphenyl-, Quarterphenyl-, Dibenzofuranyl-, Dibenzothiophenyl-, Fluorenyl- Spirobifluorenyl-, Pyridyl- oder Pyrimidylgruppe, die jeweils durch einen oder mehrere Reste $R^2$ substituiert sein kann; ganz bevorzugt ist $Ar^1$ bei jedem Auftreten gleich oder verschieden eine Phenyl-, Biphenyl-, Terphenyl- oder Quarterphenylgruppe, die jeweils durch einen oder mehrere Reste $R^2$ substituiert sein kann; besonders bevorzugt ist eine Phenylgruppe, die jeweils durch einen oder mehrere Reste $R^2$

substituiert sein kann, wobei insbesondere bevorzugt ist, wenn die Phenylgruppe unsubstituiert vorliegt.

Ganz besonders bevorzugt ist dabei die Gruppe der Formel (A-2) mit $R^1$ gleich $Ar^1$.

Der Ring A in Formel (2) kann auch die Gruppe der Formel (A-14) mit $R^1$ gleich $Ar^1$ darstellen.

Besonders bevorzugte Gruppen $Ar^1$ stellen auch die folgenden Gruppen mit den Formeln (Ar-1) bis (Ar-24) dar, wobei die Gruppen mit einem oder mehreren Resten $R^2$, die gleich oder verschieden bei jedem Auftreten sein können, substituiert sein können. Ganz besonders bevorzugte Gruppe $Ar^1$ stellen die der Formeln (Ar-1) bis (Ar-9) dar.

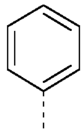
Formel (Ar-1)

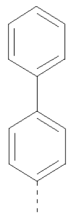
Formel (Ar-2)

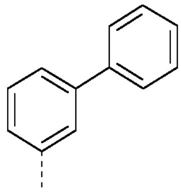
Formel (Ar-3)

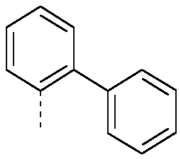
Formel (Ar-4)

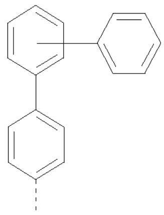
Formel (Ar-6)

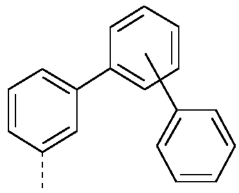
Formel (Ar-7)

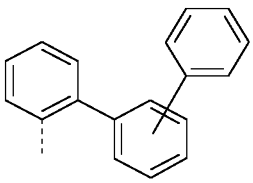
Formel (Ar-8)

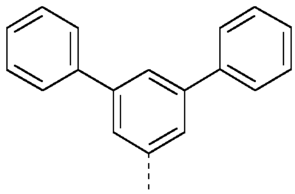
Formel (Ar-9)

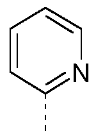
Formel (Ar-10)

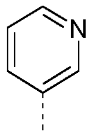
Formel (Ar-11)

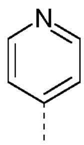
Formel (Ar-12)

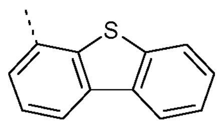
Formel (Ar-13)

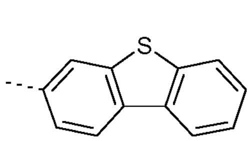
Formel (Ar-14)

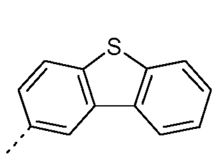
Formel (Ar-15)

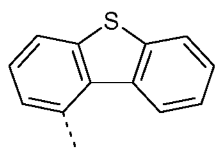
Formel (Ar-16)

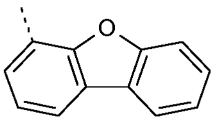

Formel (Ar-17)

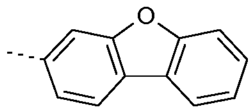

Formel (Ar-18)

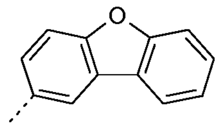

Formel (Ar-19)

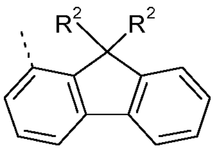

Formel (Ar-20)

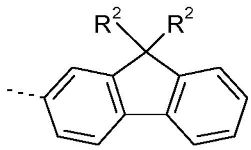

Formel (Ar-21)

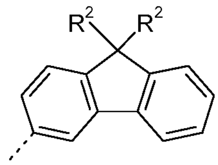

Formel (Ar-22)

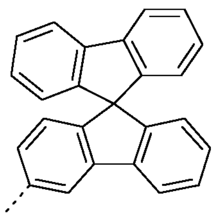

Formel (Ar-23)

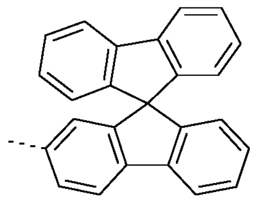

Formel (Ar-24)

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

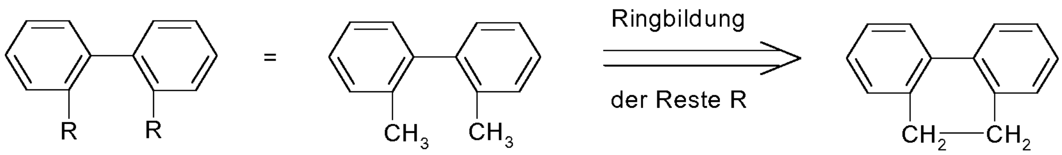


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

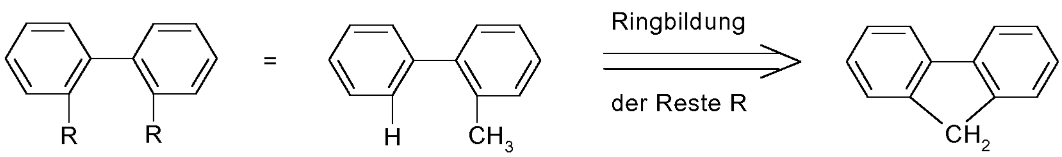


Es folgen allgemeine Definitionen für chemische Gruppen im Rahmen der vorliegenden Anmeldung:
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen

bzw. heteroaromatischen Cyclen.

Eine elektronenarme Heteroarylgruppe im Sinne der vorliegenden Erfindung ist definiert als 5-Ring-Heteroarylgruppe mit mindestens zwei Heteroatomen, beispielsweise Imidazol, Oxazol, Oxadiazol, etc., oder als 6-Ring-Heteroarylgruppe mit mindestens einem Heteroatom, beispielsweise Pyridin, Pyrimidin, Pyrazin, Triazin, etc.. Dabei können an diese Gruppen auch noch weitere 6-Ring-Aryl- oder 6-Ring-Heteroarylgruppen ankondensiert sein, wie dies beispielsweise in Benzimidazol, Chinolin oder Phenanthrolin der Fall ist.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein $sp^3$-hybridisiertes C-, Si-, N- oder O-Atom, ein $sp^2$-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Die erfindungsgemäßen Verbindungen können gemäß Schema 1 dargestellt werden. Die entsprechenden Monoboronsäuren sind kommerziell erhältlich und können durch Suzuki-Kupplung und anschließende Bromierung sowie weitere Umsetzung durch Buchwald-Kupplung zu den entsprechenden Zielmolekülen umgesetzt werden.

## Schema 1

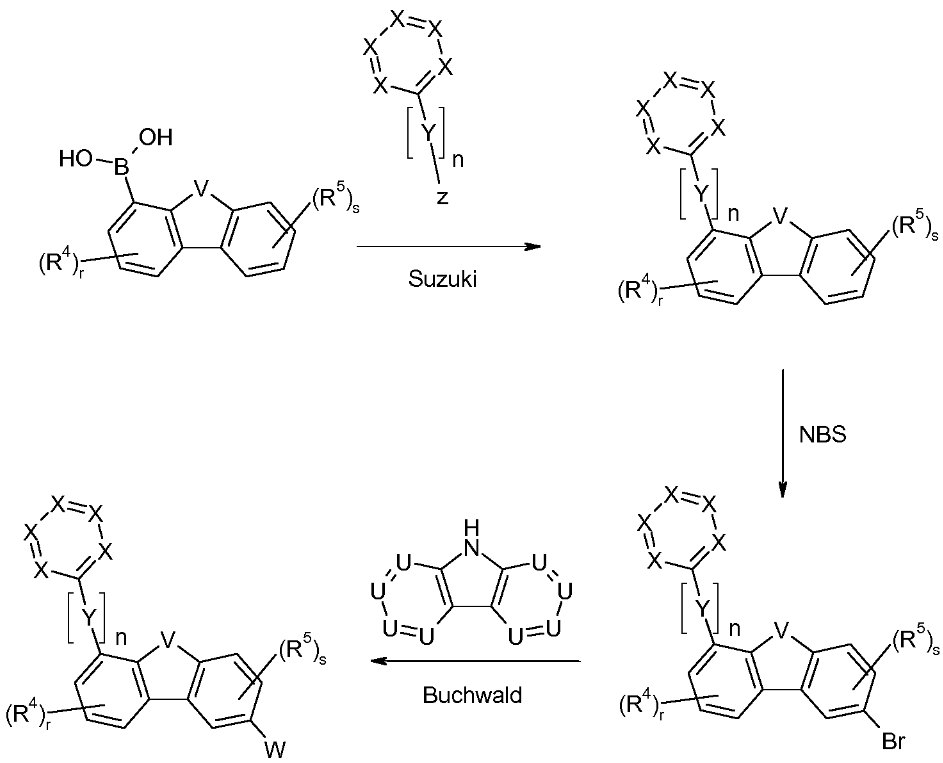


wobei Z gleich Cl, Br oder I ist und die anderen Symbole und Indizes die oben angegebenen Bedeutungen haben. Das Reaktionsschema lässt sich auch ganz analog zur Herstellung der Verbindungen gemäß Formel (1) anwenden.

Das gezeigte, allgemeine Verfahren zur Synthese der erfindungsgemäßen Verbindungen ist exemplarisch. Der Fachmann kann alternative Synthesewege im Rahmen seines allgemeinen Fachwissens entwickeln.

Die folgende Übersicht enthält eine beispielhafte Darstellung erfindungsgemäßer und nicht-erfindungsgemäßer Verbindungen, die nach einem der hierin beschriebenen Verfahren hergestellt werden können.

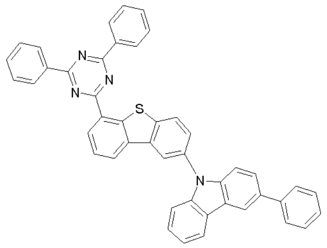
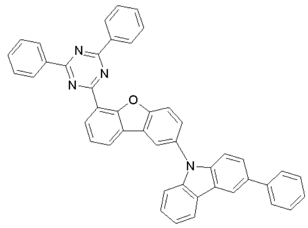
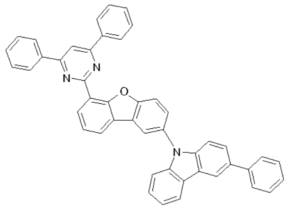

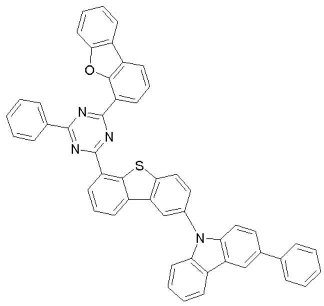
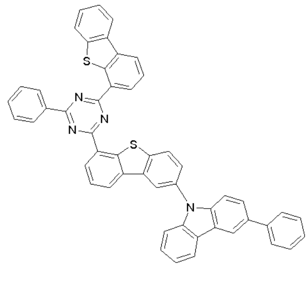
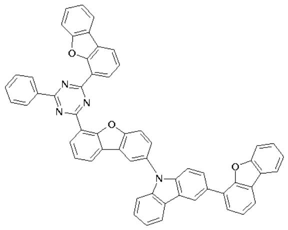

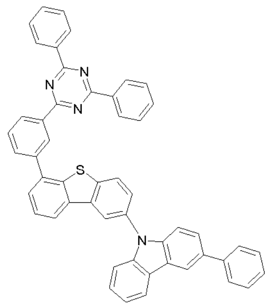
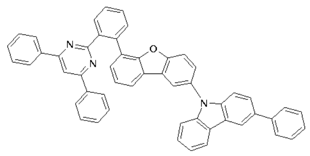
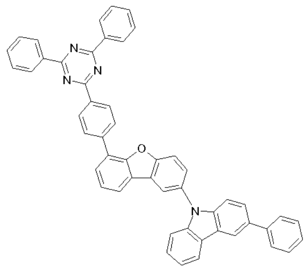

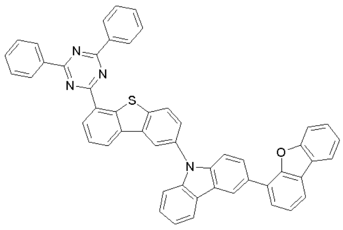
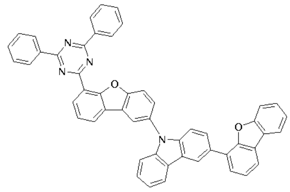
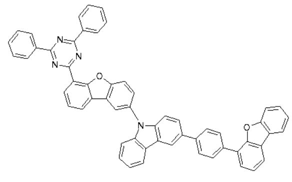

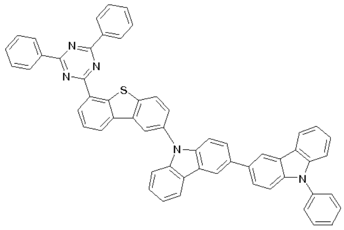
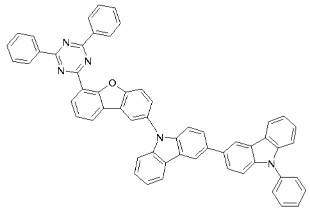
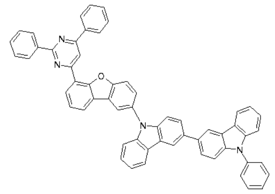

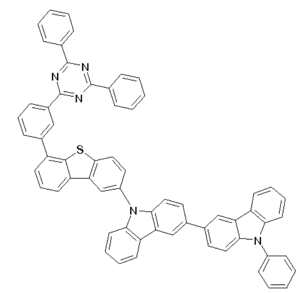
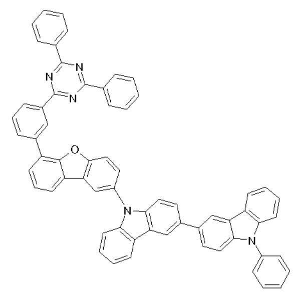
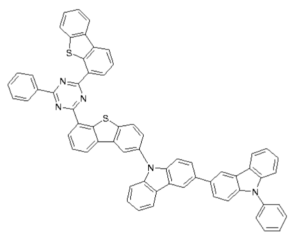
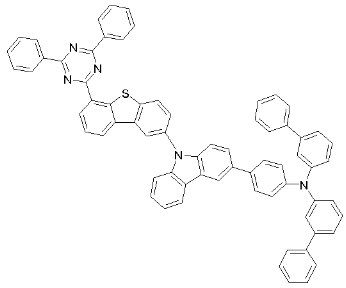
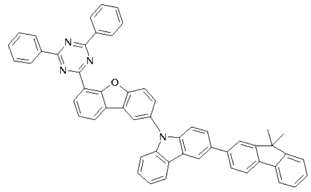
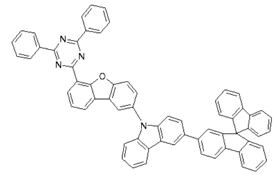
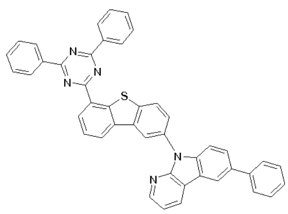
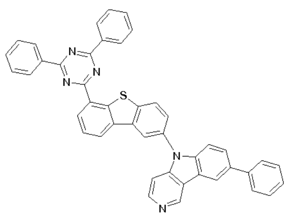
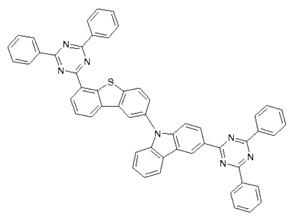
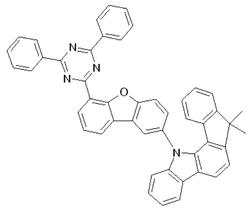
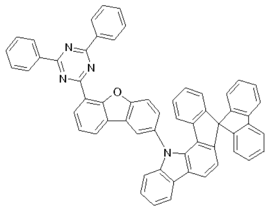
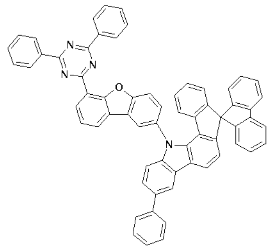
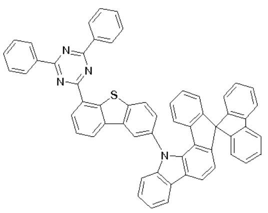
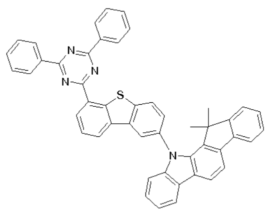
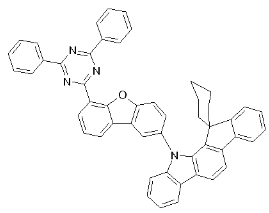
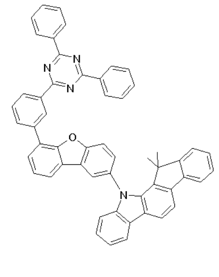
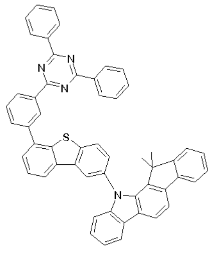
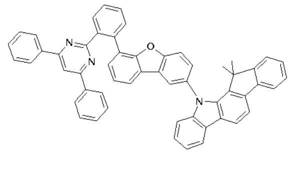

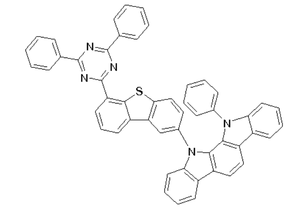 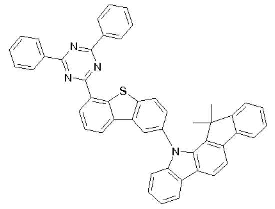 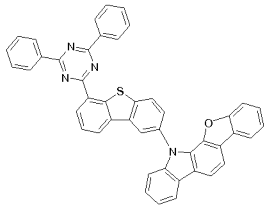

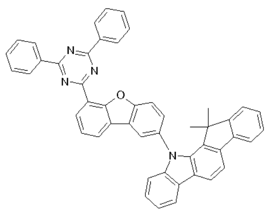 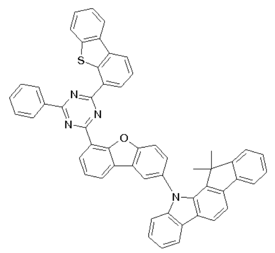 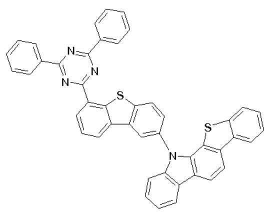

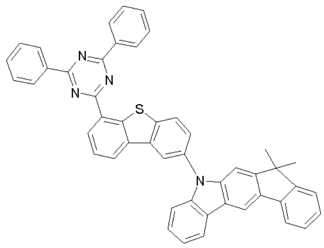 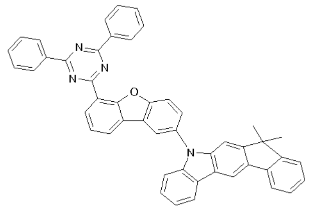 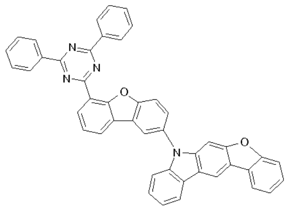

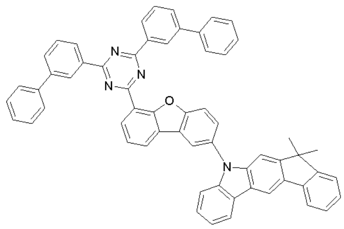 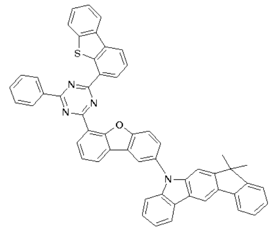 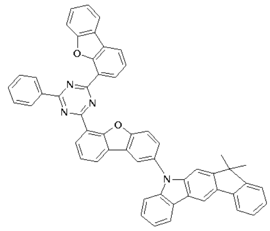

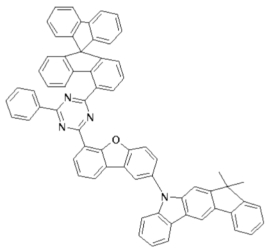 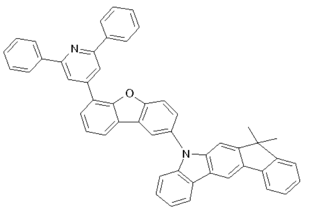 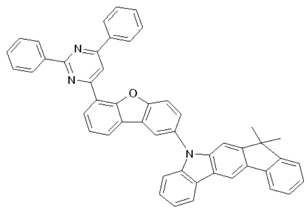

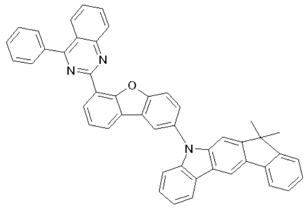 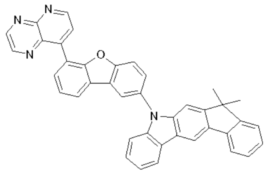 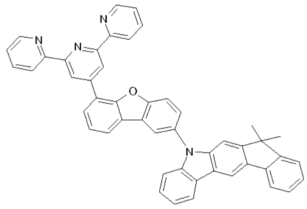

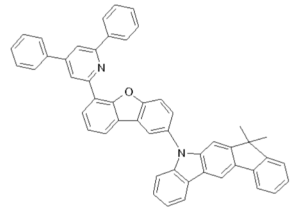
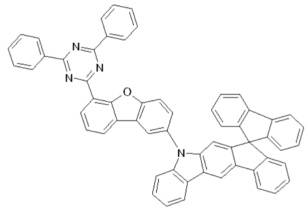
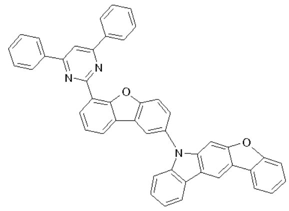

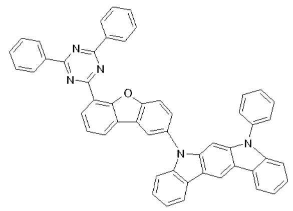
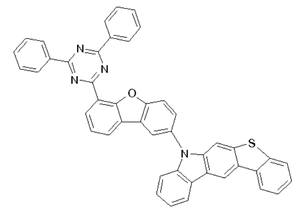
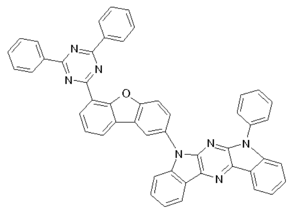

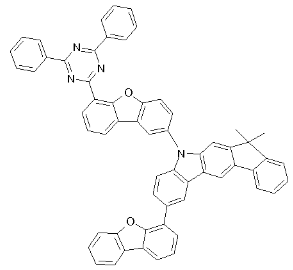
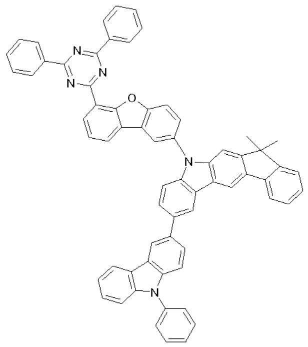
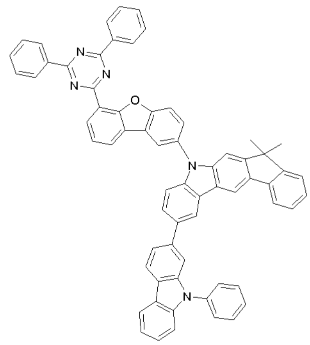

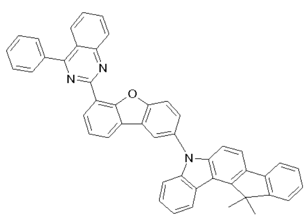
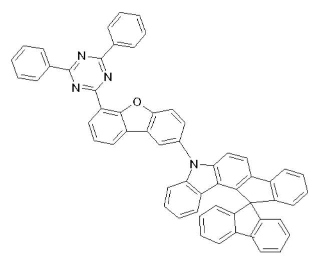
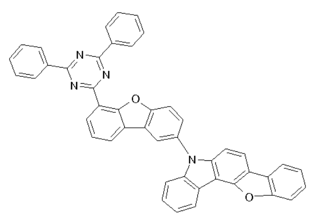

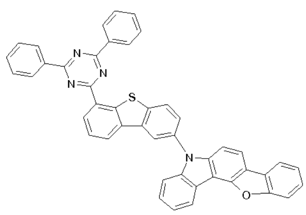
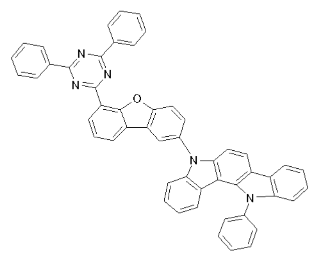
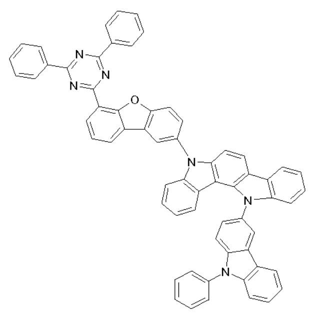

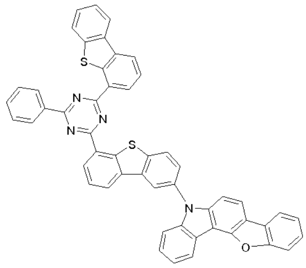

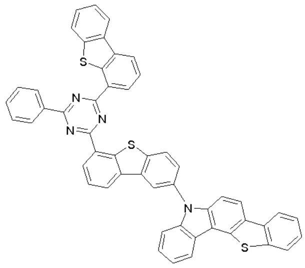

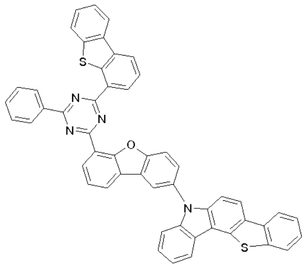

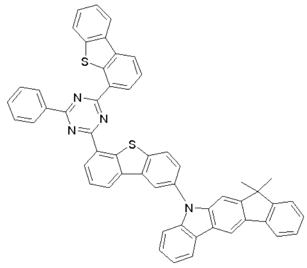

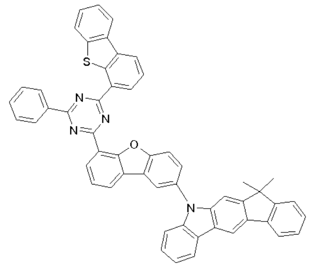

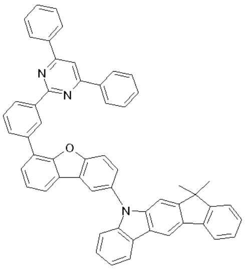

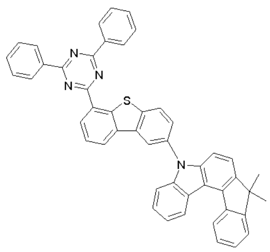

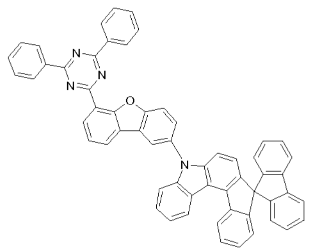

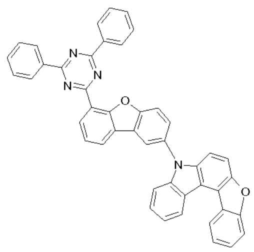

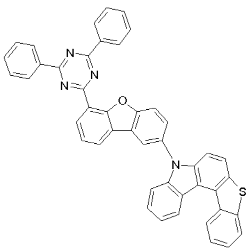

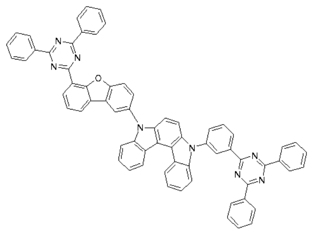

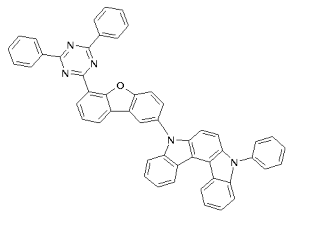

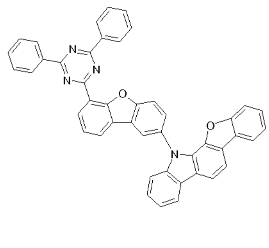

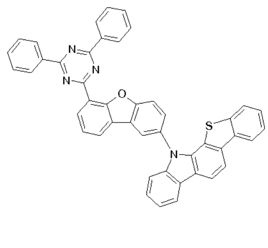

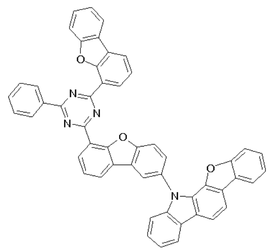

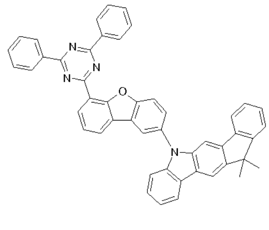

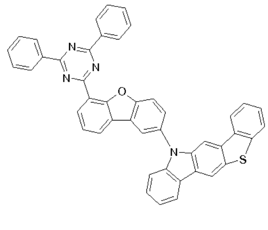

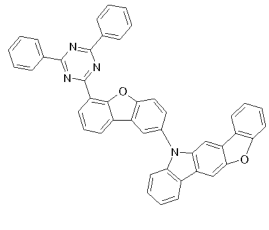

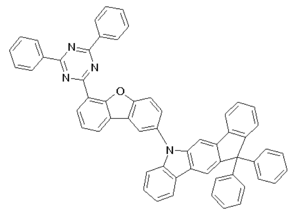
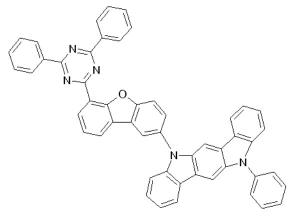
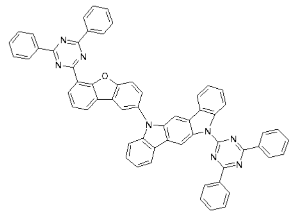

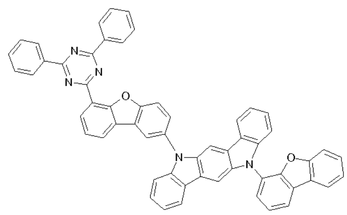
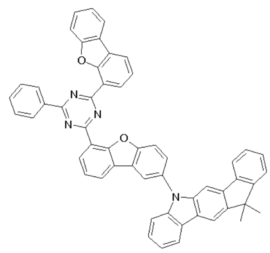
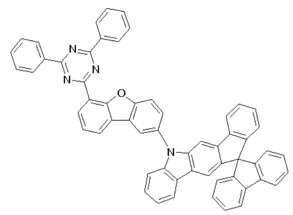

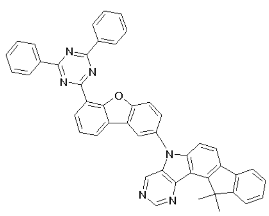
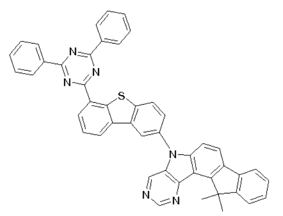
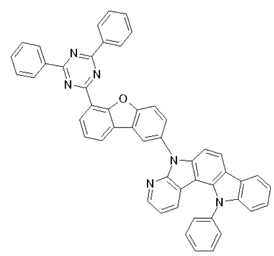

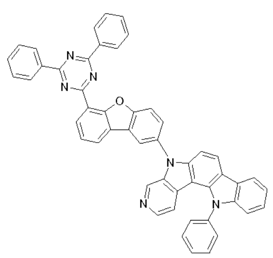
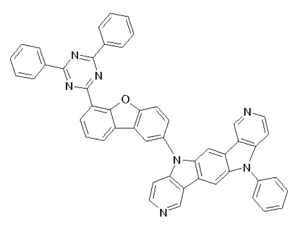
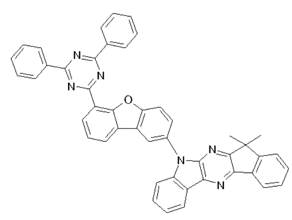

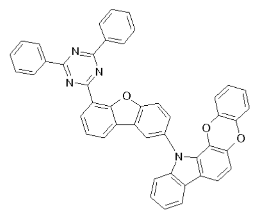
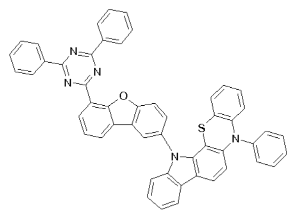
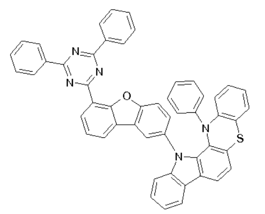

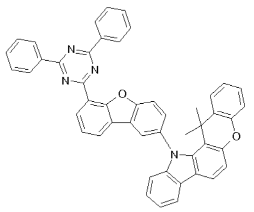
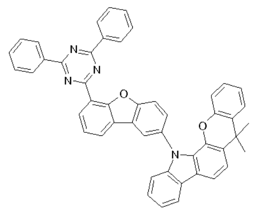
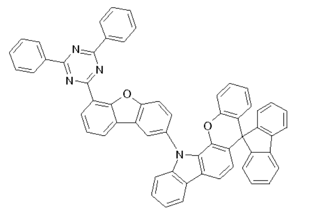

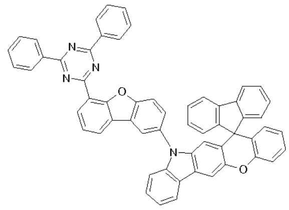
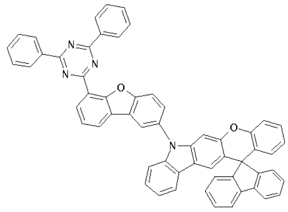
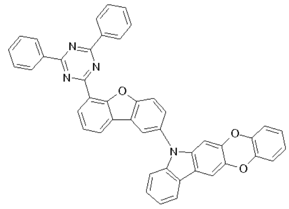

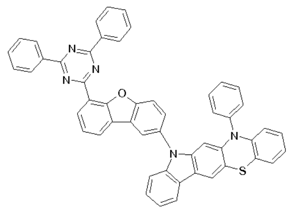
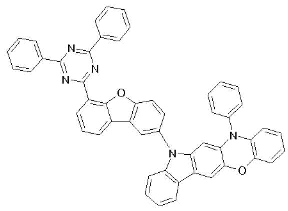
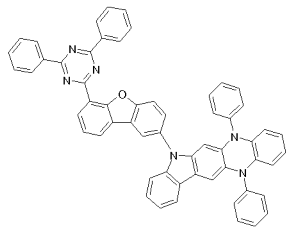

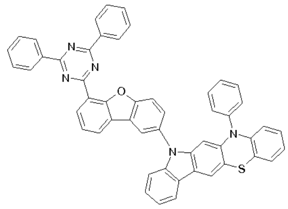
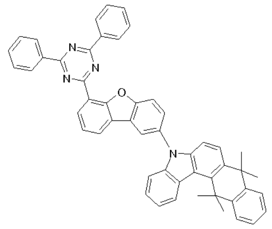
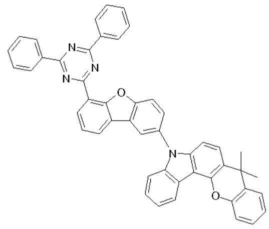

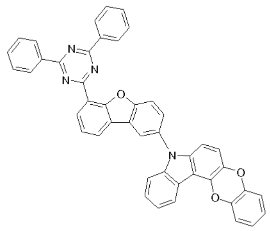
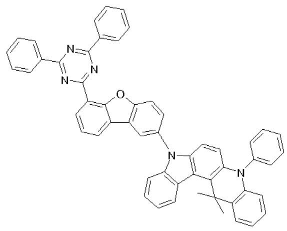
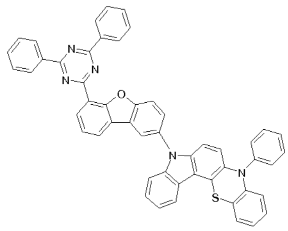

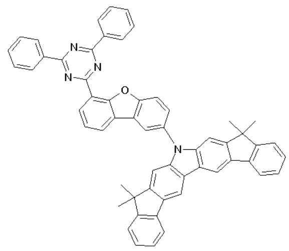
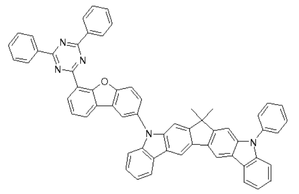
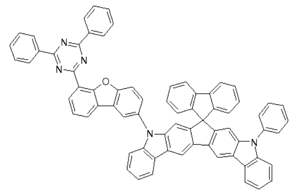

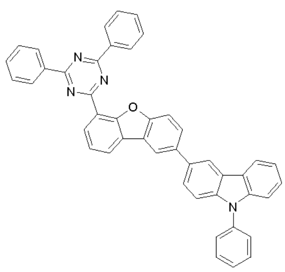
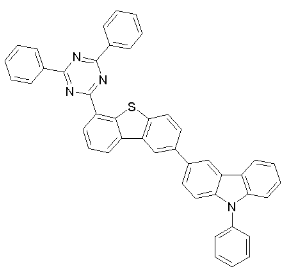
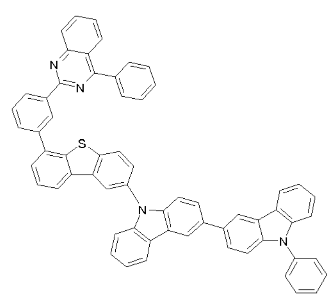

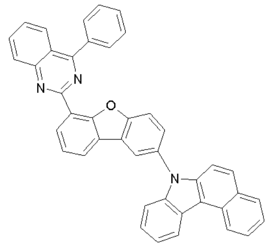
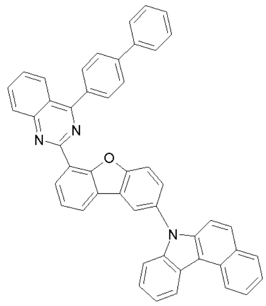
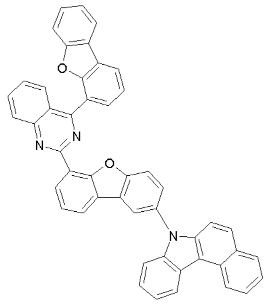

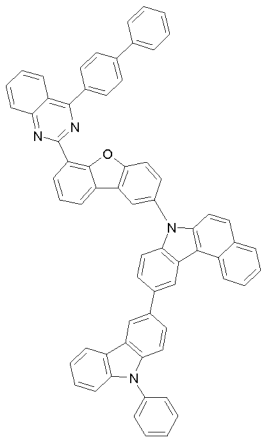
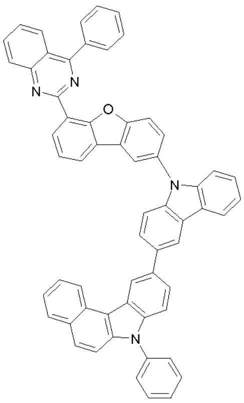
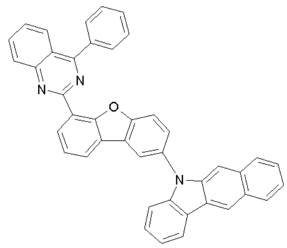

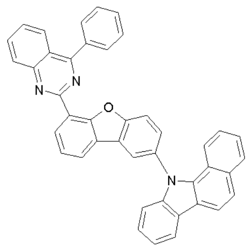
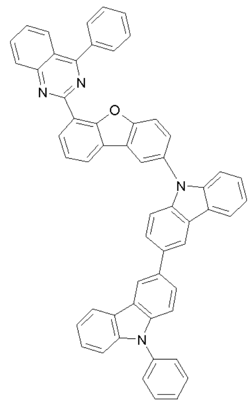
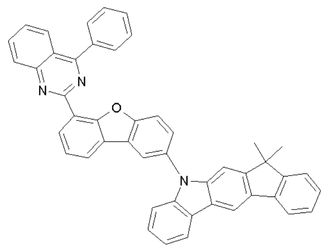

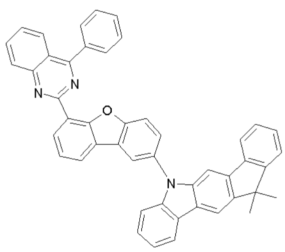
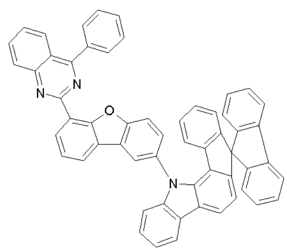
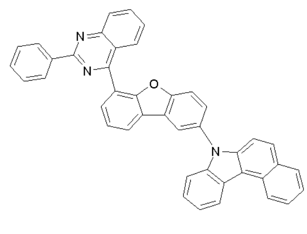

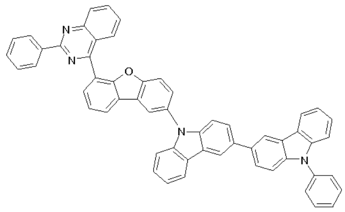

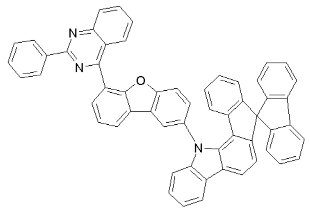

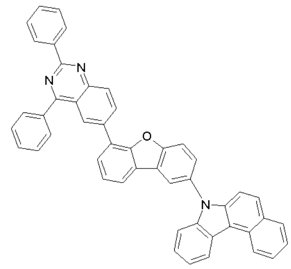

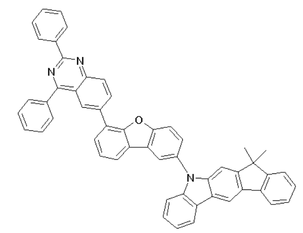

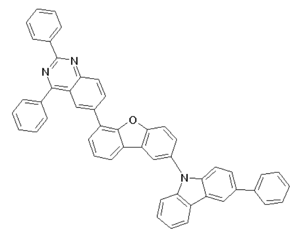

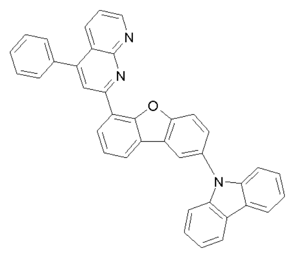

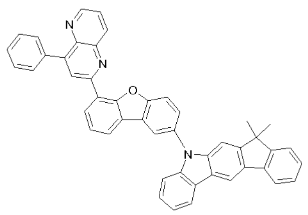

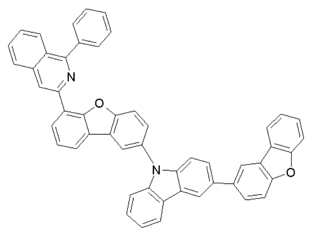

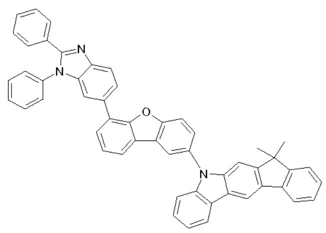

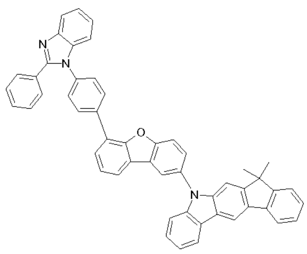

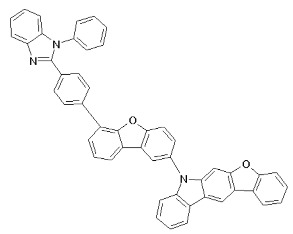

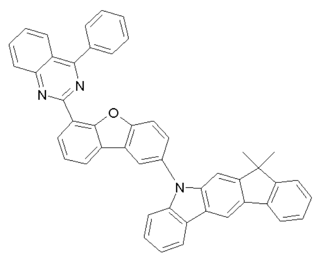

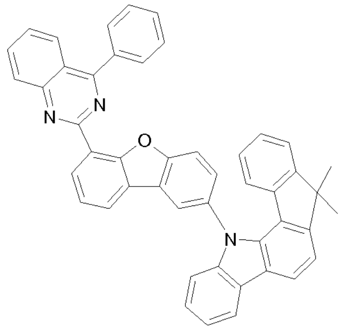

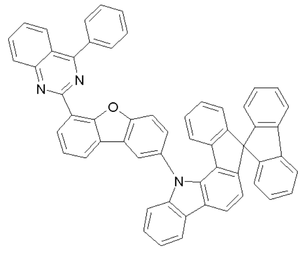

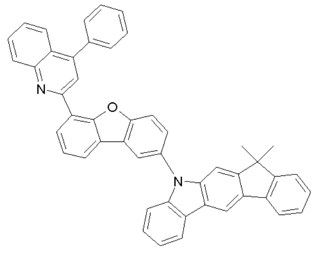

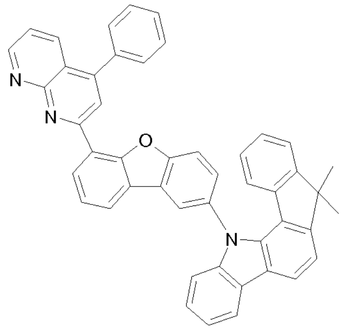

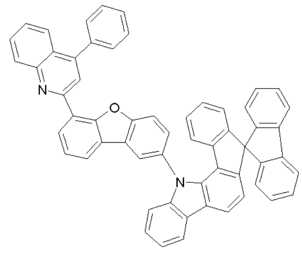

Weiterer Gegenstand der Erfindung ist die Verwendung einer Verbindung der Formel (1) in einer elektronischen Vorrichtung, bevorzugt in einer elektronentransportierenden und/oder in einer emittierenden Schicht.

Die erfindungsgemäßen Verbindungen können auch in einer Lochtransportschicht verwendet werden.

Die erfindungsgemäße elektronische Vorrichtung ist bevorzugt ausgewählt aus der Gruppe bestehend aus den organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs, LECs oder LEECs), organischen Laserdioden (O-Laser) und organischen lichtemittierenden Dioden (OLEDs). Besonders bevorzugt sind dabei die organischen Elektrolumineszenzvorrichtungen, ganz besonders bevorzugt die OLECs und OLEDs und insbesondere bevorzugt die OLEDs.

Unter OLEDs im Sinne der vorliegenden Erfindung werden sowohl organische lichtemittierende Dioden enthaltend kleine organische Moleküle (SMOLEDs) als auch polymere lichtemittierende Dioden (PLEDs) verstanden, wobei SMOLEDs bevorzugte OLEDs darstellen.

Die organische Schicht enthaltend die Verbindung der Formel (1) ist bevorzugt eine Schicht mit elektronentransportierender Funktion. Besonders bevorzugt ist sie eine Elektroneninjektionsschicht (EIL), Elektronentransportschicht (ETL), eine Lochblockierschicht (HBL)oder eine emittierende Schicht (EML), wobei noch bevorzugter ist, wenn diese Verbindung in einer emittierenden Schicht vorliegt, insbesondere als Matrixmaterial.

Eine Lochtransportschicht gemäß der vorliegenden Anmeldung ist eine Schicht mit lochtransportierender Funktion, welche sich zwischen Anode und emittierender Schicht befindet.

Eine Elektronenransportschicht gemäß der vorliegenden Anmeldung ist eine Schicht mit elektronentransportierender Funktion, welche sich zwischen Kathode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wie oben bereits erwähnt, wird die Verbindung der Formel (1) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt. Dabei ist das Matrixmaterial der Formel (1) in der elektronischen Vorrichtung in Kombination mit einem oder mehreren Dotanden, vorzugsweise phosphoreszierenden Dotanden, vorhanden.

Vom Begriff phosphoreszierende Dotanden sind typischerweise Verbindungen umfasst, bei denen die Lichtemission durch einen spinverbotenen Übergang erfolgt, beispielsweise durch einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Anmeldung alle lumineszierenden Iridium-, Platin- oder Kupferkomplexe als phosphoreszierende Verbindungen angesehen. Beispiele für phosphoreszierende Dotanden sind in einem folgenden Abschnitt aufgeführt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial

in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß Formel (1) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Die vorliegenden Erfindung betrifft daher auch eine Zusammensetzung enthaltend wenigstens eine Verbindung gemäß Formel (1) sowie wenigstens ein weiteres Matrixmaterial. Bevorzugte weitere Matrixmaterialien sind die weiter unten genannten Matrixmaterialien.

Die vorliegende Erfindung betrifft auch eine Zusammensetzung enthaltend wenigstens eine Verbindung gemäß Formel (1) sowie wenigstens ein wide band gap Material, wobei unter wide band gap Material ein Material im Sinne der Offenbarung von US 7,294,849 verstanden wird. Diese Systeme zeigen besondere vorteilhafte Leistungsdaten in elektrolumineszierenden Vorrchtungen. Es ist besonders bevorzugt, wenn die Bandlücke des wide band gap Materials 3.5 eV oder mehr beträgt, wobei unter Bandlücke der energetische Abstand zwischen HOMO und LUMO verstanden wird. Die Oribtalenergien werden nach dem oben beschriebenen Verfahren ermittelt.

Weiterhin betrifft die vorliegende Erfindung eine Zusammensetzung enthaltend wenigstens eine Verbindung gemäß Formel (1) sowie wenigstens ein weiteres organisches Halbleitermaterial ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterial ien.

Bevorzugte phosphoreszierende Dotanden zur Verwendung in Mixed-Matrix-Systemen sind die im Folgenden angebenen bevorzugten phosphoreszierenden Dotanden.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen.

Explizite Beispiele für phosphoreszierende Dotanden sind in der folgenden Tabelle aufgeführt.

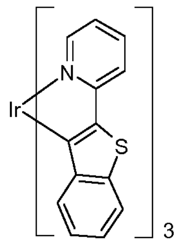
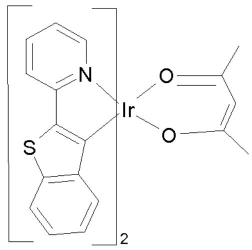
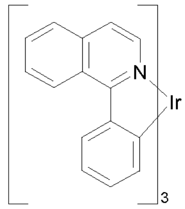
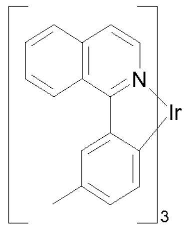
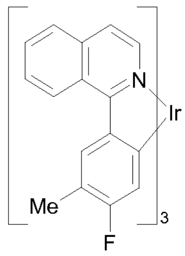
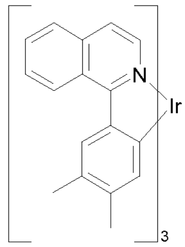
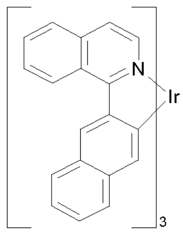
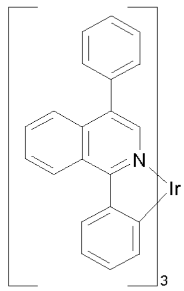
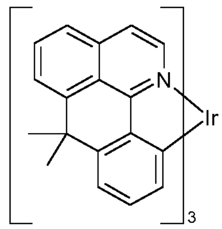
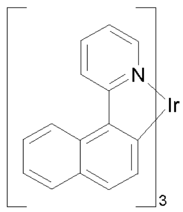
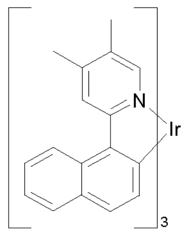
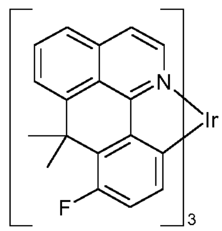
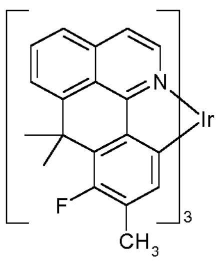
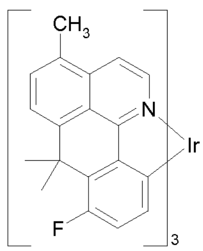
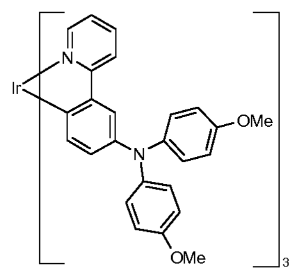

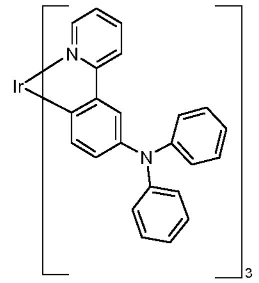
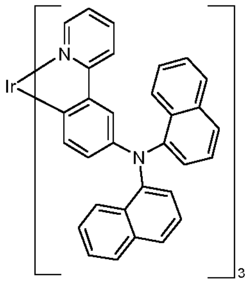
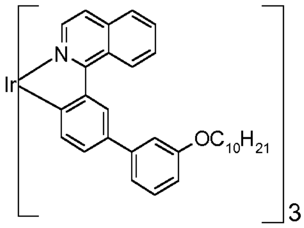

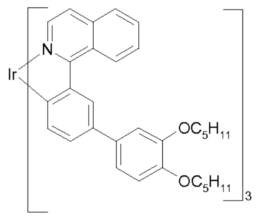
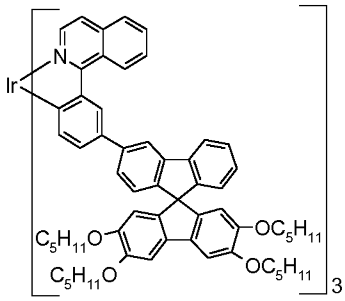
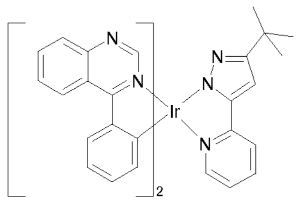

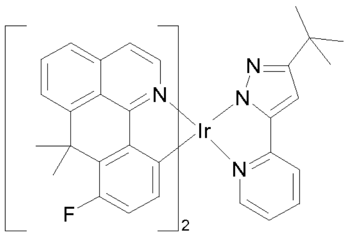
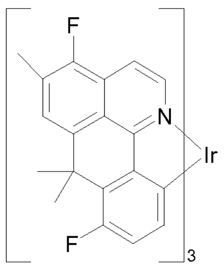
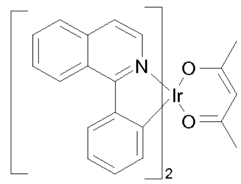

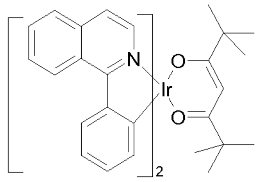
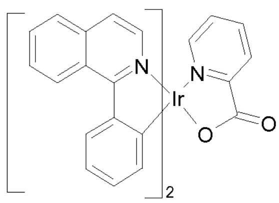
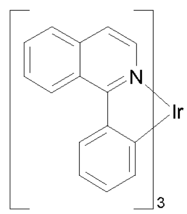

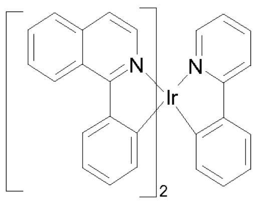
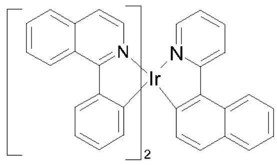
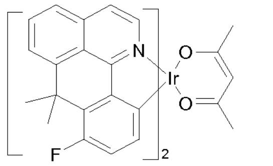

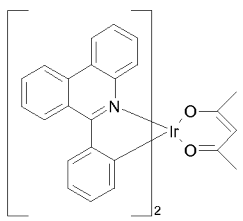
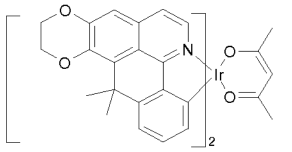
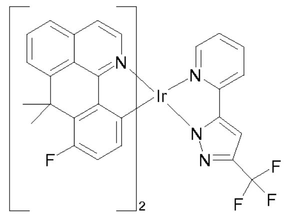

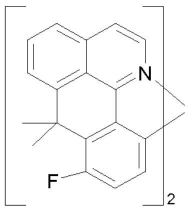
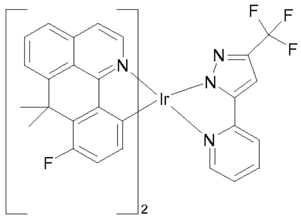
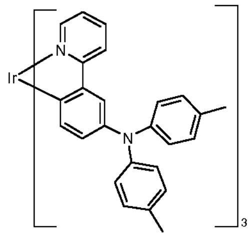

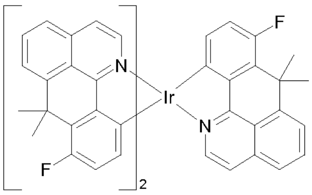
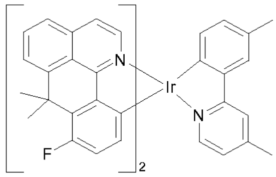
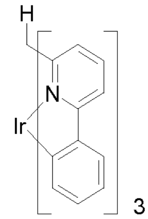

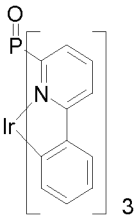
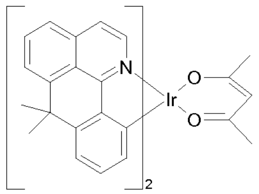
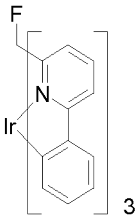

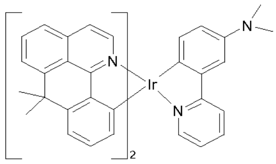
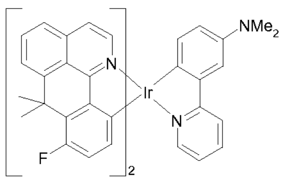
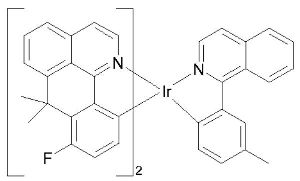

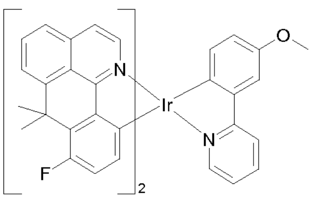
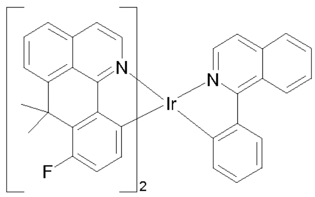
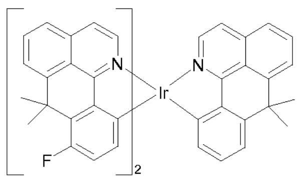

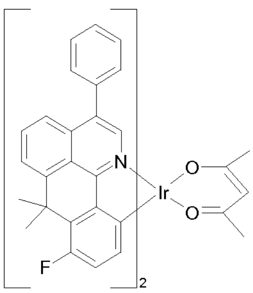
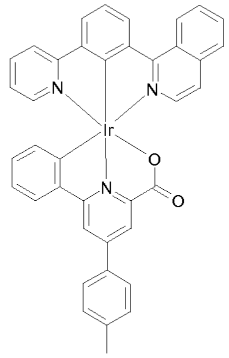
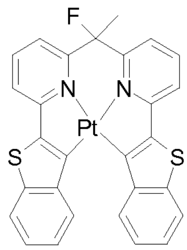

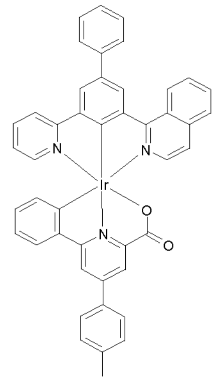
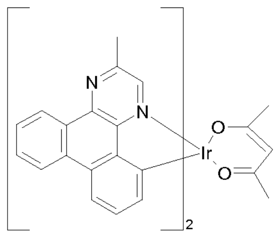
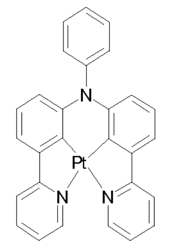

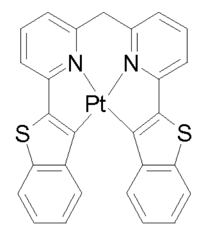
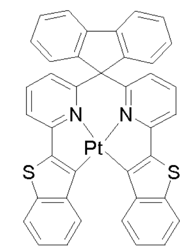
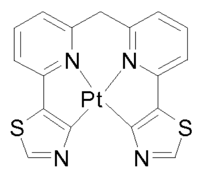

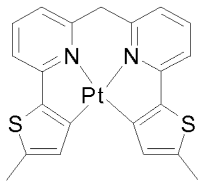
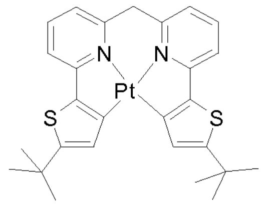
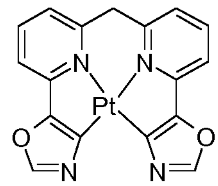

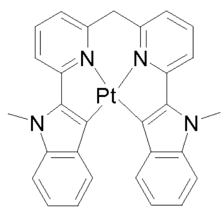
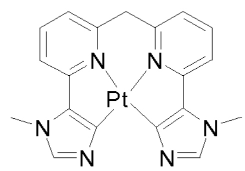
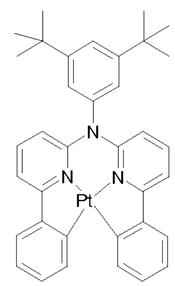

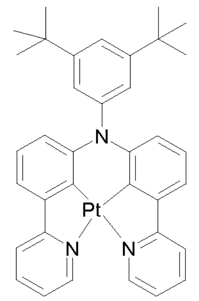
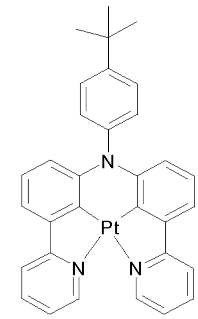
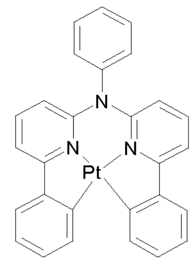

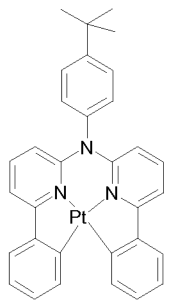

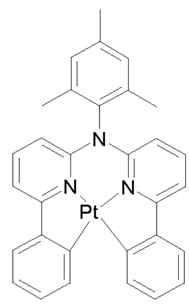

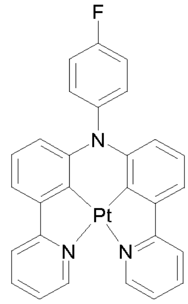

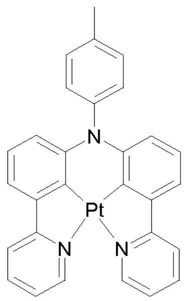

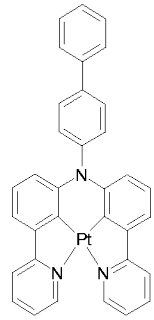

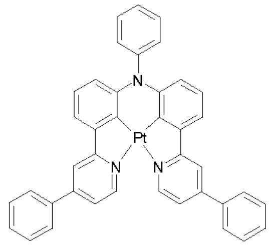

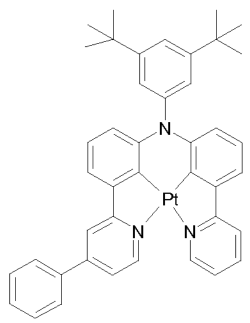

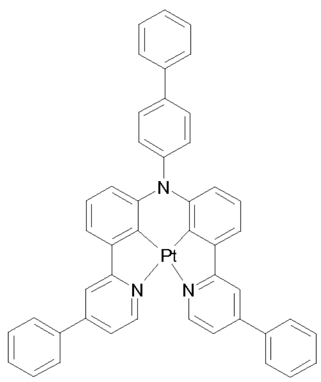

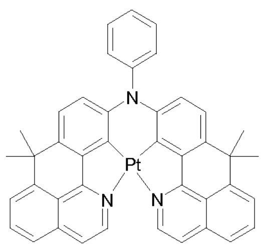

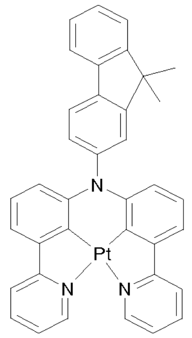

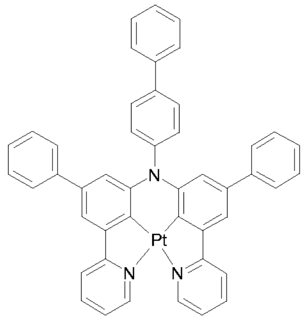

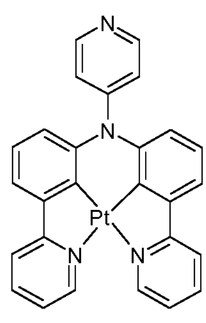

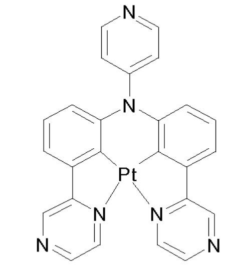
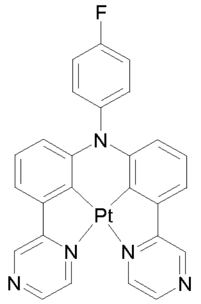
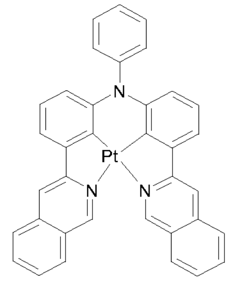

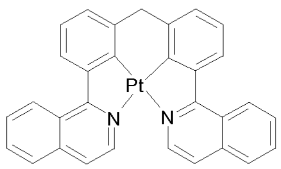
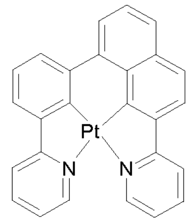
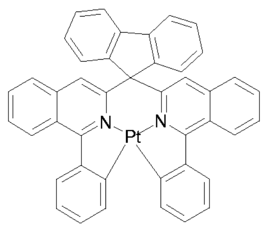

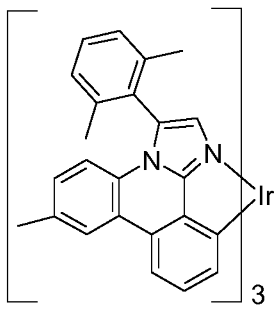
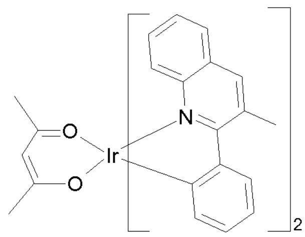
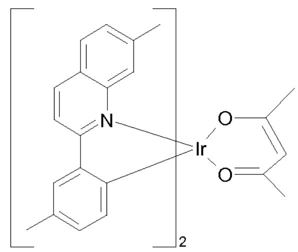

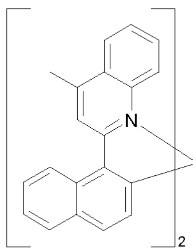
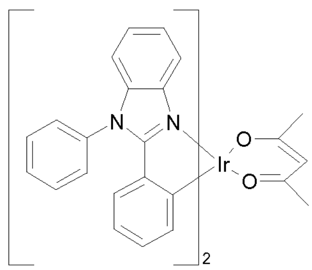
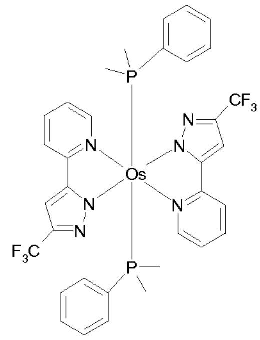

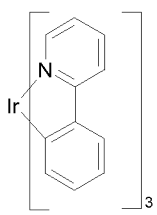
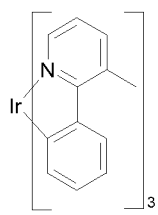
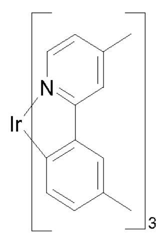

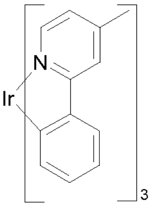
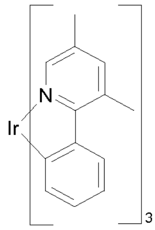
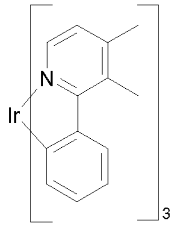

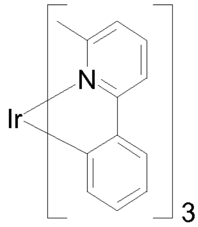
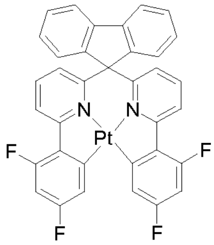
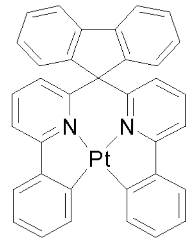

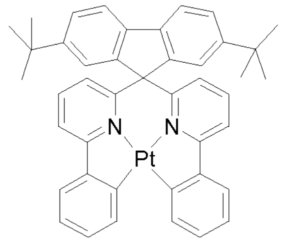
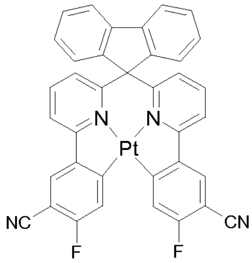
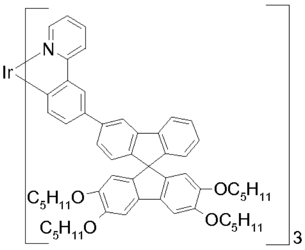

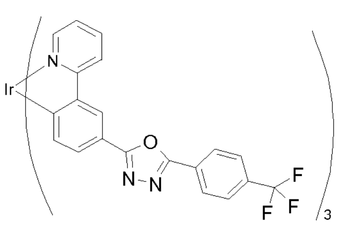
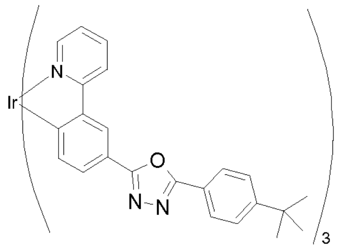
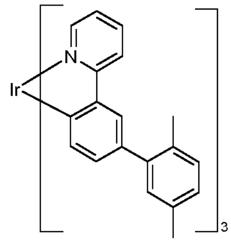

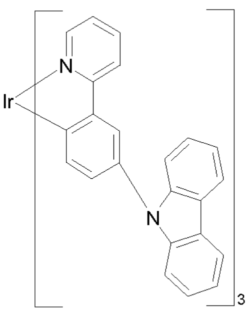
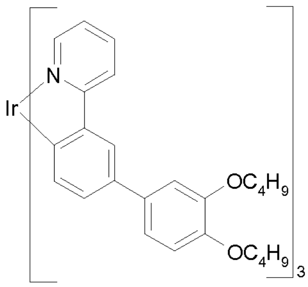
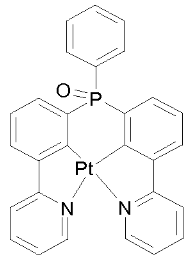

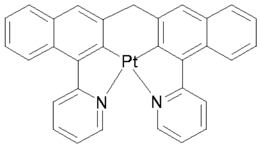
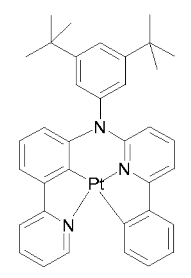
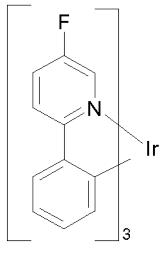

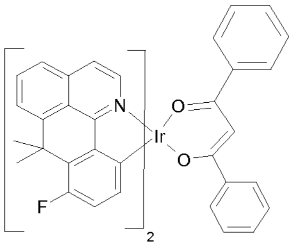
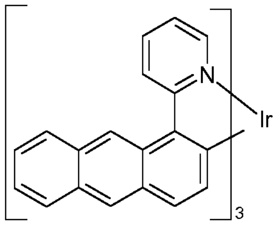
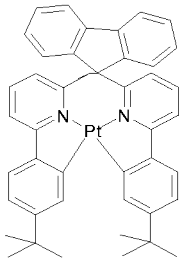

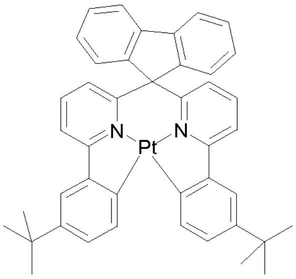
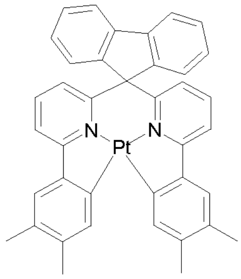
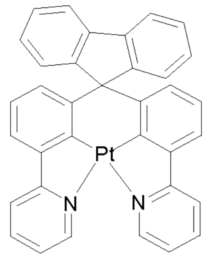

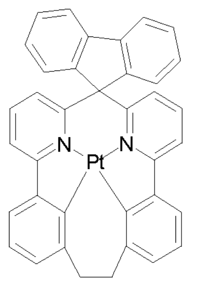
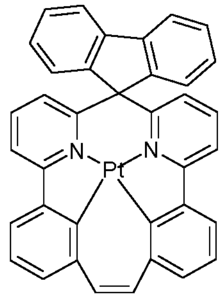
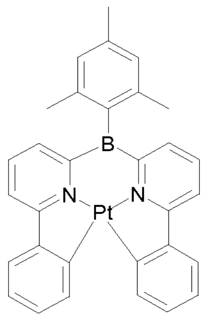

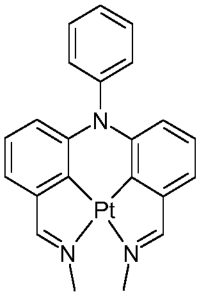
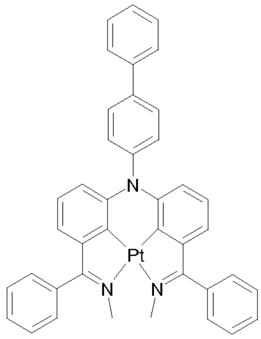
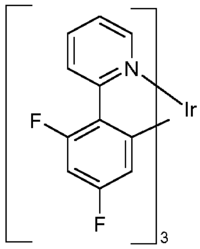

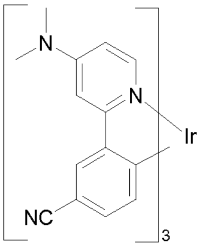
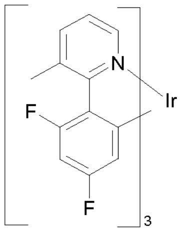
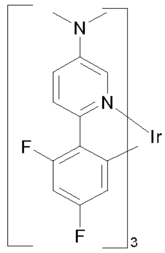

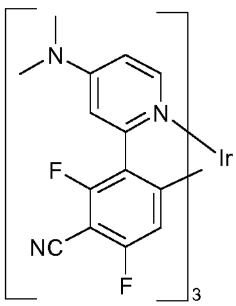
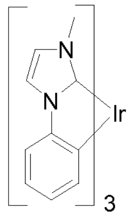
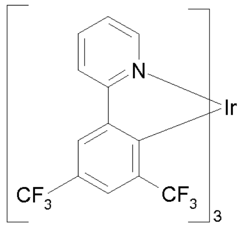

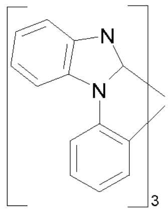
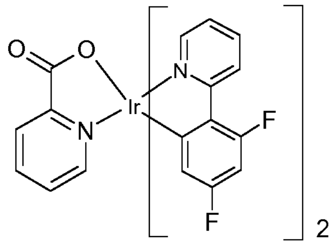
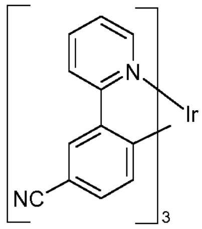

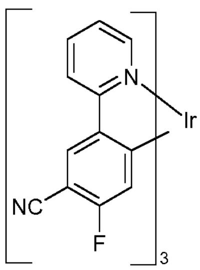
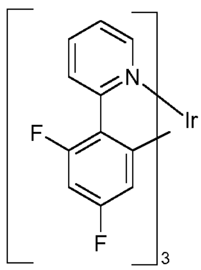
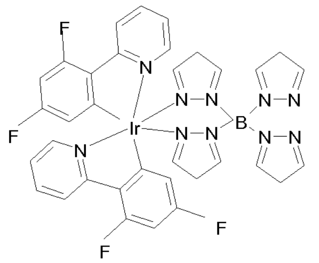

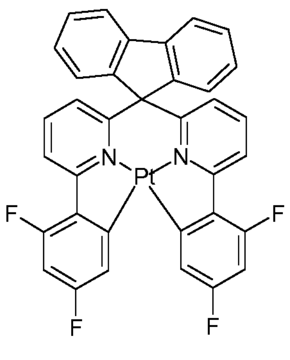
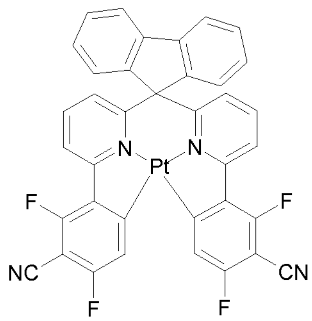
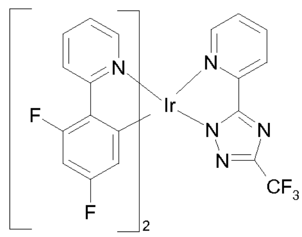

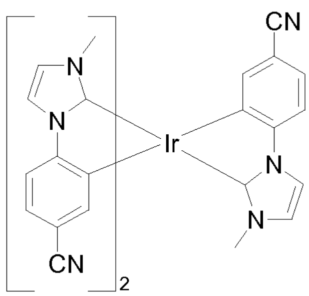
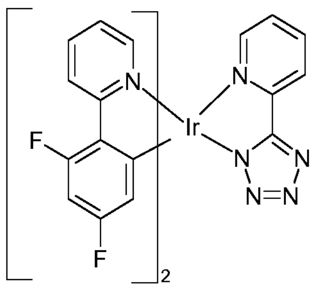
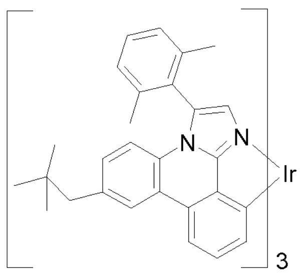

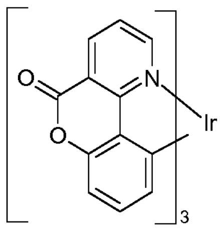
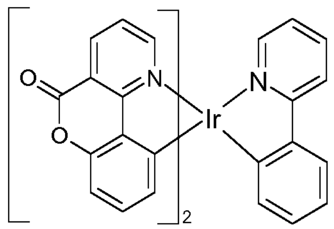
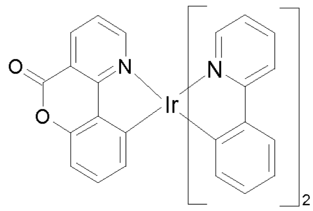

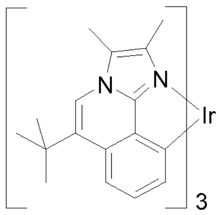
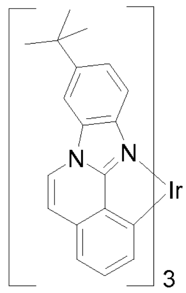
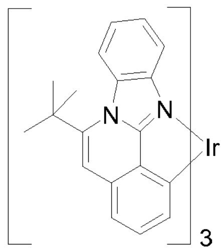

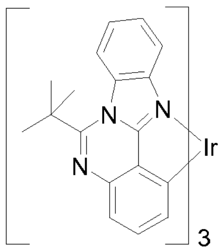
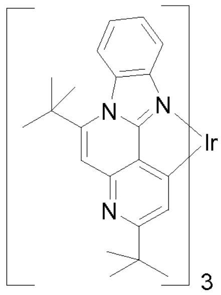
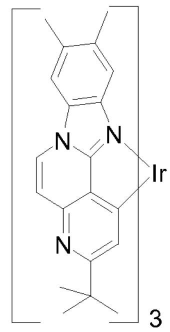

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Als Matrixmaterialien, bevorzugt für fluoreszierende Dotanden, kommen neben den Verbindungen der Formel (1) Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den

Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den Verbindungen der Formel (1) aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Außer Kathode, Anode und der Schicht enthaltend die Verbindung der Formel (1) kann die elektronische Vorrichtung noch weitere Schichten enthalten. Diese sind beispielsweise gewählt aus jeweils einer oder mehreren Lochinjektionsschichten, Lochtransportschichten, Lochblockierschichten, emittierenden Schichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Lochtransportmaterialien sind insbesondere bevorzugt Materialien, die in einer Lochtransport-, Lochinjektions- oder Elektronenblockierschicht verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß der noch nicht offengelegten Anmeldung EP 11009127.9) und Dihydroacridin-Derivate (z. B. gemäß der noch nicht offen gelegten EP 11007067.9).

Als Kathode der elektronischen Vorrichtung sind Metalle mit geringer Austrittsarbeit, Metalllegierungen oder

mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die elektronische Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Gegenstand der Erfindung ist somit weiterhin ein Verfahren zur Herstellung der erfindungsggemäßen elektronischen Vorrichtung, dadurch gekennzeichnet, dass mindestens eine organische Schicht durch Gasphasenabscheidung oder aus Lösung aufgebracht wird.

Erfindungsgemäß können die elektronischen Vorrichtungen enthaltend eine oder mehrere Verbindungen gemäß Formel (1) in Displays, als Lichtquellen in Beleuchtungsanwendungen sowie als Lichtquellen in medizinischen und/oder kosmetischen Anwendungen (z.B. Lichttherapie) eingesetzt werden.

Die vorliegende Erfindung betrifft auch eine Formulierung enthaltend wenigstens eine Verbindung gemäß Formel (1) oder wenigstens eine der oben genannten Zusammensetzungen sowie wenigstens ein Lösungsmittel.

Geeignete und bevorzugte Lösungsmittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-tetramethylbenzol, 1-Methylnaphtalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzyl ether, Diethylenglycolbutylmethylether, Triethylenglycolbutylmethylether, Diethylenglycoldibutylether, Triethylenglycoldimethylether, Diethylen-glycolmonobutylether, Tripropylenglycoldimethylether, Tetraethylenglycol-dimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser

Lösungsmittel.

Vorrichtungen enthaltend die Verbindungen nach Formel (1) können sehr vielseitig eingesetzt werden. So können, beispielsweise, elektrolumineszierende Vorrichtuungen enthaltend eine oder mehrere Verbindungen nach Formel (1) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach Formel (1) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Gegenstand der vorliegenden Erfindung ist daher eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (1) zur Verwendung in der Medizin zur Phototherapie.

Ein weiterer bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (1) zur Verwendung zur phototherapeutischen Behandlung von Hautkrankheiten.

Ein weiterer ganz bevorzugter Gegenstand der vorliegenden Erfindung betrifft eine elektronische Vorrichtung, bevorzugt eine organische Elektrolumineszenzvorrichtung, ganz bevorzugt eine OLED oder OLEC und ganz besonders bevorzugt eine OLED enthaltend wenigstens eine Verbindung gemäß Formel (1) zur Verwendung zur phototherapeutischen Behandlung von Psoriasis, atopische Dermatitis, Entzündungserkrankungen, Vitiligo, Wundheilung und Hautkrebs.

Die vorliegende Erfindung betrifft weiterhin die Verwendung der elektronischen Vorrichtung, bevorzugt einer organischen Elektrolumineszenzvorrichtung, ganz bevorzugt einer OLED oder OLEC und ganz besonders bevorzugt einer OLED enthaltend wenigstens eine Verbindung gemäß Formel (1) in der Kosmetik, bevorzugt zur Behandlung von Akne, alternder Haut (Skin Ageing), und von Zellulithe.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:

1. Die erfindungsgemäßen Verbindungen eignen sich sehr gut für den Einsatz in einer Emissionsschicht und zeigen verbesserte Leistungsdaten gegenüber Verbindungen aus dem Stand der Technik.

2. Die erfindungsgemäßen Verbindungen weisen eine relativ niedrige Sublimationstemperatur, eine hohe Temperaturstabilität und lassen sich daher unzersetzt und rückstandsfrei sublimieren. Weiterhin weisen sie eine hohe Oxidationsstabilität und eine hohe Glasübergangstemperatur auf, was sowohl für die Prozessierbarkeit, beispielweise aus Lösung oder aus der Gasphase, als auch für die Verbindung in elektronischen Vorrichtungen vorteilhaft ist.

3. Die Verwendung der erfindungsgemäßen Verbindungen in elektronischen Vorrichtungen, insbesondere eingesetzt als Elektronentransport- oder Elektroneninjektionsmaterial, aber auch als Matrixmaterial, führen zu hohen Effizienzen, geringen Betriebsspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

## Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

### Beispiel 1

**Synthese von 2-Dibenzofuran-4-yl-4,6-diphenyl-[1,3,5]triazin**

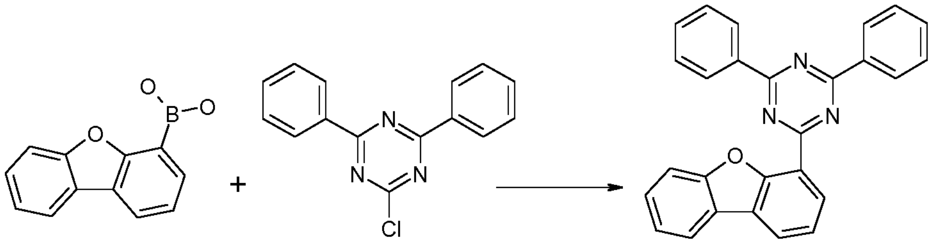

[89827-45-2] [3842-55-5]

28,9 g (136 mmol) Dibenzofuran-4-boronsäure, 33 g (124,1 mmol) 2-Chloro-4,6-diphenyl-[1,3,5]triazin, 78,9 ml (158 mmol) $Na_2CO_3$ (2 M-Lösung) werden in 120 mL Toulol, 120 mL Ethanol und 100 mL Wasser suspendiert. Zu dieser Suspension werden 2,6 g (2.2 mmol) $Pd(PPh_3)_4$ gegeben, und die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert. Die Ausbeute beträgt 45 g (112 mmol), entsprechend 91% der Theorie.

Analog können folgende Verbindungen erhalten werden:

| Edukt 1 | Edukt 2 | Produkt | Ausbeute |
|---|---|---|---|
| 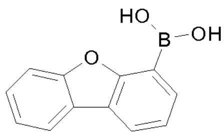 [89827-45-2] | 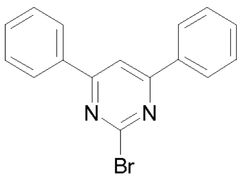 [56181-49-8] | 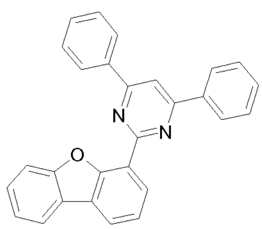 | 82% |
| 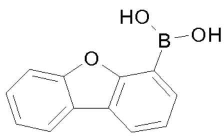 [89827-45-2] | 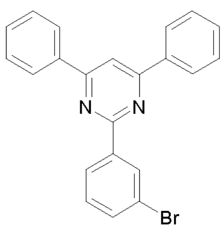 [864377-22-0] | 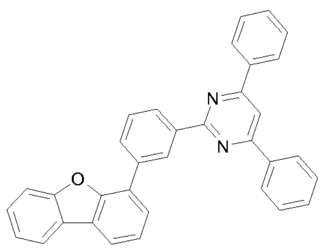 | 79% |

(fortgesetzt)

| Edukt 1 | Edukt 2 | Produkt | Ausbeute |
|---|---|---|---|
| 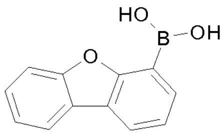<br>[89827-45-2] | 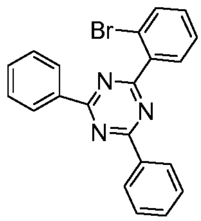<br>77989-15-2 | 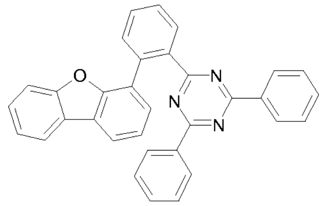 | 70% |
| 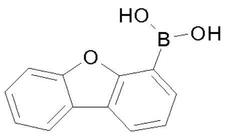<br>[89827-45-2] | 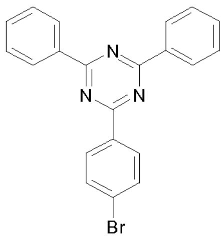<br>[23449-08-3] | 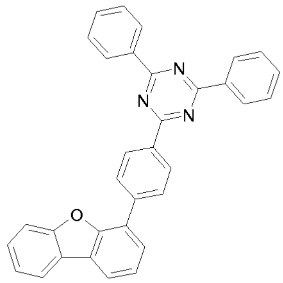 | 78% |
| 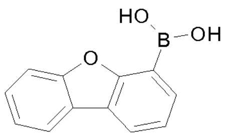<br>[89827-45-2] | 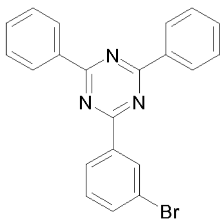<br>864377-31-1] | 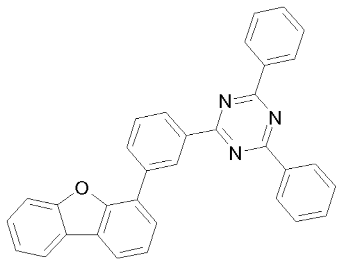 | 82% |
| 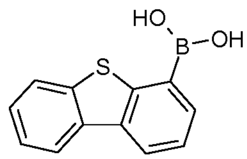<br>[108847-20-7] | 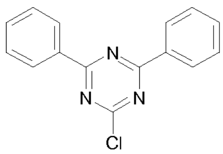<br>[3842-55-5] | 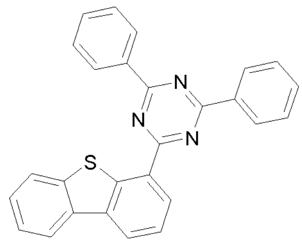 | 80% |
| 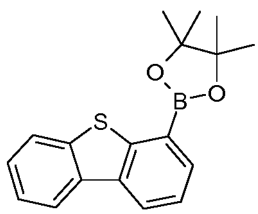<br>[912824-84-1] | 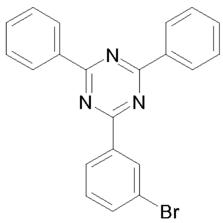<br>864377-31-1] | 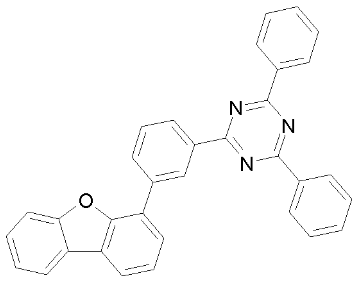 | 84% |

**Beispiel 2**

**Synthese von 2-(8-Bromo-dibenzofuran-4-yl)-4,6-diphenyl-[1,3,5]triazin**

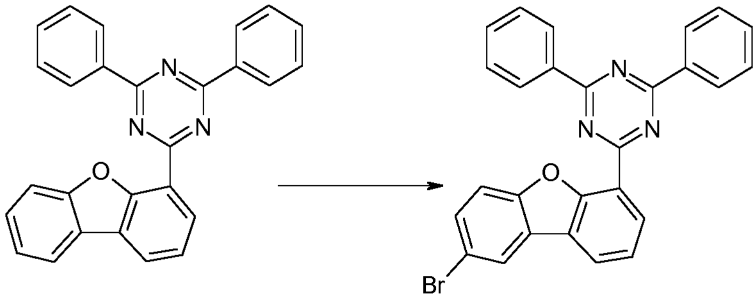

16 g (41 mmol) 2-Dibenzofuran-4-yl-4,6-diphenyl-[1,3,5]triazin werden mit 8 mg N-Bromsuccinimid (NBS) (45 mmol, 1.1 mol%) in 100 ml trockenem Dimethylformamid (DMF) vorgelegt. Die Reaktionsmischung wird 24 h auf 120°C erhitzt und danach das Lösungsmittel im Vakuum entfernt. Der Rückstand wird säulenchromatographisch an Kieselgel mit Heptan/DCM (2/1) als Eluent gereinigt. Die Ausbeute beträgt 14,6 g (30 mmol), entsprechend 75% der Theorie.

Analog können folgende Verbindungen erhalten werden:

| Edukt 1 | Produkt | Ausbeute |
|---|---|---|
| 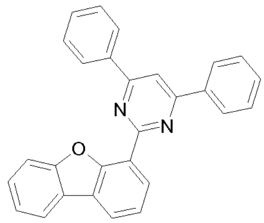 | 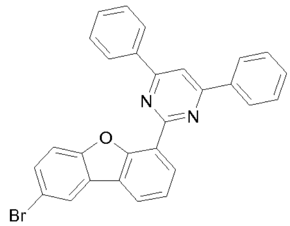 | 73% |
| 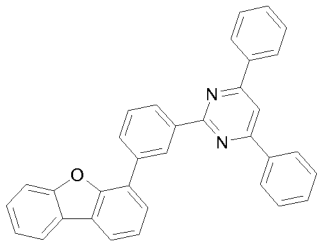 | 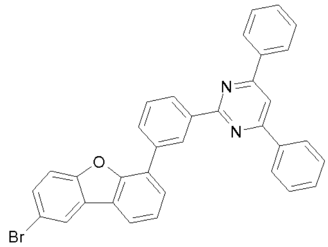 | 58% |
| 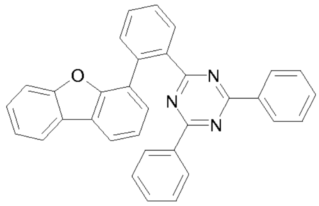 | 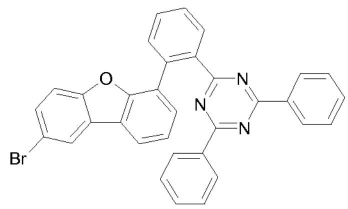 | 61% |
| 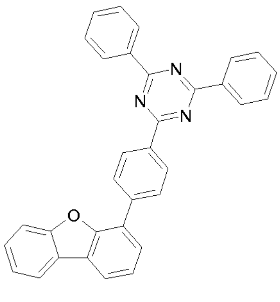 | 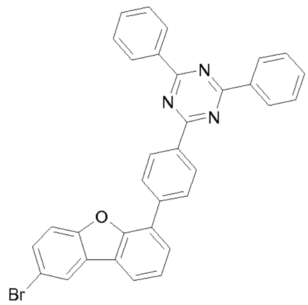 | 62% |

(fortgesetzt)

| Edukt 1 | Produkt | Ausbeute |
|---|---|---|
| 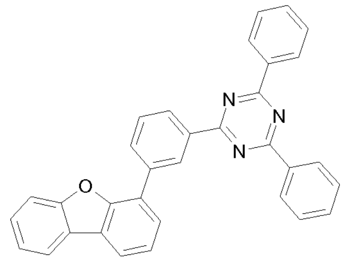 | 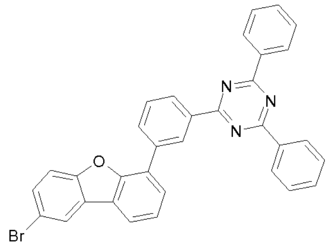 | 63% |
| 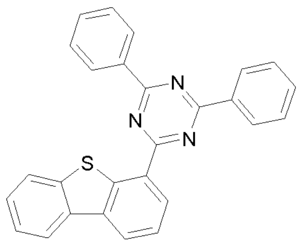 | 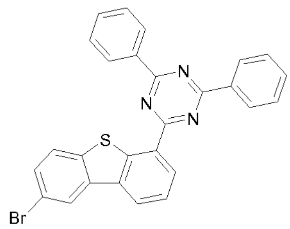 | 74% |
| 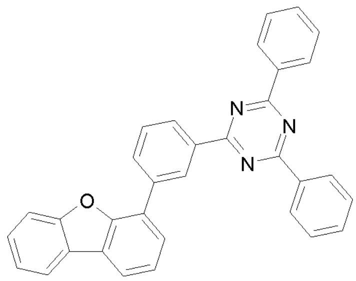 | 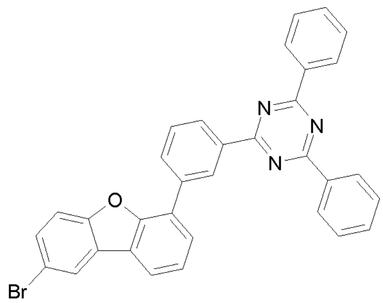 | 59% |

**Beispiel 3**

**Synthese von 9-[6-(4,6-Diphenyl-[1,3,5]triazin-2-yl)-dibenzofuran-2-yl]-3-phenyl-9H-carbazol**

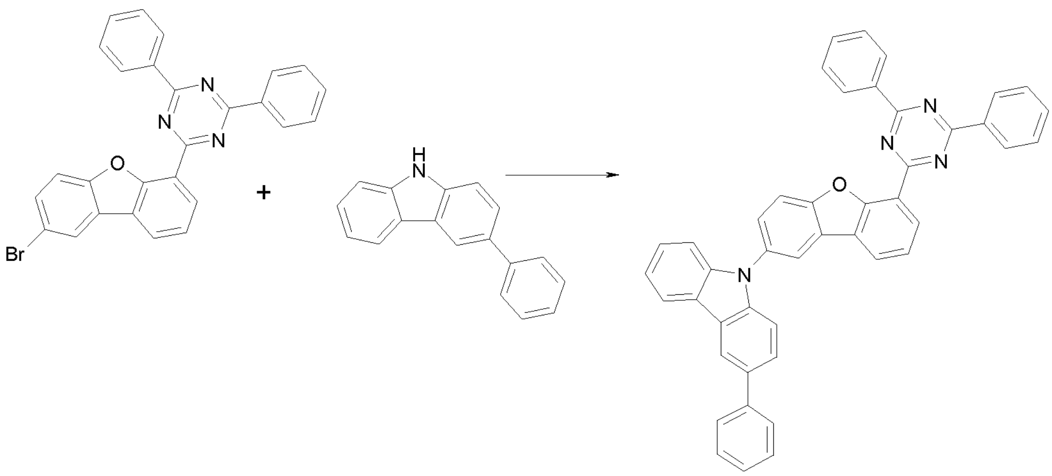

Eine entgaste Lösung von 70 g (147 mmol) 2-(8-Bromo-dibenzofuran-4-yl)-4,6-diphenyl-[1,3,5]triazin und 35,7 g (147 mmol) 3-Phenyl-9H-carbazol in 600 mL Toluol wird 1 h mit $N_2$ gesättigt. Danach wird die Lösung zuerst mit 2.09 mL (8.6 mmol) P(*t*Bu)$_3$, dann mit 1.38 g (6.1 mmol) Palladium(II)acetat versetzt und anschließend werden 17.7 g (185

mmol) NaO*t*Bu im festen Zustand zugegeben. Die Reaktionsmischung wird 1 h unter Rückfluss erhitzt. Nach dem Abkühlen auf Raumtemperatur werden vorsichtig 500 mL Wasser zugesetzt. Die wässrige Phase wird mit 3 x 50 mL Toluol gewaschen, über $MgSO_4$ getrocknet und das Lösungsmittel im Vakuum entfernt. Danach wird das Rohprodukt über Kieselgel mit Heptan/Essigsäureester (20/1) chromatographisch gereinigt. Der Rückstand wird aus Toluol umkristallisiert und abschließend im Hochvakuum (p = 5 x $10^{-6}$ mbar) sublimiert.

Die Ausbeute beträgt 77,7 g (121 mmol), entsprechend 83% der Theorie.

Analog können folgende Verbindungen erhalten werden:

| Edukt 1 | Edukt 2 | Produkt | Ausbeute |
|---|---|---|---|
| 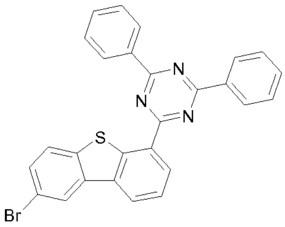 | 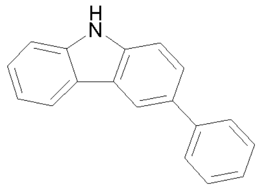 [103012-26-6] | 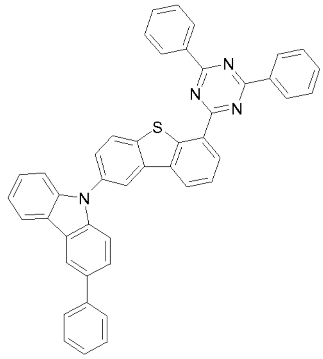 # | 92% |
| 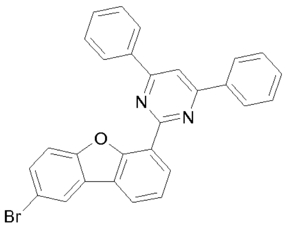 | 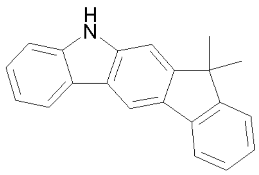 [1257220-47-5] | 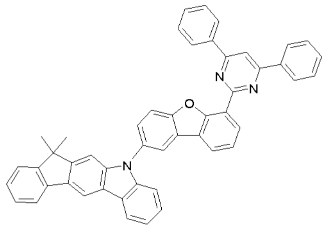 | 87% |
| 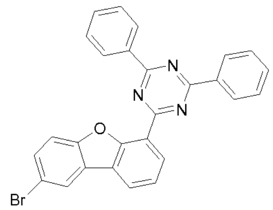 | 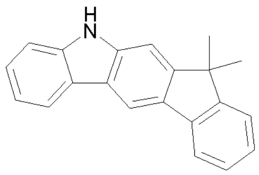 [1257220-47-5] | 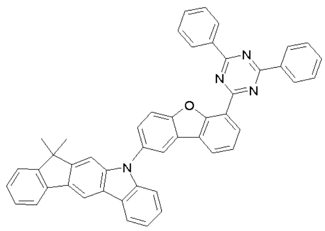 | 87% |
| 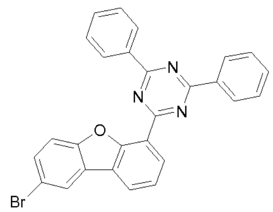 | 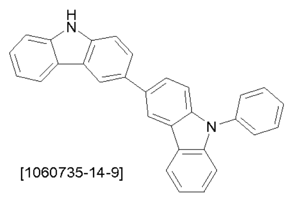 [1060735-14-9] | 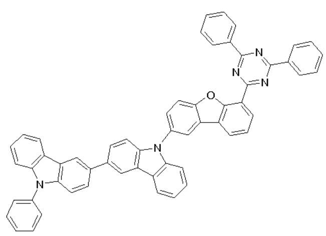 | 83% |
| 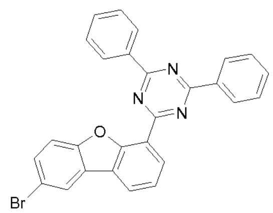 | 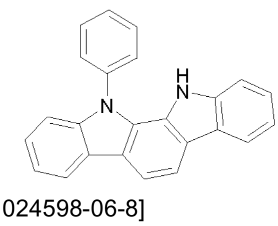 [1024598-06-8] | 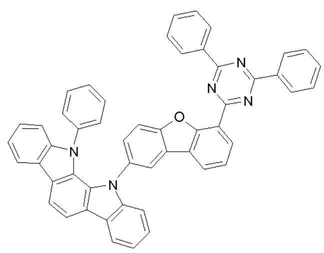 | 57% |

(fortgesetzt)

| Edukt 1 | Edukt 2 | Produkt | Ausbeute |
|---|---|---|---|
| 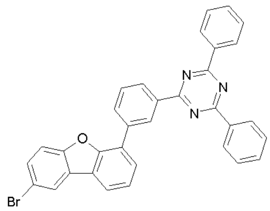 | 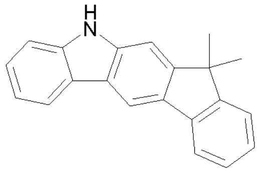 [1257220-47-5] | 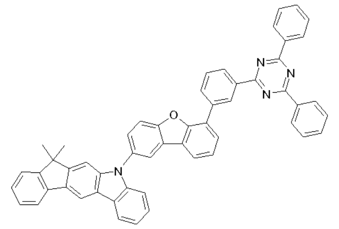 | 62% |
| 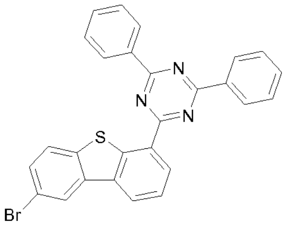 | 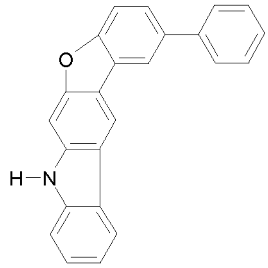 [1439927-96-4] | 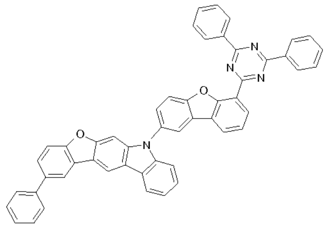 | 72% |
| 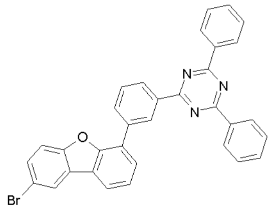 | 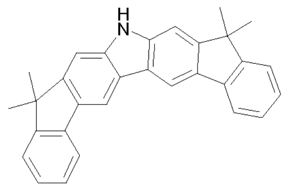 [1373281-72-1] | 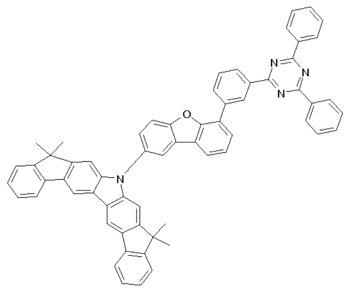 | 70% |
| 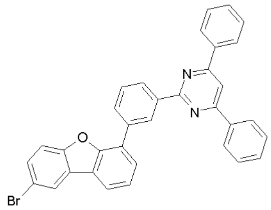 | 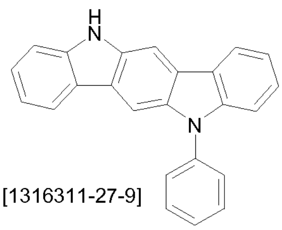 [1316311-27-9] | 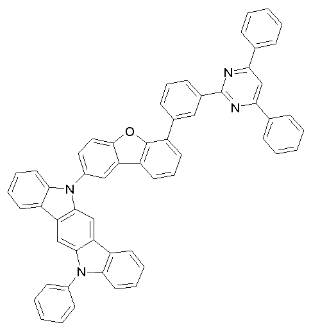 | 72% |
| 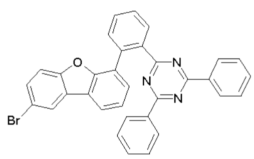 | 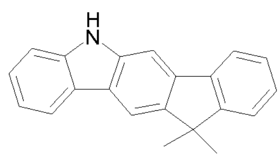 [1260228-95-2] | 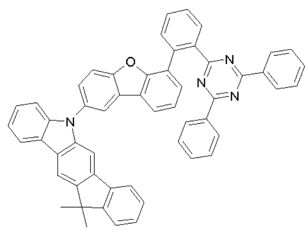 | 65% |

(fortgesetzt)

| Edukt 1 | Edukt 2 | Produkt | Ausbeute |
|---|---|---|---|
| 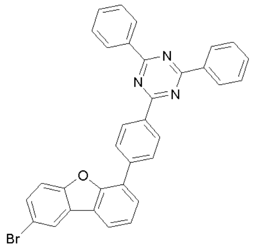 | 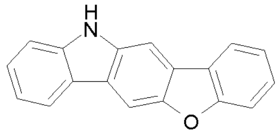 [1199350-22-5] | 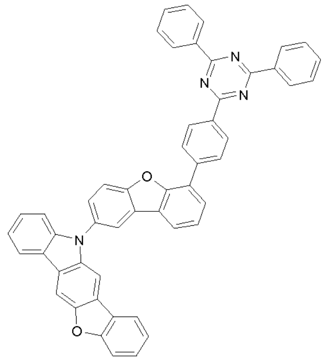 | 68% |
| 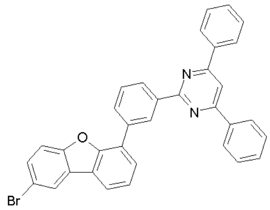 | 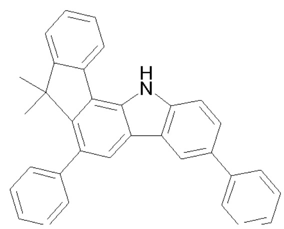 [1447708-58-8] | 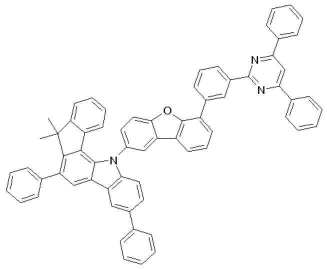 | 61% |
| 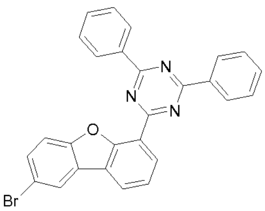 | 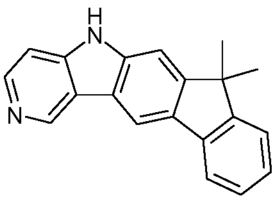 [1257248-14-8] | 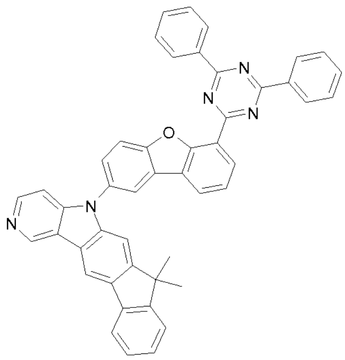 | 72% |
| 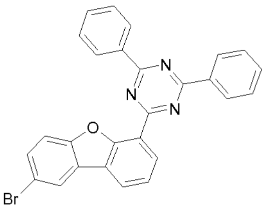 | 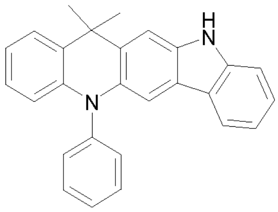 [1361126-04-6] | 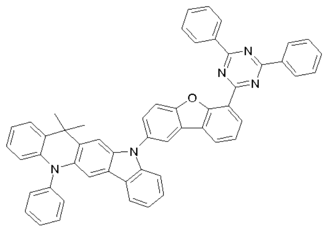 | 80% |
| 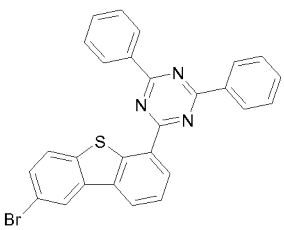 | 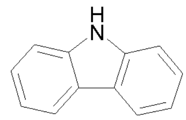 [86-74-8] | 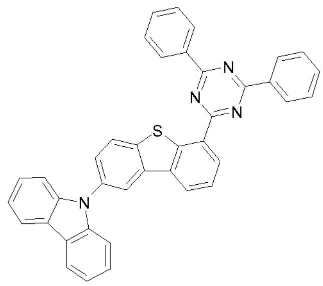 # | 95% |

(fortgesetzt)

| Edukt 1 | Edukt 2 | Produkt | Ausbeute |
|---|---|---|---|
| 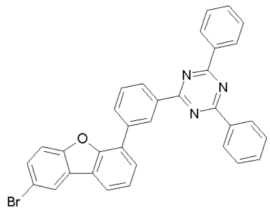 | 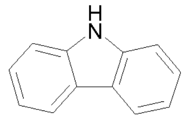 [86-74-8] | 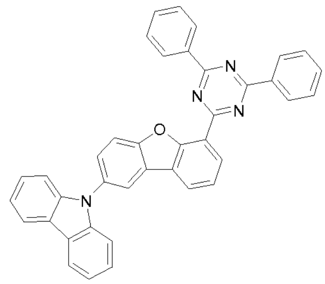 | 90% |

# nicht erfindungsgemäß

**Beispiel 4**

**Synthese von 2-Dibenzofuran-4-yl-4-phenyl-chinazolin**

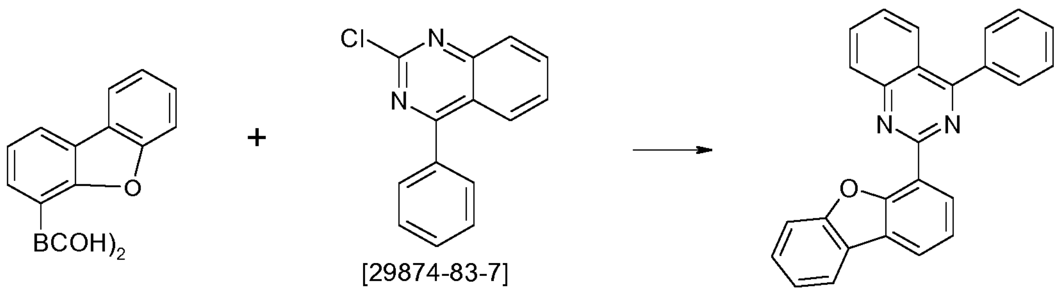
[29874-83-7]

23 g (110.0 mmol) Dibenzofuran-4-boronsäure, 29.5 g (110.0 mmol) 2-Chlor-4-phenyl-chinazolin und 26 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben. Die Reaktionsmischung wird 16 h unter Rückfluss erhitzt. Nach dem Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan /Heptan umkristallisiert. Die Ausbeute beträgt 31 g (85 mmol), entsprechend 79% der Theorie.

Analog können folgende Verbindungen erhalten werden:

| Edukt 1 | Edukt 2 | Produkt | Ausbeute |
|---|---|---|---|
| 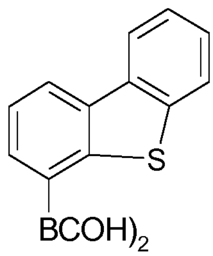 | 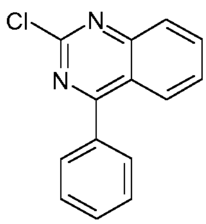 [29874-83-7] | 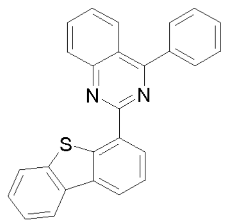 | **73%** |

(fortgesetzt)

| Edukt 1 | Edukt 2 | Produkt | Ausbeute |
|---|---|---|---|
| 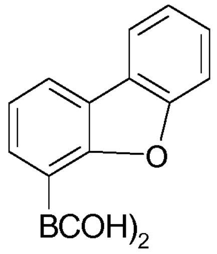 | 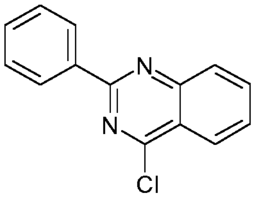 [6484-25-9] | 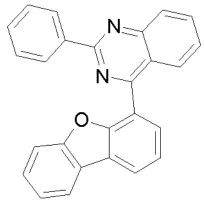 | **69%** |
| 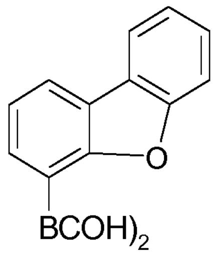 | 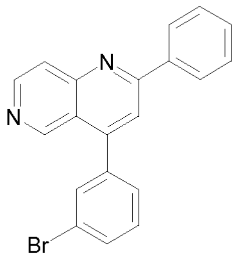 [1632307-99-3] | 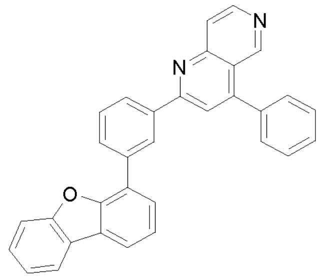 | **67%** |
| 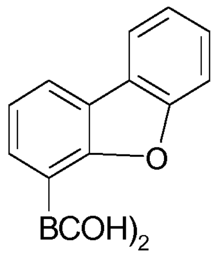 | 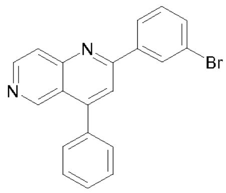 [1632307-97-1] | 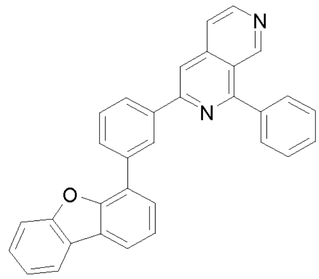 | **63%** |
| 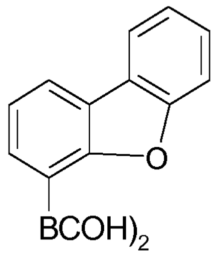 | 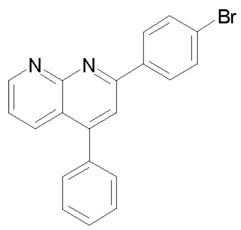 [1632294-77-9] | 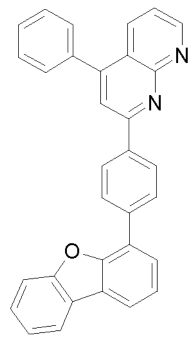 | **65%** |
| 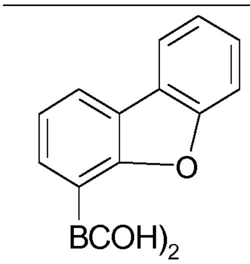 | 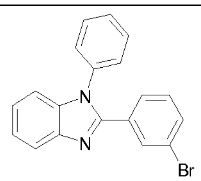 [760212-40-6] | 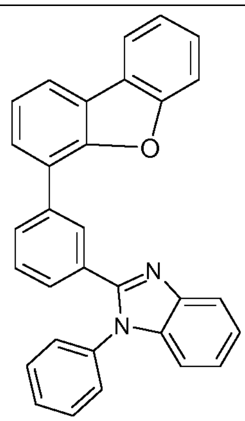 | **66%** |

(fortgesetzt)

| Edukt 1 | Edukt 2 | Produkt | Ausbeute |
|---|---|---|---|
| 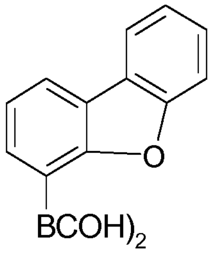 | 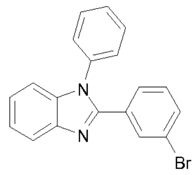 [760212-40-6] | 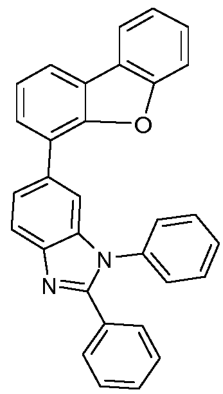 | 60% |

**Beispiel 5**

**Synthese von 2-(8-Bromo-dibenzofuran-4-yl)-4-phenyl-chinazolin**

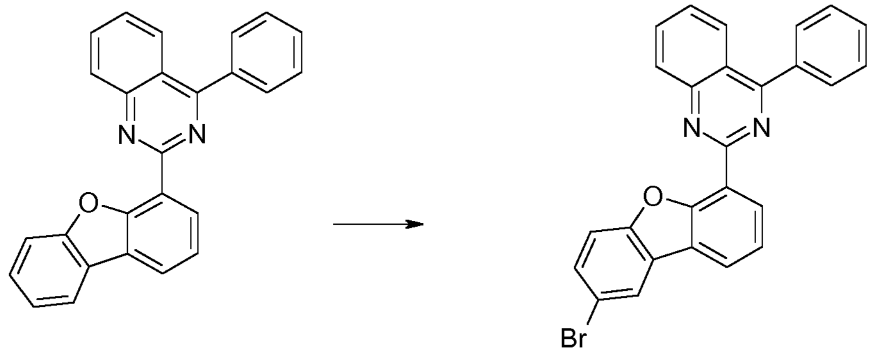

70,6 g (190.0 mmol) 2-Dibenzofuran-4-yl-4-phenyl-chinazolin werden in 2000 mL Essigsäure (100%) und 2000 mL Schwefelsäure (95-98%) suspendiert. Zu dieser Suspension werden portionsweise 34 g (190 mmol) NBS zugegeben und 2 Stunden in Dunkelheit gerührt. Danach mit Wasser/Eis versetzt und der Feststoff abgetrennt und mit Ethanol nachgewaschen. Der Rückstand wir in Toluol umkristallisiert. Die Ausbeute beträgt 59 g (130 mmol), entsprechend 69% der Theorie.

Im Falle von Thiophen-Derivate wird Nitrobenzol anstatt Schwefelsäure und elementares Brom an Stelle von NBS eingesetzt.

Analog können folgende Verbindungen erhalten werden:

| Edukt | Produkt | Ausbeute |
|---|---|---|
| 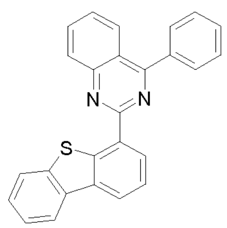 | 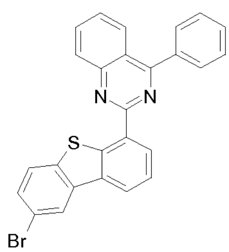 | 61% |

(fortgesetzt)

| Edukt | Produkt | Ausbeute |
|---|---|---|
| 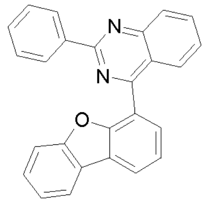 | 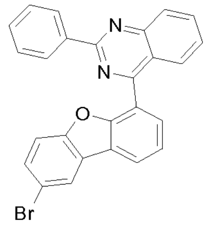 | **55%** |
| 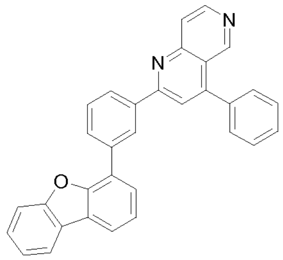 | 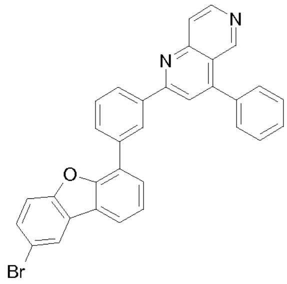 | **31%** |
| 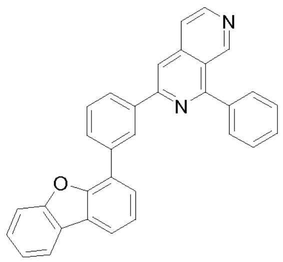 | 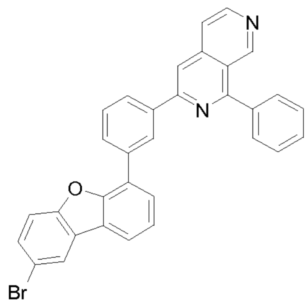 | **33%** |
| 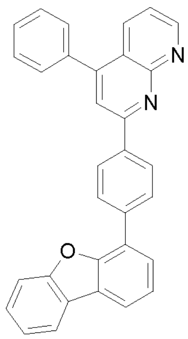 | 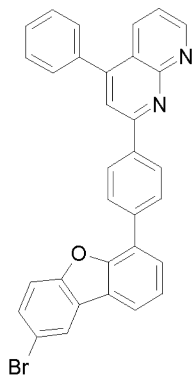 | **30%** |

(fortgesetzt)

| Edukt | Produkt | Ausbeute |
|---|---|---|
| 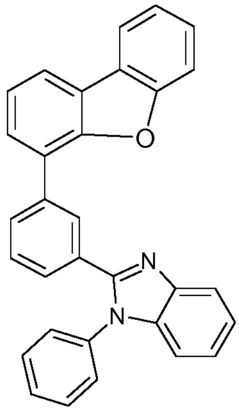 | 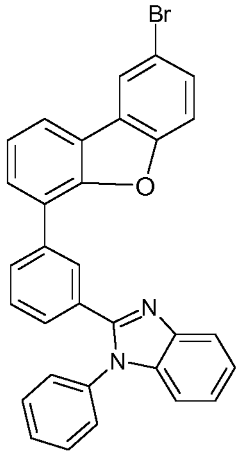 | 36% |
| 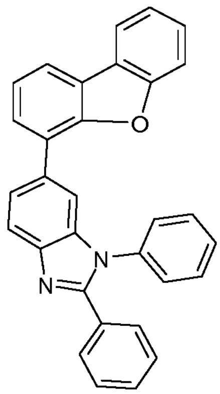 | 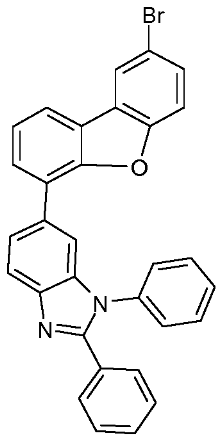 | 58% |

**Beispiel 6**

**Synthese von 3-Phenyl-9-[6-(4-phenyl-chinazolin-2-yl)-dibenzofuran-2-yl]-9H-carbazol**

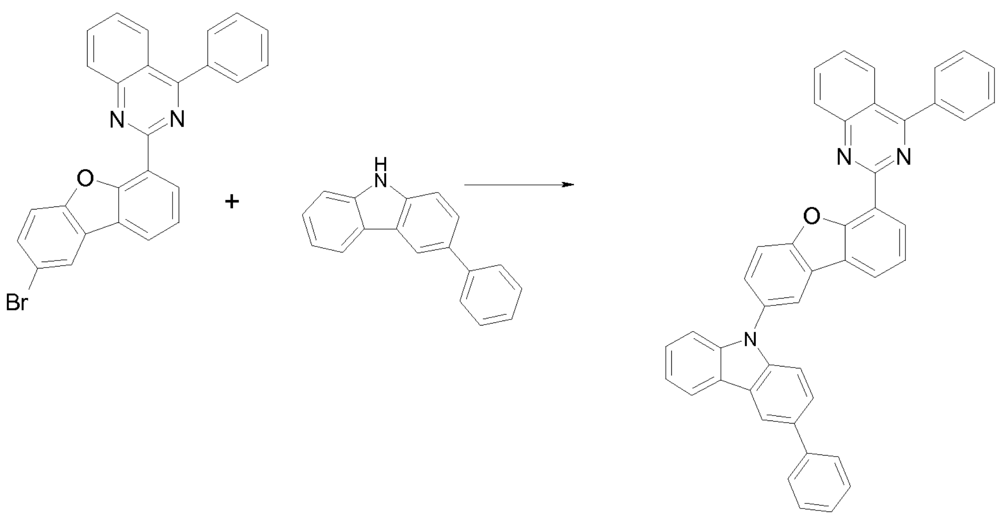

#

Eine entgaste Lösung von 70 g (147 mmol) 2-(8-Bromo-dibenzofuran-4-yl)-4-phenyl-chinazolin und 35,7 g (147 mmol) 3-Phenyl-9H-carbazol in 600 mL Toluol wird 1 h mit $N_2$ gesättigt. Danach wird die Lösung zuerst mit 2.09 mL (8.6 mmol) P(*t*Bu)$_3$, dann mit 1.38 g (6.1 mmol) Palladium(II)acetat versetzt und anschließend werden 17.7 g (185 mmol) NaO*t*Bu im festen Zustand zugegeben. Die Reaktionsmischung wird 1 h unter Rückfluss erhitzt. Nach dem Abkühlen auf Raumtemperatur werden vorsichtig 500 mL Wasser zugesetzt. Die wässrige Phase wird 3 mal mit 50 mL Toluol gewaschen, über $MgSO_4$ getrocknet und das Lösungsmittel im Vakuum entfernt. Danach wird das Rohprodukt über Kieselgel mit Heptan/Essigsäureester (20/1) chromatographisch gereinigt. Der Rückstand wird aus Toluol umkristallisiert und abschließend im Hochvakuum (p = 5 x $10^{-6}$ mbar) sublimiert.

Die Ausbeute beträgt 76 g (119 mmol), entsprechend 81% der Theorie.

Analog können folgende Verbindungen erhalten werden:

| Edukt 1 | Edukt 2 | Produkt# | Ausbeute |
|---|---|---|---|
| 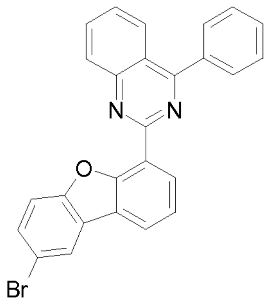 | 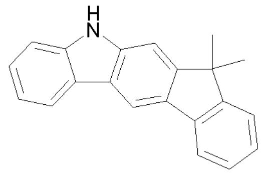 [1257200-47-5] | 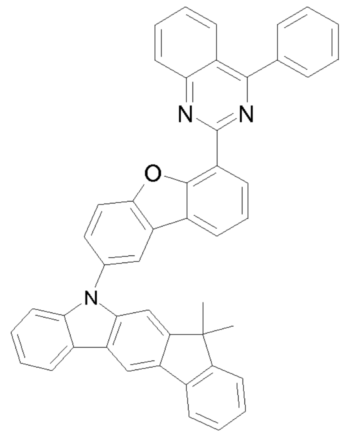 | 73% |
| 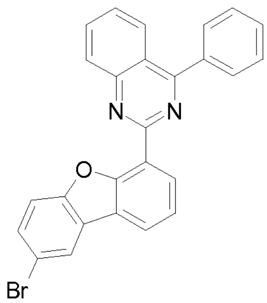 | 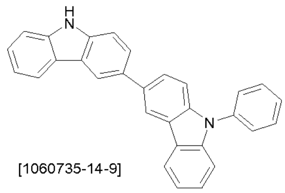 [1060735-14-9] | 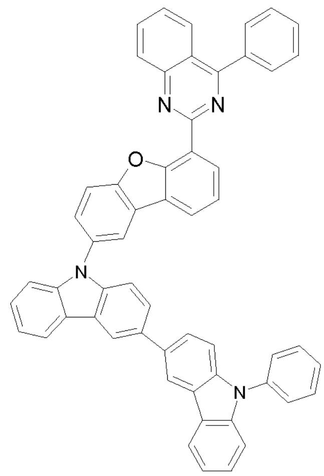 | 71% |
| 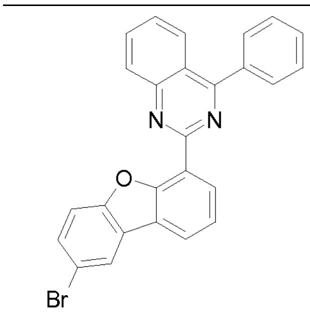 | 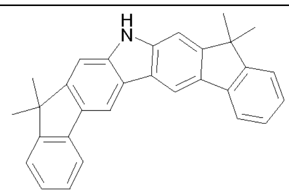 [1373281-72-1] | 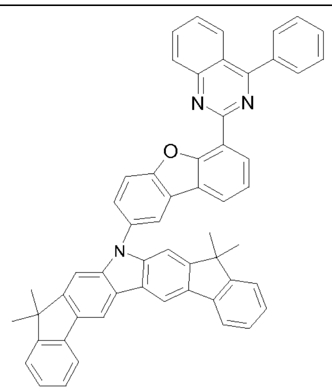 | 69% |

(fortgesetzt)

| Edukt 1 | Edukt 2 | Produkt# | Ausbeute |
|---|---|---|---|
| 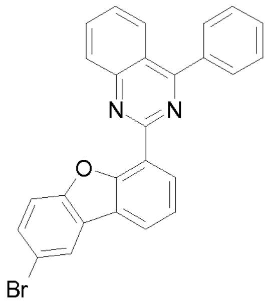 | 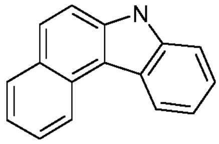 [250-25-4] | 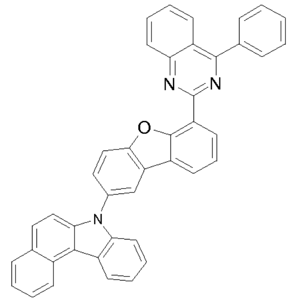 | 71% |
| 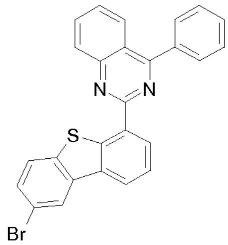 | 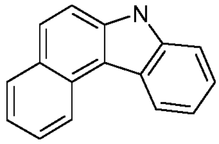 [250-25-4] | 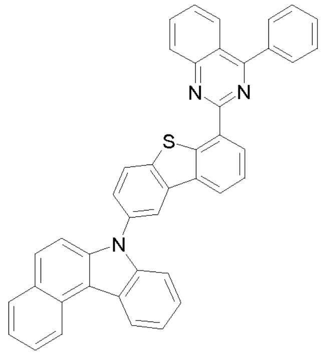 | 70% |
| 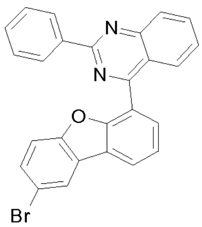 | 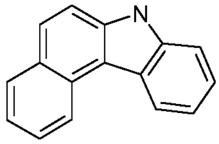 [250-25-4] | 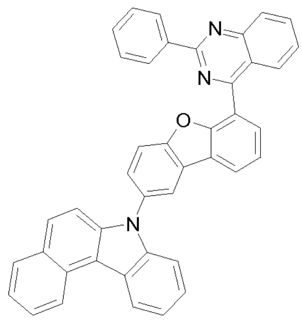 | 66% |
| 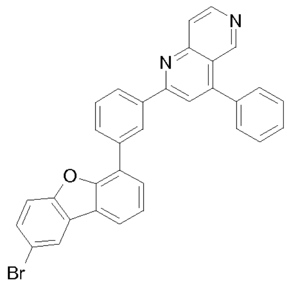 | 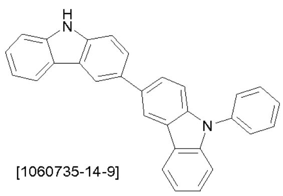 [1060735-14-9] | 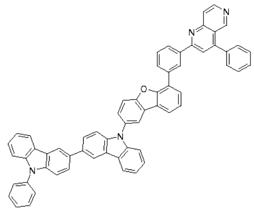 | 69% |
| 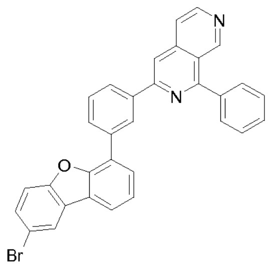 | 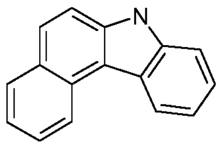 [250-25-4] | 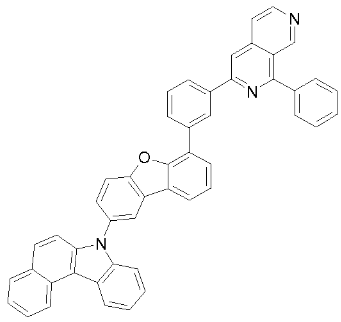 | 78% |

(fortgesetzt)

| Edukt 1 | Edukt 2 | Produkt# | Ausbeute |
|---|---|---|---|
| 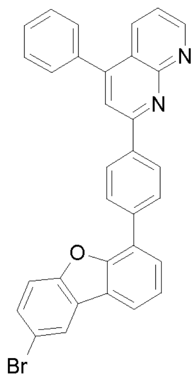 | 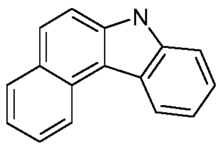 [250-25-4] | 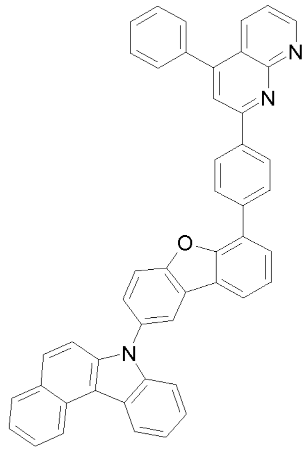 | **74%** |
| 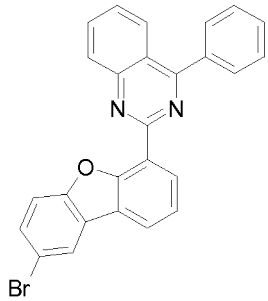 | 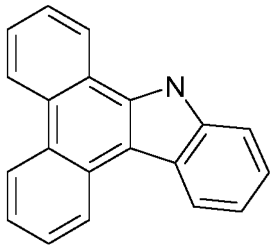 [201-67-2] | 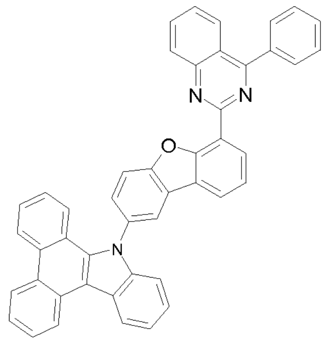 | **75%** |
| 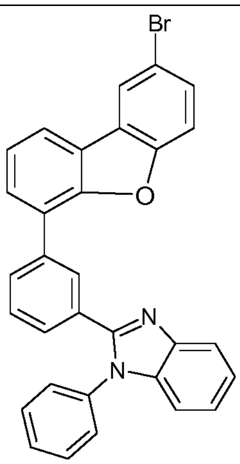 | 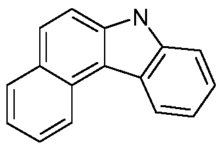 [250-25-4] | 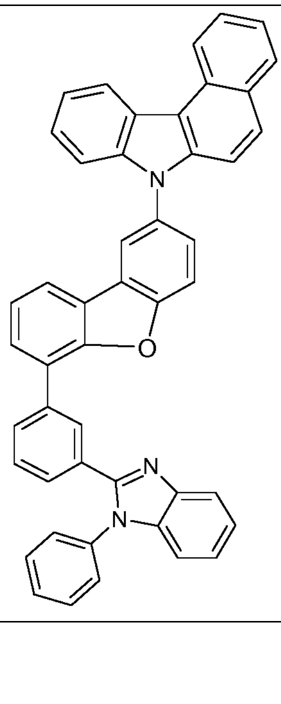 | **80%** |

(fortgesetzt)

| Edukt 1 | Edukt 2 | Produkt# | Ausbeute |
|---|---|---|---|
| 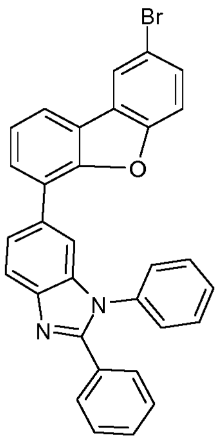 | 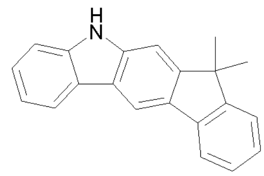 [1257220-47-5] | 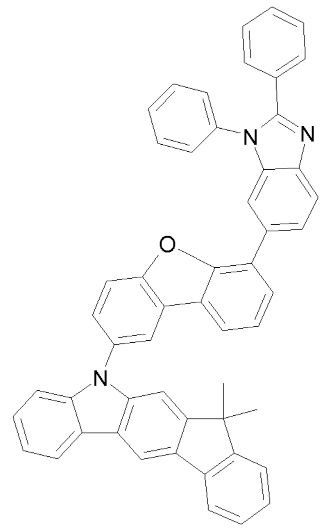 | 72% |

# nicht erfindungsgemäß

**Beispiel 7**

**Herstellung und Charakterisierung der OLED**

In den folgenden Beispielen V1 bis E12 (siehe Tabellen 1 und 2) werden die Daten verschiedener OLEDs dargestellt.

**Vorbehandlung für die Beispiele V1 bis E12:** Glasplättchen, die mit strukturiertem ITO (Indium Zinn Oxid) der Dicke 50 nm beschichtet sind, werden zur verbesserten Prozessierung mit 20 nm PEDOT:PSS beschichtet (Poly(3,4-ethylendioxythiophen) poly(styrolsulfonat), bezogen als CLEVIOS™ P VP AI 4083 von Heraeus Precious Metals GmbH Deutschland, aus wässriger Lösung aufgeschleudert). Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / optionale Zwischenschicht (IL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC3:TEG1 (55%:35%:10%) bedeutet hierbei, dass das Material IC1 in einem Volumenanteil von 55%, IC3 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$. Als Lebensdauer LD wird die Zeit definiert, nach der die Leuchtdichte bei Betrieb mit konstantem Strom von der Startleuchtdichte auf einen gewissen Anteil L1 absinkt. Eine Angabe von L0;j0 = 4000 cd/$m^2$ und L1 = 70% bedeutet, dass die angegebene Lebensdauer der Zeit entspricht, nach der die Anfangsleuchtdichte von 4000 cd/$m^2$ auf 2800 cd/$m^2$ absinkt. Analog bedeutet L0;j0 = 20mA/$cm^2$, L1 = 80%, dass die Leuchtdichte bei Betrieb mit 20mA/$cm^2$ nach der Zeit LD auf 80% ihres Anfangswertes absinkt.

Die Daten der verschiedenen OLEDs sind in Tabelle 2 zusammengefasst. Die Beispiele V1 bis V5 sind Vergleichsbeispiele gemäß dem Stand der Technik, die Beispiele E1 bis E12 zeigen Daten von erfindungsgemäßen OLEDs.

Im Folgenden werden einige der Beispiele näher erläutert, um die Vorteile der erfindungsgemäßen OLEDs zu verdeutlichen.

**Verwendung von erfindungsgemäßen Mischungen in der Emissionsschicht phosphoreszenter OLEDs**

Die erfindungsgemäßen Materialien ergeben bei Einsatz als Matrix-materialien in phosphoreszierenden OLEDs wesentliche Verbesserungen der Leistungseffizienz gegenüber dem Stand der Technik. Durch Einsatz der erfindungsgemäßen Verbindungen EG1 und EG2 in Kombination mit dem grün emittierenden Dotanden TEG1 lässt sich eine Steigung der der Leistungseffizienz um bis zu 20% gegenüber dem Stand der Technik beobachten (Vergleich der Beispiele E1 mit V1 sowie Vergleich von E2 mit V2, V3, V4 und V5). Desweiteren führen die erfindungsgemäßen Verbindungen zu einer signifikanten Verbesserung der Lebensdauer der Bauteile. So verbessert sich die Lebensdauer von Bauteil E2 mit der erfindungsgemäßen Matrix EG2 im Vergleich zum Stand der Technik V4 mit SdT4 von 125h auf 210h (L0;j0 = 20mA/$cm^2$, L1 = 80%).

Tabelle 1: Aufbau der OLEDs

| HTL/IL (HATCN; 5nm)/EBL/EML/HBL/ETL/EIL | | | | | | |
|---|---|---|---|---|---|---|
| Bsp | HTL Dicke | EBL Dicke | EML Dicke | HBL Dicke | ETL Dicke | EIL Dicke |
| V1 | SpA1 70nm | SpMA1 90nm | SdT1:TEG1 (90%:10%) 30nm | ST2 10nm | ST2:LiQ (50%:50%) 30nm | --- |
| V2 | SpA1 70nm | SpMA1 90nm | SdT2:TEG1 (90%:10%) 30nm | ST2 10nm | ST2:LiQ (50%:50%) 30nm | --- |
| V3 | SpA1 70nm | SpMA1 90nm | SdT3:TEG1 (90%:10%) 30nm | ST2 10nm | ST2:LiQ (50%:50%) 30nm | --- |
| V4 | SpA1 70nm | SpMA1 90nm | SdT4:TEG1 (90%:10%) 30nm | ST2 10nm | ST2:LiQ (50%:50%) 30nm | --- |
| V5 | SpA1 70nm | SpMA1 90nm | SdT5:TEG1 (90%:10%) 30nm | ST2 10nm | ST2:LiQ (50%:50%) 30nm | --- |
| E1 | SpA1 70nm | SpMA1 90nm | EG1:TEG1 (90%:10%) 30nm | ST2 10nm | ST2:LiQ (50%:50%) 30nm | --- |
| E2 | SpA1 70nm | SpMA1 90nm | EG2:TEG1 (90%:10%) 30nm | ST2 10nm | ST2:LiQ (50%:50%) 30nm | --- |
| E3 | SpA1 70nm | SpMA1 90nm | EG3:TEG1 (90%:10%) 30nm | ST2 10nm | ST2:LiQ (50%:50%) 30nm | --- |
| E4 | SpA1 70nm | SpMA1 90nm | EG4:TEG1 (90%:10%) 30nm | ST2 10nm | ST2:LiQ (50%:50%) 30nm | --- |
| E5 | SpA1 70nm | SpMA1 90nm | EG5:TEG1 (90%:10%) 30nm | ST2 10nm | ST2:LiQ (50%:50%) 30nm | --- |
| E6 | SpA1 70nm | SpMA1 90nm | EG6:TEG1 (95%:5%) 30nm | ST2 10nm | ST2:LiQ (50%:50%) 30nm | --- |
| E7 | SpA1 70nm | SpMA1 90nm | IC1:TEG1 (90%:10%) 30nm | EG7 10nm | ST2:LiQ (50%:50%) 30nm | --- |
| E8 | SpA1 70nm | SpMA1 90nm | EG8:TEG1 (90%:10%) 30nm | ST2 10nm | ST2:LiQ (50%:50%) 30nm | --- |
| E9 | SpA1 70nm | SpMA1 90nm | EG9:IC3:TE G1 (60%:35%:5 %) 30nm | IC1 10nm | ST2:LiQ (50%:50%) 30nm | --- |
| E10 | SpA1 90nm | SpMA1 130nm | E10:TER1 (92%:8%) 40nm | --- | ST2:LiQ (50%:50%) 40nm | --- |

(fortgesetzt)

| HTL/IL (HATCN; 5nm)/EBL/EML/HBL/ETL/EIL | | | | | | |
|---|---|---|---|---|---|---|
| Bsp | HTL Dicke | EBL Dicke | EML Dicke | HBL Dicke | ETL Dicke | EIL Dicke |
| E11 | SpA1 90nm | SpMA1 130nm | E11:TER1 (92%:8%) 40nm | --- | ST2:LiQ (50%:50%) 40nm | --- |
| E12 | SpA1 70nm | SpMA1 90nm | IC1:TEG1 (90%:10%) 30nm | --- | EG12:ST2 (50%:50%) 40nm | LiQ 3nm |

Tabelle 2: Daten der OLEDs

| Bsp. | U1000 (V) | SE1000 (cd/A) | LE1000 (lm/W) | EQE 1000 | CIE x/y bei 1000 cd/m$^2$ |
|---|---|---|---|---|---|
| V1 | 3.5 | 48 | 43 | 12.8% | 0.32/0.64 |
| V2 | 3.6 | 51 | 44 | 13.7% | 0.33/0.63 |
| V3 | 4.1 | 50 | 38 | 13.3% | 0.33/0.63 |
| V4 | 3.4 | 52 | 49 | 14.1% | 0.33/0.62 |
| V5 | 4.4 | 48 | 34 | 12.9% | 0.33/0.62 |
| E1 | 3.3 | 53 | 51 | 14.2% | 0.33/0.63 |
| E2 | 3.2 | 54 | 53 | 13.9% | 0.32/0.65 |
| E3 | 3.4 | 53 | 49 | 14.5% | 0.32/0.63 |
| E4 | 3.6 | 58 | 51 | 15.4% | 0.32/0.64 |
| E5 | 3.4 | 46 | 43 | 13.1% | 0.33/0.62 |
| E6 | 3.5 | 51 | 46 | 13.8% | 0.32/0.63 |
| E7 | 3.3 | 61 | 58 | 16.7% | 0.33/0.63 |
| E8 | 3.6 | 51 | 45 | 14.0% | 0.33/0.63 |
| E9 | 3.4 | 56 | 49 | 15.7% | 0.33/0.62 |
| E10 | 4.1 | 13 | 10 | 12.3% | 0.67/0.33 |
| E11 | 4.3 | 12 | 9 | 12.4% | 0.66/0.34 |
| E12 | 3.4 | 62 | 57 | 16.9% | 0.33/0.63 |

Tabelle 3: Strukturformeln der Materialien für die OLEDs

| | |
|---|---|
| 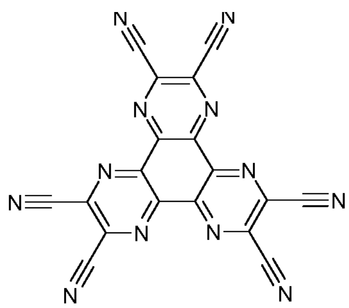 | 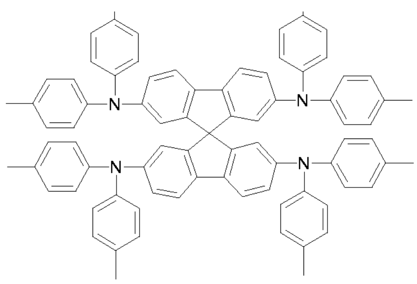 |
| HATCN | SpA1 |

(fortgesetzt)

| | |
|---|---|
| 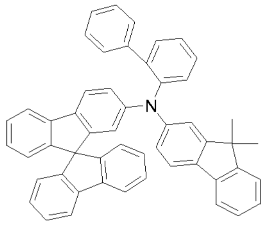 | 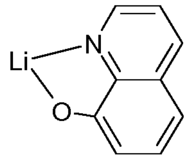 |
| SpMA1 | LiQ |
| 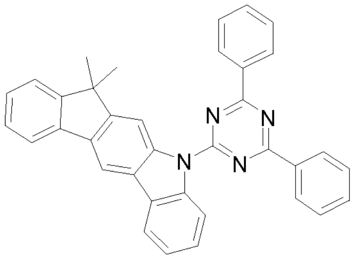 | 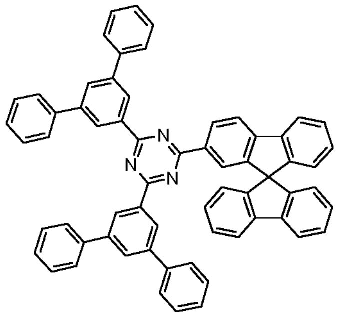 |
| IC1 | ST2 |
| | 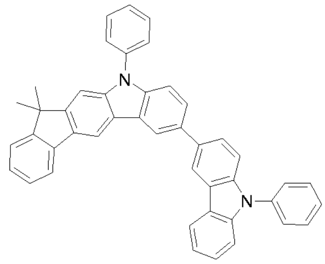 |
| | IC3 |
| 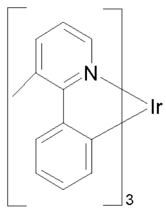 | 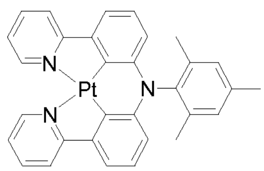 |
| TEG1 | TER1 |
| 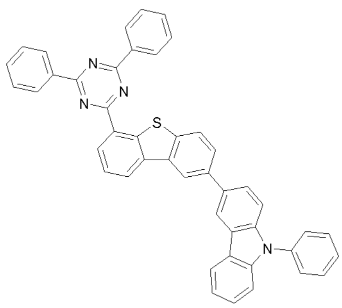 | 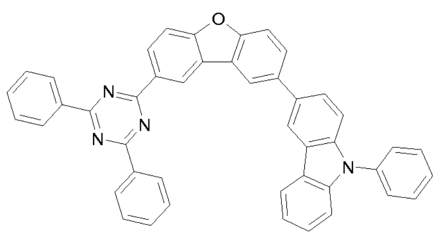 |
| SdT1 | SdT2 |

(fortgesetzt)

| | |
|---|---|
| 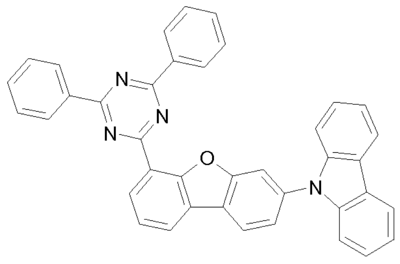 | 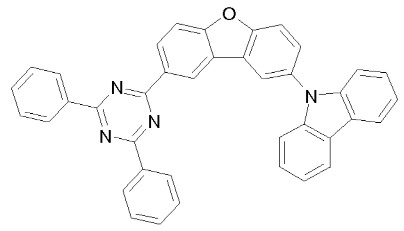 |
| SdT3 | 4SdT |
| 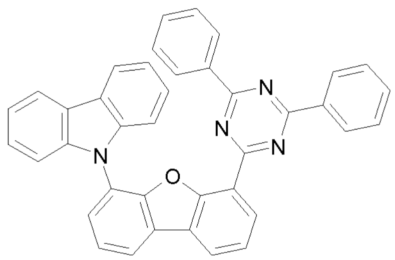 | 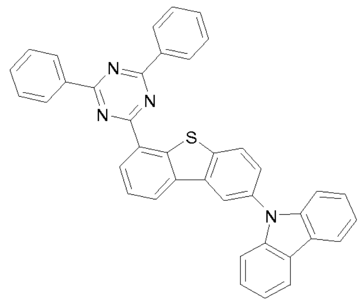 |
| SdT5 | EG1 # |
| 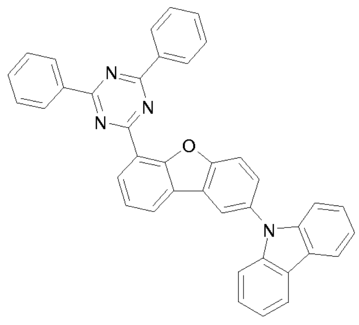 | 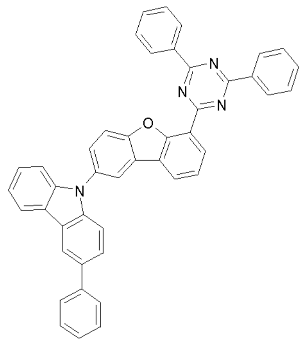 |
| EG2 | EG3 |
| 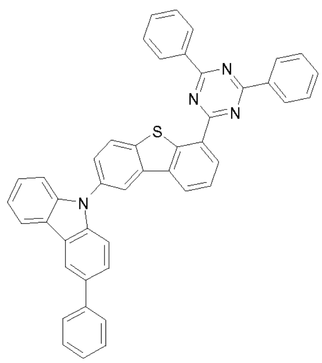 | 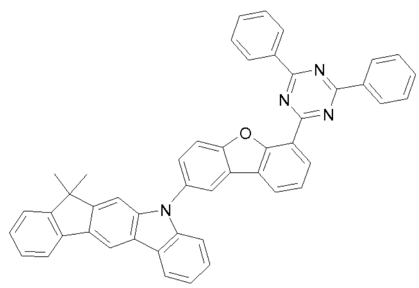 |
| EG4# | EG5 |

(fortgesetzt)

| | |
|---|---|
| 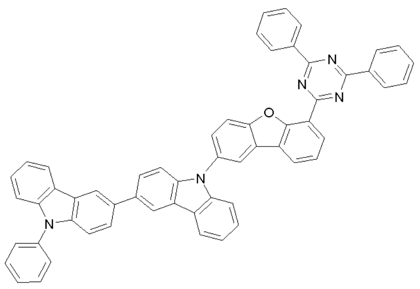 | 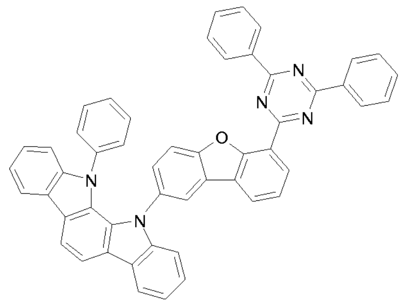 |
| EG6 | EG7 |
| 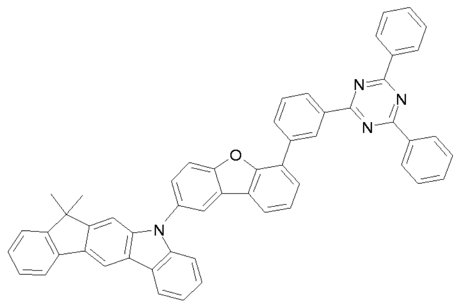 | 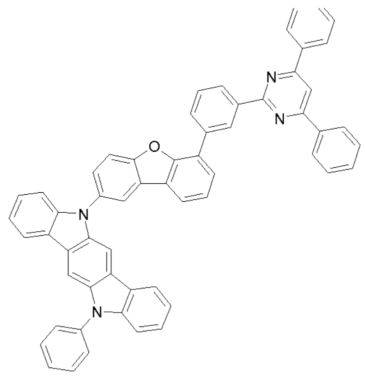 |
| EG8 | EG9 |
| 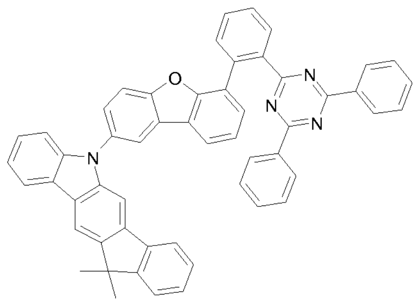 | 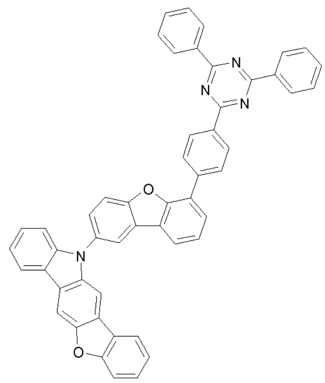 |
| EG10 | EG11 |
| 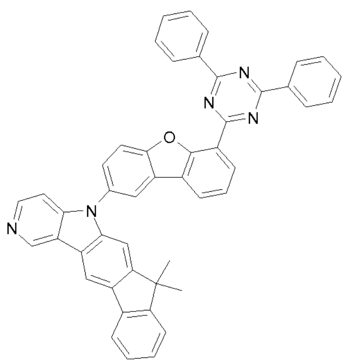 | |
| EG12 | |
| # nicht erfindungsgemäß | |

## Patentansprüche

**1.** Verbindung der allgemeinen Formel (1)

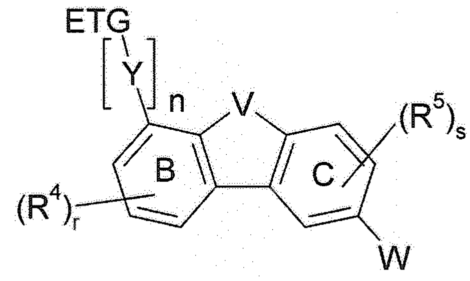

Formel (1)

wobei für die verwendeten Symbole und Indizes gilt:

ETG ist eine organische elektronentransportierende Gruppe aus der Gruppe der Triazine, Pyrimidine und Pyrazine ausgewählt sind; die ETG kann mit einem oder mehreren Resten $R^1$, die bei jedem Auftreten gleich oder verschieden sein können, substituiert sein;

W ist eine elektronenreiche organische Gruppe, die Löcher leitet, wobei W die Struktur der Formel (W-1) aufweist

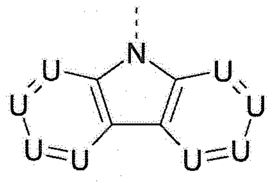

Formel (W-1)

U ist N oder $CR^1$, bevorzugt $CR^1$; wobei die gepunktete Linie die Bindung der Gruppe W and den Ring C kennzeichnet.

V ist O;

Y ist ein aromatisches Ringsystem mit 5 bis 60 Ringatomen;

n ist entweder 0 oder 1, bevorzugt 0, wobei n gleich 0 bedeutet, dass die ETG und der Ring B direkt durch eine Einfachbindung miteinander verknüpft sind;

r ist eine ganze Zahl aus 0, 1, 2 oder 3, bevorzugt 0 oder 1 und ganz bevorzugt 0;

s ist eine ganze Zahl aus 0, 1, 2 oder 3, bevorzugt 0 oder 1 und ganz bevorzugt 0;

$R^1$ ist gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C- Atomen, die jeweils mit einem oder mehreren Resten $R^2$ substituiert sein kann, wobei eine oder mehrere nicht benachbarte $CH_2$-Gruppen durch $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy-, Arylalkoxy- oder Heteroaryloxygruppe mit 5 bis 60 aromatischen Ringatomen, die durch einen oder mehrere Reste $R^2$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^2$ substituiert sein kann, oder eine Kombination aus zwei oder mehr dieser Gruppen oder eine vernetzbare Gruppe Q; dabei können zwei oder mehrere benachbarte Reste $R^1$ miteinander ein mono- oder polycyclisches, aliphatisches oder aromatisches Ringsystem bilden;

$R^2$ ist gleich oder verschieden bei jedem Auftreten H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, eine geradkettige Alkyl-, Alkoxy- oder Thioalkoxygruppe mit 1 bis

40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^3$ substituiert sein kann, oder eine Aryloxy, Arylalkoxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination aus zwei oder mehr dieser Gruppen; dabei können zwei oder mehrere benachbarte Reste $R^2$ miteinander ein mono- oder polycyclisches, aliphatisches oder aromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten $R^3$ auch miteinander ein mono- oder polycyclisches, aliphatisches oder aromatisches Ringsystem bilden;

$R^4$, $R^5$ sind gleich oder verschieden bei jedem Auftreten H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, eine geradkettige Alkyl-, Alkoxy-oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen.

**2.** Verbindung gemäß Anspruch 1 mit der allgemeinen Formel (2)

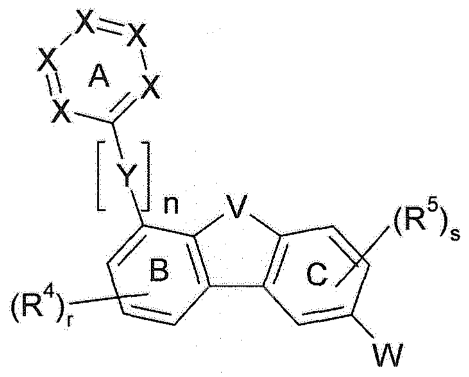

Formel (2)

wobei gilt:

X ist N oder $CR^1$, wobei drei der fünf Gruppen X in Ring A gleich N sind und es sich bei dem Ring A um ein 1,3,5-Triazin handelt.

**3.** Verbindung gemäß Anspruch 1 oder 2, **dadurch gekennzeichnet, dass** W durch die Formel (W-2) definiert ist

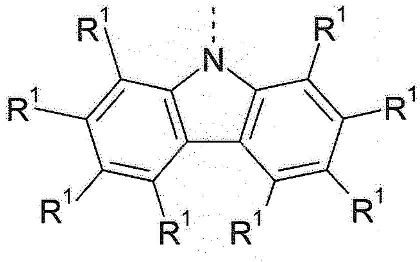

Formel (W-2)

wobei $R^1$ wie in Anspruch 1 angegeben definiert ist.

**4.** Verbindung gemäß einem oder mehreren der Ansprüche 1 bis 3, **dadurch gekennzeichnet, dass** die Verbindung die folgende Formel aufweist

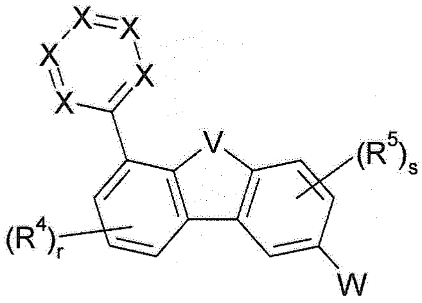

Formel (4)

wobei X wie in Anspruch 2 angegeben definiert ist.

**5.** Verbindung gemäß einem oder mehreren der Ansprüche 1 bis 4, **dadurch gekennzeichnet, dass** die Verbindung die allgemeine Formel (7) aufweist.

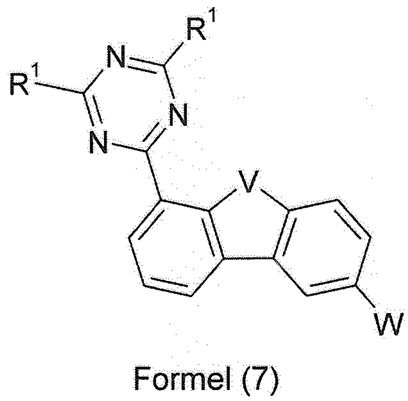

Formel (7)

**6.** Verbindung gemäß einem oder mehreren der Ansprüche 1 bis 5, **dadurch gekennzeichnet, dass** es sich bei der Gruppe W um ein Carbazol, Indenocarbazol oder Indolocarbazol handelt.

**7.** Verbindung gemäß einem oder mehreren der Ansprüche 1 bis 6, **dadurch gekennzeichnet, dass** es sich bei der Gruppe W um eine Gruppe der Formel (W-5) handelt.

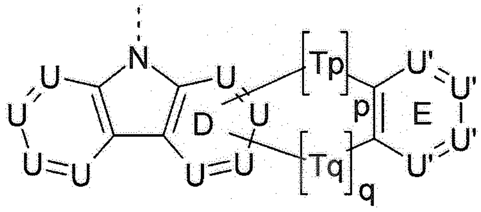

Formel (W-5)

wobei für die verwendeten Indizes und Symbole obige Definitionen gelten und wobei weiterhin gilt:

Tp, Tq sind gleich oder verschieden eine bivalente Brücke; bevorzugt sind Tp und Tq ausgewählt aus $N(R^2)$, $B(R^2)$, O, $C(R^2)_2$, $Si(R^2)_2$, C=O, $C=NR^2$, $C=C(R^2)_2$, S, S=O, $SO_2$, $P(R^2)$ und $P(=O)R^2$; ganz bevorzugt sind dabei $N(R^2)$, O, $C(R^2)_2$ und S und insbesondere bevorzugt sind $N(R^2)$ und $C(R^2)_2$;
U' ist gleich oder verschieden bei jedem Auftreten $CR^2$ oder N, bevorzugt $CR^2$;
p ist 0 oder 1; wobei p gleich 0 bedeutet, dass der Ring E und der Ring D durch eine Einfachbindung verknüpft sind;
q ist 0 oder 1; wobei q gleich 0 bedeutet, dass der Ring E und der Ring D durch eine Einfachbindung verknüpft sind;

und wobei gilt, dass p + q = 1 oder 2 und bevorzugt gleich 1 ist;
und wobei Tp und Tq jeweils an benachbarte Gruppen U des Rings D in jeder möglichen Orientierung binden; und wobei weiterhin gilt, dass jede Gruppe U, die an Tp oder Tq bindet ein Kohlenstoffatom darstellt.

**8.** Zusammensetzung enthaltend wenigstens eine Verbindung gemäß einem oder mehreren der Ansprüche 1 bis 7 sowie wenigstens eine weitere Verbindung ausgewählt aus der Gruppe bestehend aus fluoreszierenden Emittern, phosphoreszierenden Emittern, Host Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

**9.** Zusammensetzung gemäß Anspruch 8, dadurch charakterisiert, dass die zusätzliche Verbindung ein Host- oder Matrixmaterial ist.

**10.** Zusammensetzung gemäß Anspruch 8 oder 9, dadurch charakterisiert, dass die zusätzliche Verbindung ein band gap von 2.5 eV oder mehr, bevorzugt 3.0 eV oder mehr, ganz bevorzugt von 3.5 eV oder mehr aufweist.

**11.** Formulierung enthaltend wenigstens einer Verbindung gemäß einem oder mehreren der Ansprüche 1 bis 7 oder wenigstens einer Zusammensetzung gemäß einem oder mehreren der Ansprüche 8 bis 10 sowie wenigstens ein Lösungsmittel.

**12.** Verwendung wenigstens einer Verbindung gemäß einem oder mehreren der Ansprüche 1 bis 7 oder wenigstens einer Zusammensetzung gemäß einem oder mehreren der Ansprüche 8 bis 10 in einer elektronischen Vorrichtung, bevorzugt in einer organischen Elektrolumineszenzvorrichtung, ganz bevorzugt in einer organischen lichtemittierenden Diode (OLED) oder organischen lichtemittierenden elektrochemischen Zelle (OLEC, LEEC, LEC), ganz besonders bevorzugt in einer OLED, bevorzugt in einer Emissionsschicht (EML), Elektronentransportschicht (ETL) und in einer Lochblockierschicht (HBL), ganz bevorzugt in einer EML und ETL und ganz besonders bevorzugt in einer EML.

**13.** Elektronische Vorrichtung enthaltend wenigstens eine Verbindung gemäß einem oder mehreren der Ansprüche 1 bis 7 oder wenigstens eine Zusammensetzung gemäß einem oder mehreren der Ansprüche 8 bis 10, bevorzugt in einer Emissionsschicht (EML), Elektronentransportschicht (ETL) und in einer Lochblockierschicht (HBL), ganz bevorzugt in einer EML und ETL und ganz besonders bevorzugt in einer EML.

**14.** Elektronische Vorrichtung gemäß Anspruch 13, **dadurch gekennzeichnet, dass** sie gewählt ist aus organischen integrierten Schaltungen (OICs), organischen Feld-Effekt-Transistoren (OFETs), organischen Dünnfilmtransistoren (OTFTs), organischen Elektrolumineszenzvorrichtungen, organischen Solarzellen (OSCs), organischen optischen Detektoren, organischen Photorezeptoren, bevorzugt eine organische Elektroluminesszenzvorrichtung.

**15.** Elektronische Vorrichtung gemäß Anspruch 13 oder 14, **dadurch gekennzeichnet, dass** sie eine organische Elektrolumineszenzvorrichtung ist, die ausgewählt ist auch der Gruppe bestehend aus organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und organischen lichtemittierenden Dioden (OLEDs), bevorzugt OLECs und OLEDs, ganz bevorzugt OLEDs.

**16.** Verfahren zur Herstellung einer elektronischen Vorrichtung gemäß einem oder mehreren der Ansprüche 13 bis 15, **dadurch gekennzeichnet, dass** mindestens eine organische Schicht durch Gasphasenabscheidung oder aus Lösung aufgebracht wird.

**17.** Elektronische Vorrichtung gemäß Anspruch 15, zur Verwendung in der Medizin zur Phototherapie, bevorzugt zur Phototherapie der Haut.

## Claims

**1.** Compound of the general formula (1)

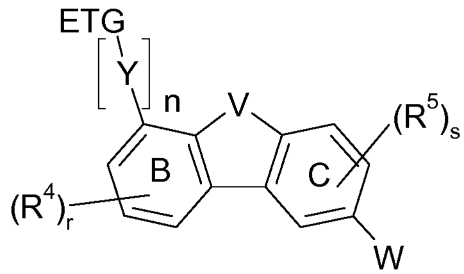

formula (1)

where the following applies to the symbols and indices used:

ETG is an organic electron-transporting group from the group of the triazines, pyrimidines and pyrazines; the ETG may be substituted by one or more radicals $R^1$, which may be identical or different on each occurrence;
W is an electron-rich organic group which conducts holes, where W has the structure of the formula (W-1)

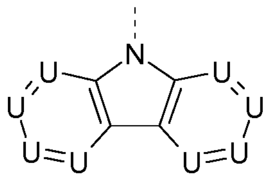

formula (W-1)

U is N or $CR^1$, preferably $CR^1$, where the dotted line denotes the bond from the group W to the ring C.
V is O;
Y is an aromatic ring system having 5 to 60 ring atoms;
n is either 0 or 1, preferably 0, where n equals 0 means that the ETG and the ring B are linked directly to one another by a single bond;
r is an integer from 0, 1, 2 or 3, preferably 0 or 1 and very preferably 0;
s is an integer from 0, 1, 2 or 3, preferably 0 or 1 and very preferably 0;
$R^1$ is, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, which may in each case be substituted by one or more radicals $R^2$, where one or more non-adjacent $CH_2$ groups may be replaced by $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where

one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy, arylalkoxy or heteroaryloxy group having 5 to 60 aromatic ring atoms, which may be substituted by one or more radicals $R^2$, or a diarylamino group, diheteroarylamino group or arylheteroarylamino group having 10 to 40 aromatic ring atoms, which may be substituted by one or more radicals $R^2$, or a combination of two or more of these groups or a cross-linkable group Q; two or more adjacent radicals $R^1$ may form a mono- or polycyclic, aliphatic or aromatic ring system with one another;

$R^2$ is, identically or differently on each occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms, each of which may be substituted by one or more radicals $R^3$, where one or more non-adjacent $CH_2$ groups may be replaced by $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S or $CONR^3$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^3$, or an aryloxy, arylalkoxy or heteroaryloxy group having 5 to 60 aromatic ring atoms, which may be substituted by one or more radicals $R^3$, or a diarylamino group, diheteroarylamino group or arylheteroarylamino group having 10 to 40 aromatic ring atoms, which may be substituted by one or more radicals $R^3$, or a combination of two or more of these groups; two or more adjacent radicals $R^2$ may form a mono- or polycyclic, aliphatic or aromatic ring system with one another;

$R^3$ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents $R^3$ may also form a mono- or polycyclic, aliphatic or aromatic ring system with one another;

$R^4$, $R^5$ are, identically or differently on each occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy, alkylalkoxy or thioalkoxy group having 3 to 40 C atoms.

**2.** Compound according to Claim 1 having the general formula (2)

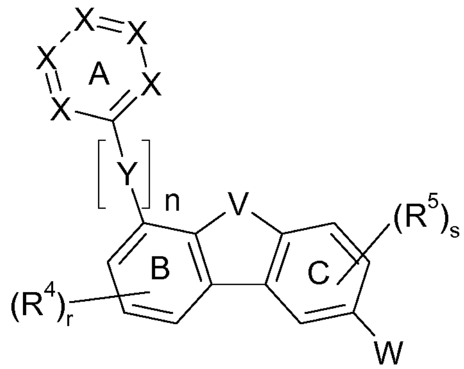

formula (2)

where:

X is N or $CR^1$, where three of the five groups X in ring A are equal to N, and the ring A is a 1,3,5-triazine.

**3.** Compound according to Claim 1 or 2, **characterised in that** W is defined by the formula (W-2)

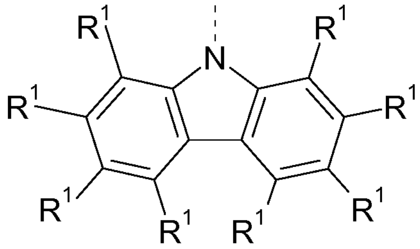

formula (W-2)

where $R^1$ is defined as indicated in Claim 1.

**4.** Compound according to one or more of Claims 1 to 3, **characterised in that** the compound has the following formula:

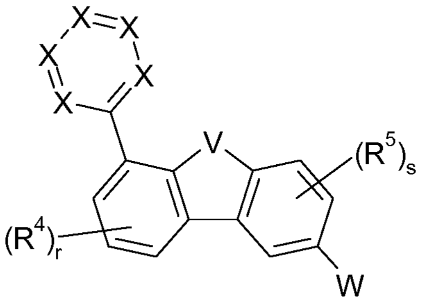

formula (4)

where X is defined as indicated in Claim 2.

**5.** Compound according to one or more of Claims 1 to 4, **characterised in that** the compound has the general formula (7):

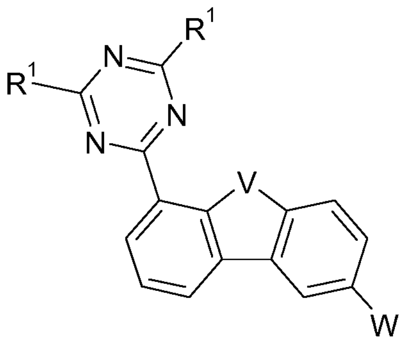

formula (7)

**6.** Compound according to one or more of Claims 1 to 5, **characterised in that** the group W is a carbazole, indenocarbazole or indolocarbazole.

**7.** Compound according to one or more of Claims 1 to 6, **characterised in that** the group W is a group of the formula (W-5):

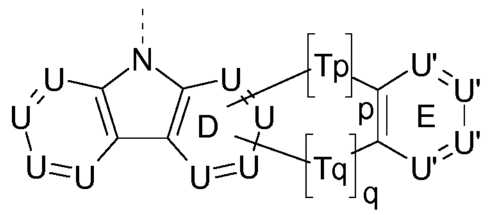

formula (W-5)

where the above definitions apply to the indices and symbols used and where furthermore:

Tp, Tq are, identically or differently, a divalent bridge; Tp and Tq are preferably selected from $N(R^2)$, $B(R^2)$, O, $C(R^2)_2$, $Si(R^2)_2$, C=O, $C=NR^2$, $C=C(R^2)_2$, S, S=O, $SO_2$, $P(R^2)$ and $P(=O)R^2$; $N(R^2)$, O, $C(R^2)_2$ and S are very preferred here and $N(R^2)$ and $C(R^2)_2$ are especially preferred;
U' is, identically or differently on each occurrence, $CR^2$ or N, preferably $CR^2$;
p is 0 or 1; where p equals 0 means that the ring E and the ring D are linked by a single bond;
q is 0 or 1; where q equals 0 means that the ring E and the ring D are linked by a single bond;

and where p + q = 1 or 2 and is preferably equal to 1;
and where Tp and Tq are each bonded to adjacent groups U of the ring D in each possible orientation; and where furthermore each group U which is bonded to Tp or Tq represents a carbon atom.

**8.** Composition comprising at least one compound according to one or more of Claims 1 to 7 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**9.** Composition according to Claim 8, **characterised in that** the additional compound is a host material or matrix material.

**10.** Composition according to Claim 8 to 9, **characterised in that** the additional compound has a band gap of 2.5 eV or more, preferably 3.0 eV or more, very preferably 3.5 eV or more.

**11.** Formulation comprising at least one compound according to one or more of Claims 1 to 7 or at least one composition according to one or more of Claims 8 to 10 and at least one solvent.

**12.** Use of at least one compound according to one or more of Claims 1 to 7 or at least one composition according to one or more of Claims 8 to 10 in an electronic device, preferably in an organic electroluminescent device, very preferably in an organic light-emitting diode (OLED) or organic light-emitting electrochemical cell (OLEC, LEEC, LEC), very particularly preferably in an OLED, preferably in an emission layer (EML), electron-transport layer (ETL) and in a hole-blocking layer (HBL), very preferably in an EML and ETL and very particularly preferably in an EML.

**13.** Electronic device comprising at least one compound according to one or more of Claims 1 to 7 or at least one composition according to one or more of Claims 8 to 10, preferably in an emission layer (EML), electron-transport layer (ETL) and in a hole-blocking layer (HBL), very preferably in an EML and ETL and very particularly preferably in an EML.

**14.** Electronic device according to Claim 13, **characterised in that** it is selected from organic integrated circuits (OICs), organic field-effect transistors (OFETs), organic thin-film transistors (OTFTs), organic electroluminescent devices, organic solar cells (OSCs), organic optical detectors, organic photoreceptors, preferably an organic electroluminescent device.

**15.** Electronic device according to Claim 13 or 14, **characterised in that** it is an organic electroluminescent device selected from the group consisting of organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs), preferably OLECs and OLEDs, very preferably OLEDs.

**16.** Process for the production of an electronic device according to one or more of Claims 13 to 15, **characterised in that** at least one organic layer is applied by gas-phase deposition or from solution.

**17.** Electronic device according to Claim 15, for use in medicine for phototherapy, preferably for phototherapy of the skin.

**Revendications**

**1.** Composé de la formule générale (1) :

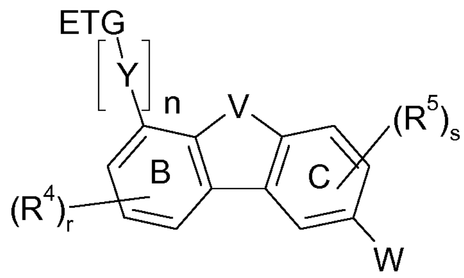

formule (1)

dans laquelle ce qui suit s'applique aux symboles et aux indices qui sont utilisés :

ETG est un groupe de transport d'électrons organique pris parmi le groupe des triazines, des pyrimidines et des pyrazines ; l'ETG peut être substitué par un radical ou par plusieurs radicaux $R^1$, lequel peut être identique ou différent pour chaque occurrence ;
W est un groupe organique riche en électrons qui assure la conduction de trous, où W présente la structure de la formule (W-1) :

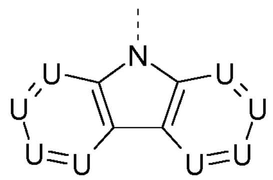

formule (W-1)

U est N ou $CR^1$, de préférence $CR^1$, où la ligne en pointillés représente la liaison depuis le groupe W jusqu'au cycle C ;
V est O ;
Y est un système de cycle aromatique qui comporte de 5 à 60 atomes de cycle ;
n est soit 0, soit 1, de préférence 0, où n égal à 0 signifie que l'ETG et le cycle B sont liés directement l'un à l'autre par une liaison simple ;
r est un entier de 0, 1, 2 ou 3, de préférence de 0 ou de 1 et de façon très préférable, de 0 ;
s est un entier de 0, 1, 2 ou 3, de préférence de 0 ou de 1 et de façon très préférable, de 0 ;
$R^1$ est, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$ $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/ peuvent être remplacé(s) par $R^2C=CR^2$, C=C, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy, arylalcoxy ou hétéroaryloxy qui comporte de 5 à 60 atomes de cycle aromatique, lequel peut être substitué par

un radical ou par plusieurs radicaux $R^2$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^2$, ou une combinaison de deux de ces groupes ou plus ou un groupe réticulable Q ; deux radicaux $R^1$ adjacents ou plus peuvent former un système de cycle aliphatique ou aromatique, mono- ou polycyclique l'un avec l'autre ou les uns avec les autres ;

$R^2$ est, de manière identique ou différente pour chaque occurrence, H, D, F, Cl, Br, I, $N(R^3)_2$, CN, $NO_2$, $Si(R^3)_3$, $B(OR^3)_2$, $C(=O)R^3$, $P(=O)(R^3)_2$, $S(=O)R^3$, $S(=O)_2R^3$, $OSO_2R^3$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C, dont chacun peut être substitué par un radical ou par plusieurs radicaux $R^3$, où un ou plusieurs groupe(s) $CH_2$ non adjacents peut/peuvent être remplacé(s) par $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S ou $CONR^3$ et où un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^3$, ou un groupe aryloxy, arylalcoxy ou hétéroaryloxy qui comporte de 5 à 60 atomes de cycle aromatique, lequel peut être substitué par un radical ou par plusieurs radicaux $R^3$, ou un groupe diarylamino, un groupe dihétéroarylamino ou un groupe arylhétéroarylamino qui comporte de 10 à 40 atomes de cycle aromatique, lequel peut être substitué par un radical ou par plusieurs radicaux $R^3$, ou une combinaison de deux de ces groupes ou plus ; deux radicaux $R^2$ adjacents ou plus peuvent former un système de cycle aliphatique ou aromatique, mono- ou polycyclique l'un avec l'autre ou les uns avec les autres ;

$R^3$ est, de manière identique ou différente pour chaque occurrence, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants $R^3$ ou plus peuvent également former un système de cycle aliphatique ou aromatique mono- ou polycyclique l'un avec l'autre ou les uns avec les autres ;

$R^4$, $R^5$ sont, de manière identique ou différente pour chaque occurrence, H, D, F, Cl, Br, I, $N(R^2)_2$, CN, $NO_2$, $Si(R^2)_3$, $B(OR^2)_2$, $C(=O)R^2$, $P(=O)(R^2)_2$, $S(=O)R^2$, $S(=O)_2R^2$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy, alkylalcoxy ou thioalcoxy ramifié ou cyclique qui comporte de 3 à 40 atomes de C.

2. Composé selon la revendication 1, présentant la formule générale (2) :

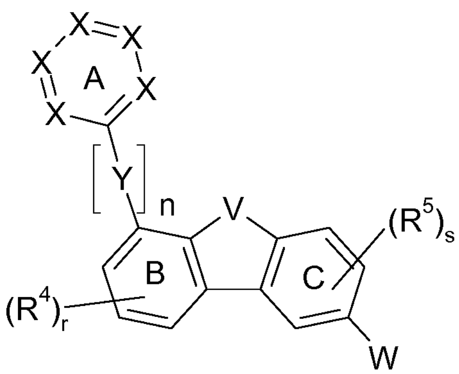

formule (2)

dans laquelle :

X est N ou $CR^1$, où trois des cinq groupes X dans le cycle A sont égaux à N, et le cycle A est une 1,3,5-triazine.

3. Composé selon la revendication 1 ou 2, **caractérisé en ce que** W est défini par la formule (W-2) :

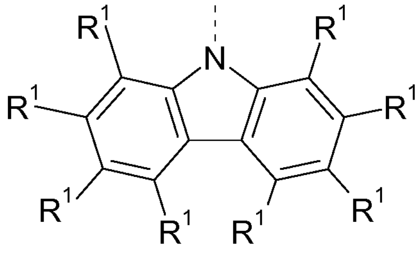

formule (W-2)

dans laquelle $R^1$ est défini tel qu'indiqué selon la revendication 1.

4. Composé selon une ou plusieurs des revendications 1 à 3, **caractérisé en ce que** le composé présente la formule qui suit :

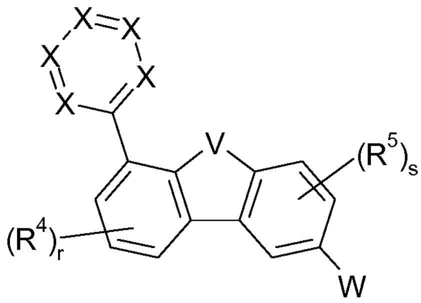

formule (4)

dans laquelle X est défini tel qu'indiqué selon la revendication 2.

5. Composé selon une ou plusieurs des revendications 1 à 4, **caractérisé en ce que** le composé présente la formule générale (7) :

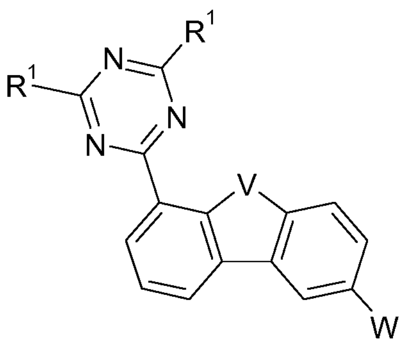

formule (7)

6. Composé selon une ou plusieurs des revendications 1 à 5, **caractérisé en ce que** le groupe W est un carbazole, un indénocarbazole ou un indolocarbazole.

7. Composé selon une ou plusieurs des revendications 1 à 6, **caractérisé en ce que** le groupe W est un groupe de la formule (W-5) :

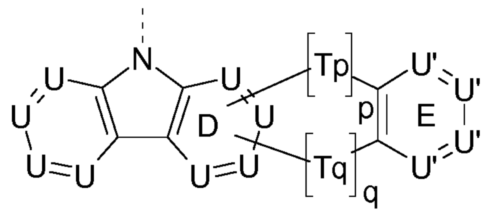

formule (W-5)

dans laquelle les définitions qui ont été présentées ci-avant s'appliquent aux indices et aux symboles qui sont utilisés, et dans laquelle en outre :

Tp, Tq sont, de manière identique ou différente, un pont divalent ; Tp et Tq sont de préférence sélectionnés parmi $N(R^2)$, $B(R^2)$, O, $C(R^2)_2$, $Si(R^2)_2$, C=O, $C=NR^2$, $C=C(R^2)_2$, S, S=O, $SO_2$, $P(R^2)$ et $P(=O)R^2$ ; $N(R^2)$, O, $C(R^2)_2$ et S ont une grande préférence ici et $N(R^2)$ et $C(R^2)_2$ sont tout particulièrement préférés ;
U' est, de manière identique ou différente pour chaque occurrence, $CR^2$ ou N, de préférence $CR^2$ ;
p est 0 ou 1 ; où p égal à 0 signifie que le cycle E et le cycle D sont liés par une liaison simple ;
q est 0 ou 1 ; où q égal à 0 signifie que le cycle E et le cycle D sont liés par une liaison simple ;

et où p + q = 1 ou 2 et est de préférence égal à 1 ;
et où Tp et Tq sont chacun liés sur des groupes U adjacents du cycle D selon chaque orientation possible ; et où, en outre, chaque groupe U qui est lié sur Tp ou sur Tq représente un atome de carbone.

**8.** Composition comprenant au moins un composé selon une ou plusieurs des revendications 1 à 7 et au moins un autre composé qui est sélectionné parmi le groupe qui est constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

**9.** Composition selon la revendication 8, **caractérisée en ce que** le composé additionnel est un matériau hôte ou un matériau de matrice.

**10.** Composition selon la revendication 8 ou 9, **caractérisée en ce que** le composé additionnel présente une bande interdite de 2,5 eV ou plus, de préférence de 3,0 eV ou plus, de façon très préférable de 3,5 eV ou plus.

**11.** Formulation comprenant au moins un composé selon une ou plusieurs des revendications 1 à 7 ou au moins une composition selon une ou plusieurs des revendications 8 à 10 et au moins un solvant.

**12.** Utilisation d'au moins un composé selon une ou plusieurs des revendications 1 à 7 ou d'au moins une composition selon une ou plusieurs des revendications 8 à 10 dans un dispositif électronique, de préférence dans un dispositif électroluminescent organique, de façon très préférable dans une diode à émission de lumière organique (OLED) ou une cellule électrochimique à émission de lumière organique (OLEC, LEEC, LEC), de façon très particulièrement préférable dans une OLED, de préférence dans une couche d'émission (EML), une couche de transport d'électrons (ETL) et dans une couche de blocage de trous (HBL), de façon très préférable dans une EML et une ETL et de façon très particulièrement préférable dans une EML.

**13.** Dispositif électronique comprenant au moins un composé selon une ou plusieurs des revendications 1 à 7 ou au moins une composition selon une ou plusieurs des revendications 8 à 10, de préférence dans une couche d'émission (EML), une couche de transport d'électrons (ETL) et dans une couche de blocage de trous (HBL), de façon très préférable dans une EML et une ETL et de façon très particulièrement préférable dans une EML.

**14.** Dispositif électronique selon la revendication 13, **caractérisé en ce qu'**il est sélectionné parmi les circuits intégrés organiques (OIC), les transistors à effet de champ organiques (OFET), les transistors à film mince organiques (OTFT), les dispositifs électroluminescents organiques, les cellules solaires organiques (OSC), les détecteurs optiques organiques, les photorécepteurs organiques, de préférence un dispositif électroluminescent organique.

**15.** Dispositif électronique selon la revendication 13 ou 14, **caractérisé en ce qu'**il s'agit d'un dispositif électroluminescent organique qui est sélectionné parmi le groupe qui est constitué par les transistors à émission de lumière organiques (OLET), les dispositifs à extinction de champ organiques (OFQD), les cellules électrochimiques à émission de lumière organiques (OLEC, LEC, LEEC), les diodes laser organiques (O-laser) et les diodes à émission de lumière organiques (OLED), de préférence les OLEC et les OLED, de façon très préférable les OLED.

**16.** Procédé pour la production d'un dispositif électronique selon une ou plusieurs des revendications 13 à 15, **caractérisé en ce qu'**au moins une couche organique est appliquée au moyen d'un dépôt en phase gazeuse ou à partir d'une solution.

**17.** Dispositif électronique selon la revendication 15, pour une utilisation en médecine pour la photothérapie, de préférence pour la photothérapie de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- WO 2004093207 A
- WO 2010006680 A
- WO 2005003253 A
- WO 2008056746 A
- EP 0906947 A
- EP 0908787 A
- EP 0906948 A
- US 5935721 A
- WO 03095445 A
- CN 1362464
- WO 01076323 A
- WO 01021729 A
- WO 2004013073 A
- WO 2004018588 A
- WO 2003087023 A
- WO 2004018587 A
- WO 2004016575 A
- WO 2008145239 A
- WO 2005039246 A
- US 20050069729 A
- JP 2004288381 A
- EP 1205527 A
- WO 2008086851 A
- WO 2010136109 A
- WO 2011000455 A
- WO 2010015306 A
- WO 2007063754 A
- WO 2009069442 A
- JP 2009021336 A
- WO 2011057706 A
- WO 2011057706 A2
- WO 2012130709 A1
- JP 2013131518 A
- WO 2010108579 A
- US 7294849 B
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- WO 2006108497 A
- WO 2006122630 A
- WO 2008006449 A
- WO 2007140847 A
- WO 2010012328 A
- EP 676461 A
- WO 2004081017 A
- WO 2004058911 A
- WO 2005084081 A
- WO 2005084082 A
- WO 2006048268 A
- WO 2006117052 A
- WO 2011088877 A
- WO 2011128017 A
- EP 1617710 A
- EP 1617711 A
- EP 1731584 A
- JP 2005347160 A
- WO 2007137725 A
- WO 2005111172 A
- EP 652273 A
- WO 2009062578 A
- WO 2010054729 A
- WO 2010054730 A
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- EP 12000929 A
- EP 12005369 A
- EP 12005370 A
- EP 12005371 A
- EP 11009127 A
- EP 11007067 A

**In der Beschreibung aufgeführte Nicht-Patentliteratur**


- **M. A. BALDO et al.** *Appl. Phys. Lett.,* 1999, vol. 75, 4-6
- **D. J. TOZER et al.** *J. Chem. Phys.,* 2008, vol. 128, 044118
- **T. MATSUMOTO ; T. NAKADA ; J. ENDO ; K. MORI ; N. KAWAMURA ; A. YOKOI ; J. KIDO.** *Multiphoton Organic EL Device Having Charge Generation Layer*
- **Y. SHIROTA et al.** *Chem. Rev.,* 2007, vol. 107 (4), 953-1010
- **M. S. ARNOLD et al.** *Appl. Phys. Lett.,* 2008, vol. 92, 053301